(19) Europäisches Patentamt
European Patent Office
Office européen des brevets

(11) Publication number: 0 074 599
A1

# (12) EUROPEAN PATENT APPLICATION

(21) Application number: 82108210.4

(22) Date of filing: 07.09.82

(51) Int. Cl.³: C 07 D 487/04
A 61 K 31/40
//C07D498/04, C07D205/08,
(C07D487/04, 209/00, 205/00)

(30) Priority: 09.09.81 PC T/JP81/00226
02.02.82 PC T/JP82/00031

(43) Date of publication of application:
23.03.83 Bulletin 83/12

(84) Designated Contracting States:
CH DE FR GB IT LI NL SE

(71) Applicant: Takeda Chemical Industries, Ltd.
27, Doshomachi 2-chome
Higashi-ku Osaka, 541(JP)

(72) Inventor: Natsugari, Hideaki
35-602, 11 Otani-cho
Nishinomiya-shi Hyogo 662(JP)

(72) Inventor: Matsushita, Yoshihiro
4-14, Komatsuminamicho 2-chome
Nishinomiya-shi Hyogo 663(JP)

(72) Inventor: Yoshioka, Kouichi
5-7, Oharano-kamisatotorimicho
Nishikyo-ku Kyoto-shi Kyoto 610-11(JP)

(74) Representative: von Kreisler, Alek, Dipl.-Chem. et al,
Deichmannhaus am Hauptbahnhof
D-5000 Köln 1(DE)

(54) 5,6-cis-Carbapenem derivatives, their production and use.

(57) A compound of the general formula:

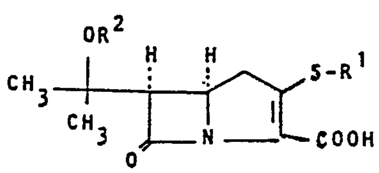

(1)

wherein $R^1$ is alkyl, cycloalkyl, cycloalkenyl, alkynyl, aryl, aralkyln, or a 5 to 8 membered heterocyclic group containing 1 to 4 hetero-atoms, each of the above members may be further substituted; and $R^2$ is hydrogen or hydroxy-protecting group, or a pharmaceutically acceptable salt thereof, or an ester thereof is novel and possesses excellent antimicrobial activity and β-lactamase inhibitory activity, and is utilizable as a treatment agent for infections in man and mammals.

EP 0 074 599 A1

# 5,6-cis-Carbapenem Derivatives, Their Production and Use

The present invention relates to novel 5,6-cis-carbapenem derivatives which exhibit excellent antimicrobial activity and β-lactamase inhibitory activity, their process for production and their use

Carbapenem compounds having the basic skeleton of the formula:

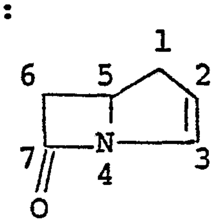

, which have 1-hydroxy-1-methylethyl group in the 6-position and the cis-configuration in the 5- and 6-positions, were found in nature, and it was disclosed that the compounds exhibit a strong antimicrobial activity and a potent β-lactamase inhibitory activity (e.g., Dutch laid open patent application No. 8000628)

However, the substituent groups in the 2-position of such compounds found in nature, which have a 1-hydroxy-1-methylethyl group in the 6-position and the cis-configuration in the 5- and 6-positions, are extremely restricted, and therefore, establishment of the stereo-selective and general method for the synthesis of such compounds and development of such compounds having various substituent groups in the 2-position have been strongly demanded.

In the detailed explanation except examples and reference examples in this specification, various compounds

are explained for the sake of simplification by the use of term "carbapenem" according to the numbering of the above skeleton formula.

However, in examples and reference examples in the present specification, the above skeleton is named as 7-oxo-1-azabicyclo[3.2.0]hept-2-ene according to the systematic nomenclature. In this case, the numbering is as follows.

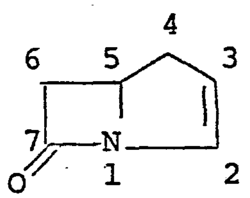

More concretely, the present invention relates to novel derivatives of 5,6-<u>cis</u>-carbapenem of the general formula

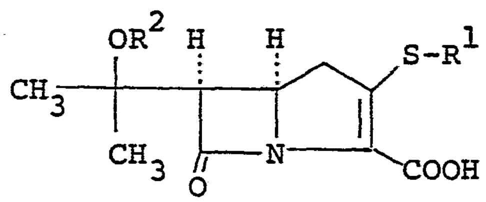

wherein $R^1$ is alkyl, cycloalkyl, cycloalkenyl, alkynyl, aryl, aralkyl, or a 5 to 8 membered heterocyclic group containing 1 to 4 hetero-atoms, each of the above members may be further substituted, and $R^2$ is hydrogen or hydroxy-protecting group, or a pharmaceutically acceptable salt thereof, or an ester thereof.

As the alkyls represented by $R^1$, straight-chain or branched lower alkyls having 1 to 6 carbon atoms are preferred, and use is for example made of methyl, ethyl, n-propyl, isopropyl, n-butyl, isobutyl, sec-butyl, tert-butyl, n-pentyl, isopentyl, n-hexyl, isohexyl, etc.; as the cycloalkyls represented by $R^1$, cycloalkyls having 3 to 8 carbon atoms are preferable, and use is for example made of cyclopropyl, cyclobutyl, cyclopentyl, cyclohexyl, cycloheptyl, adamantyl, etc.; as the alkynyls represented by $R^1$, straight-chain or branched lower alkynyls having

2 to 6 carbon atoms are preferred, and use is for example made of ethynyl, 1-propynyl, 2-propynyl, etc.; as the cycloalkenyls represented by $R^1$, use is for example made of cycloalkenyls having 3 to 8 carbon atoms such as 1-cyclopropenyl, 1-cyclobutenyl, 1-cyclopentenyl, 2-cyclopentenyl, 3-cyclopentenyl, 1-cyclohexenyl, 2-cyclohexenyl, 3-cyclohexenyl, 1-cycloheptenyl and 1,4-cyclohexadienyl, and particularly cycloalkenyls having 4 to 6 carbon atoms are preferable; as the aryls represented by $R^1$, use is for example made of phenyl, α-naphthyl, β-naphthyl, biphenyl, anthryl, etc., and use is preferably made of phenyl, naphthyl, etc.; and as the aralkyls represented by $R^1$, use is for example made of benzyl, phenethyl, phenylpropyl, naphthylmethyl, etc.

As a 5- to 8-membered heterocyclic group represented by $R^1$, use is made of a ring residue or a condensed ring residue containing 1 to 4 hetero-atoms such as nitrogen (which may be oxidized to form N-oxide), oxygen and sulfur atoms and having chemical bonds on carbon atoms, and use is for example made of 2- or 3-pyrrolyl, 2- or 3-furyl, 2- or 3-thienyl, 2- or 3-pyrrolidinyl, 2-, 3- or 4-pyridyl, N-oxido-2-, 3- or 4-pyridyl, 2-, 3- or 4-piperidinyl, 2-, 3- or 4-pyranyl, 2-, 3- or 4-thiopyranyl, pyrazinyl, 2-, 4- or 5-thiazolyl, 2-, 4- or 5-oxazolyl, 3-, 4- or 5-isothiazolyl, 3-, 4- or 5-isoxazolyl, 2-, 4- or 5-imidazolyl, 3-, 4- or 5-pyrazolyl, 3- or 4-pyridazinyl, N-oxido-3- or 4-pyridazinyl, 2-, 4- or 5-pyrimidinyl, N-oxido-2-, 4- or 5-pyrimidinyl, piperadinyl, 4- or 5-(1,2,3-thiadiazolyl), 3- or 5-(1,2,4-thiadiazolyl), 1,3,4-thiadiazolyl, 1,2,5-thiadiazolyl, 4- or 5-(1,2,3-oxadiazolyl), 3- or 5-(1,2,4-oxadiazolyl), 1,3,4-oxadiazolyl, 1,2,5-oxadiazolyl, 1,2,3- or 1,2,4-triazolyl, 1H- or 2H-tetrazolyl, pyrido[2,3-d]-pyrimidyl,benzopyranyl, 1,8-, 1,5-, 1,6-, 1,7-, 2,7- or 2,6-naphthylidyl, quinolyl, thieno[2,3-b]pyridyl, etc.

The alkyls and alkynyls represented by $R^1$ may be substituted for 1 to 3 hydrogen atoms by, for example,

cycloalkyl, cycloalkenyl, aryl, a 5 to 8 membered heterocyclic group containing 1 to 4 hetero-atoms, alkoxycarbonyl, acyl, oxo, halogen, cyano, hydroxy, alkoxy, aryloxy, acyloxy, carbamoyloxy, hydroxysulfonyloxy, alkylsulfonyloxy, arylsulfonyloxy, nitro, amino, carboxy, aminocarbonyl, alkylthiocarbonyl, mercapto, alkylthio, aminoalkylthio, acylaminoalkylthio, aralkylthio, arylthio, a group of Het-S in which Het means a 5 to 8 membered heterocyclic group containing 1 to 4 hetero-atoms, quaternary ammonium, etc. As the specific, substituted alkyl groups, for example, use is also made of alkyls of the formula:

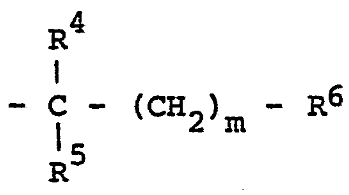

[wherein m is 0, 1, 2 or 3; $R^4$ and $R^5$ are the same or different and each means hydrogen, alkyl, cycloalkyl, alkenyl, aralkyl, aryl, a 5 to 8 membered heterocyclic group containing 1 to 4 hetero-atoms, alkoxycarbonyl or acyl, or $R^4$ and $R^5$ combine together to form oxo; $R^6$ is hydrogen, alkyl, cycloalkyl, aryl, a 5 to 8 membered heterocyclic group containing 1 to 4 hetero-atoms, halogen, cyano, hydroxy, alkoxy, aryloxy, acyloxy, carbamoyloxy, hydroxysulfonyloxy, alkylsulfonyloxy, arylsulfonyloxy, nitro, amino, carboxy, alkoxycarbonyl, aminocarbonyl, alkylthiocarbonyl, acyl, mercapto, alkylthio, aminoalkylthio, acylaminoalkylthio, aralkylthio, arylthio, a group of Het-S in which Het means a 5 to 8 membered heterocyclic group containing 1 to 4 hetero-atoms or quaternary ammonium]. As the substituent groups for the cycloalkyl, cycloalkenyl, aralkyl, aryl, a 5 to 8 membered heterocyclic group containing 1 to 4 hetero-atoms or quaternary ammonium represented by $R^1$ or designated as the substituent groups for the above-mentioned alkyl or alkynyl, further, use is for example made of alkyl, alkoxy, alkenyl, aryl, aralkyl, mercapto, alkylthio, arylthio, aralkylthio, alkylsulfonyl, arylsulfonyl, aralkylsulfonyl,

trihalogenoalkyl, hydroxy, oxo, thioxo, halogen, nitro, amino, cyano, carbamoyl, carboxyl, acyl, acyloxy, acylamino, hydroxyalkyl, carboxyalkyl, halogenoalkyl, mono- or di-alkylaminoalkyl, etc. As the alkoxy, straight-chain or branched lower alkoxy having 1 to 6 carbon atoms are preferable, and use is for example made of methoxy, ethoxy, n-propoxy, isopropoxy, n-butoxy, isobutoxy, sec-butoxy, tert-butoxy, n-pentyloxy, isopentyloxy, n-hexyloxy, isohexyloxy, etc.; as the halogen, use is made of fluorine, chlorine, bromine, iodine; as the quaternary ammonium group, use is for example made of pyridinium, quinolinium, etc.; as the acyl group, use is made of acyl group such as formyl, alkylcarbonyl, arylcarbonyl, aralkylcarbonyl and Het-acetyl in which Het means a 5 to 8 membered heterocyclic group containing 1 to 4 hetero-atoms, and among others, use is for example made of acetyl, propionyl, n-butyryl, isobutyryl, n-pentanoyl, n-hexanoyl, benzoyl, 4-hydroxybenzoyl, 4-methoxybenzoyl, phenylacetyl, 4-hydroxyphenylacetyl, 4-methoxyphenylacetyl, 2-thienylcarbonyl, 2-furylcarbonyl, 2-, 4- or 5-thiazolylacetyl, 2- or 3-thienylacetyl, 2- or 3-furylacetyl, 2-amino-4- or 5-thiazolylacetyl, etc.; and as the alkyl, cycloalkyl, cycloalkenyl, aryl, aralkyl, a 5 to 8 membered heterocyclic group containing 1 to 4 hetero-atoms, use is made of the above mentioned ones; as the alkenyls, straight-chain or branched lower alkenyls having 2 to 6 carbon atoms are preferred, and use is for example made of vinyl, allyl, isopropenyl, 2-methallyl, 2-butenyl, 3-butenyl, etc.

In cases in which the substituent groups in abovementioned $R^1$ include an amino group, the amino group may be substituted or protected, and in the case of carboxyl and hydroxyl groups being involved, these may also be protected. As the substituent groups for the amino group, use is made of acyl, alkyl, hydroxyalkyl, aralkyl, aryl, a 5 to 8 membered heterocyclic group containing 1 to 4 hetero-atoms, amidino, aminomethylene, carbamoyl, sulfo (sulfonic

acid group), alkylsulfonyl, aralkylsulfonyl, arylsulfonyl, alkoxycarbonyl, aralkyloxycarbonyl, aryloxycarbonyl, etc. (as the acyl, alkyl, alkoxy, aralkyl, aryl and a 5 to 8 membered heterocyclic group containing 1 to 4 hetero-atoms, use is made of the above-mentioned ones). The amino group, together with such substituent groups, may form cyclic amino group such as pyrrolidino, piperidino, morpholino and piperazino. As the amino protecting group, those used for such purpose in the field of β-lactam and peptide synthesis are conveniently employable; for example, use is made of aromatic acyl groups such as phthaloyl, p-nitrobenzoyl, p-tert-butylbenzoyl, p-tert-butylbenzenesulfonyl, benzenesulfonyl and toluenesulfonyl, aliphatic acyl groups such as formyl, acetyl, propionyl, monochloroacetyl, dichloroacetyl, trichloroacetyl, methanesulfonyl, ethanesulfonyl, trifluoroacetyl, maleyl and succinyl, esterified carboxyl groups such as methoxycarbonyl, ethoxycarbonyl, t-butoxycarbonyl, isopropoxycarbonyl, 2-cyanoethoxycarbonyl, 2,2,2-trichloroethoxycarbonyl, 2-trimethylsilylethoxycarbonyl, benzyloxycarbonyl, 2-methylsulfonylethoxycarbonyl, p-nitrobenzyloxycarbonyl, p-methoxybenzyloxycarbonyl, diphenylmethyloxycarbonyl, methoxymethyloxycarbonyl, acetylmethyloxycarbonyl, isobornyloxycarbonyl and phenyloxycarbonyl, and furthermore other amino protecting groups such as trityl, 2-nitrophenylthio, benzylidene, 4-nitrobenzylidene or trialkylsilyl, benzyl and p-nitrobenzyl. The selection of said protective groups is not specifically limited in the present invention. As the carboxyl protecting group, any group that can be normally used as the carboxyl protecting group in the fields of β-lactam and organic chemistry can be utilized, and use is made of ester residue and silyl group such as methyl, ethyl, n-propyl, isopropyl, tert-butyl, tert-amyl, benzyl, p-nitrobenzyl, o-nitrobenzyl, p-methoxybenzyl, benzhydryl, phenacyl, phenyl, p-nitrophenyl, methoxymethyl, ethoxymethyl, benzyloxymethyl, acetoxymethyl, pivaloyloxymethyl, 2-methylsulfonylethyl, 2-trimethylsilylethyl, methylthiomethyl, trityl, 2,2,2-trichloroethyl, 2-iodoethyl, trimethylsilyl, dimethylsilyl, acetylmethyl, p-nitrobenzoylmethyl, p-mesylbenzoylmethyl, phthalimidomethyl, propionyloxymethyl, 1,1-dimethylpropyl, 3-methyl-3-butenyl, succinimidomethyl, 3,5-di-tert-butyl-4-hydroxybenzyl, mesylmethyl, benzenesulfonylmethyl, phenylthiomethyl, dimethylaminoethyl, pyridine-1-oxide-2-methyl, methylsulfinylmethyl, bis(p-methoxyphenyl)methyl and 2-cyano-1,1-dimethylethyl. As the hydroxyl protecting group, further, any group that can be normally used as the hydroxyl protecting group in the fields of β-lactam and organic chemistry can be utilized, and use is for example made of ester residues such as acetyl and chloroacetyl, esterified carboxyl residues such as 2,2,2-trichloroethoxycarbonyl and 2-trimethylsilylethoxycarbonyl, ether residues such as tert-butyl, benzyl, p-nitrobenzyl, trityl, methoxymethyl, methylthiomethyl and β-methoxyethoxymethyl, silyl ether residues such as trimethylsilyl and tert-butyldimethylsilyl, acetal residues such as 2-tetrahydropyranyl and 4-methoxy-4-tetrahydropyranyl, sulfonic acid groups, etc. In the present invention, the selection of the above-mentioned protective groups is not specifically restricted as is the case with the amino or carboxyl protecting groups.

With reference to the above-mentioned formulas, $R^2$ is hydrogen or a hydroxyl protecting group. As the hydroxyl protecting group represented by $R^2$, use is for example made of those as described above. Among others, frequent use is made of protective groups capable of forming an ether linkage such as β-methoxyethoxymethyl, methoxymethyl and methylthiomethyl groups.

The compounds (I) of the present invention, in the event of a carboxyl group being present in the 3- position or being contained in $R^1$, may be in the free form, but can also be in the form of pharmaceutically acceptable salts by means of the methods known per se. For example, the

compounds (I) may also be in the form of salts with nontoxic cations such as sodium and potassium, basic amino acids such as arginine, ornithine, lysine and histidine, and polyhydroxyalkylamines such as N-methylglutamine, diethanolamine, triethanolamine and trishydroxymethylaminomethane. In the case of a basic group being included in $R^1$, furthermore, the compounds may also be in the form of salts with organic acids such as acetic acid, tartaric acid and methanesulfonic acid, inorganic acids such as hydrochloric acid, hydrobromic acid, sulfuric acid and phosphoric acid, acidic amino acids such as arginine, aspartic acid and glutamic acid, and others.

The compounds (I) of the present invention may be in the ester form. As the ester residue use is for example made of the carboxyl-protecting group which is mentioned above for substituent group in $R^1$.

The compounds (I) of the present invention, like starting compounds, in some instances exist as the racemic form (dl-form), and the racemic form also falls into the scope of the present invention. The racemic mixtures, as such or as the d- or ℓ-form after resolution of the racemic form, can be used as medicine.

Specific examples of the compounds (I) of the present invention include the compounds of the formula described in the following:

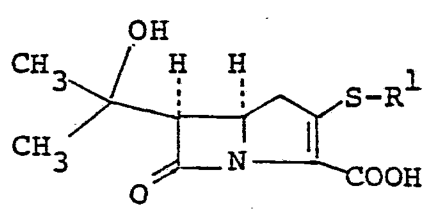

| $R^1$ | $R^1$ |
|---|---|
| $-CH_2CH_2NH_2$ | $-CH_2CH_2OH$ |
| $-CH_2CH_2NHCOOCH_2-$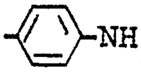$-NH_2$ | $-CH_2CH_2Cl$ |
| $-CH_2CH_2NHCOCH_3$ | $-CH_2CH_2OCH_3$ |
| $-CH_2CH_2NHCOC_2H_5$ | $-CH_2CH_2OC_2H_5$ |
| $-CH_2CH_2NHCO-n-C_3H_5$ | $-CH_2CH_2OCOCH_3$ |
| $-CH_2CH_2NHCO-iso-C_3H_5$ | $-CH_2CH_2N_3$ |
| $-CH_2CH_2NHCOC_6H_5$ | $-CH_2CH_2N(CH_3)_2$ |
| $-CH_3$ | $-CH_2CH_2CH_2NHCONHC_6H_5$ |
| $-CH_2CH_2NHCH_3$ | $-CH_2CH_2CH_2NHCSNHC_6H_5$ |
| $-CH_2CH_2NHC_2H_5$ | $-CH_2CH_2CH_2NHCSNHCH_3$ |
| $-CH_2COOH$ | $-CH_2CH_2CH_2NHCONHCH_3$ |
| $-CH_2COOCH_3$ | $-CH_2CH_2CH_2NHCSNHC_2H_5$ |
| $-CH_2COOC_2H_5$ | $-CH_2CH_2CH_2NHCONHC_2H_5$ |
| $-CH_2CH_2COOCH_3$ | $-CH_2CH_2CH_2N=\overset{H}{C}-NH_2$ |
| $-CH_2CH_2COOC_2H_5$ | $-CH_2CH_2CH_2N=\overset{CH_3}{C}-NH_2$ |
| $-CH_2CONH_2$ | $-CH_2CH_2CH_2OH$ |
| $-CH_2CH_2SCH_3$ | $-CH_2CH_2CH_2OCH_3$ |
| $-CH_2CH_2N=\overset{H}{C}-NH_2$ | $-CH_2CH_2NHCONHCH_3$ |
| $-CH_2CH_2N=\overset{CH_3}{C}-NH_2$ | $-CH_2CH_2NHCSNHCH_3$ |
| $-CH_2CH_2N=\overset{H}{C}-NHCH_3$ | $-CH_2CH_2NHCONHC_2H_5$ |
| $-CH_2CH_5N=\overset{H}{C}-NHC_2H_5$ | $-CH_2CH_5NHCSNHC_2H_5$ |
| $-C_2H_5$ | $-CH_2CH_2NHCONHC_6H_5$ |
| $-isoC_3H_7$ | $-CH_2CH_2NHCSNHC_6H_5$ |

| $R^1$ | $R^1$ |
|---|---|
| 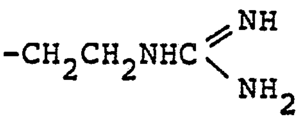 | 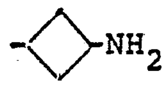 |
| $-CH_2CH_2CH_2NH_2$ | 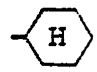 |
| $-CH_2CH_2CH_2NHCOCH_3$ | |
| $-CH_2CH_2CH_2NHCOC_2H_5$ | $-C_6H_5$ |
| $-C_6H_5$-m-Cl | $-C_6H_5$-o-Cl |
| $-C_6H_5$-o-$NH_2$ | $-C_6H_5$-p-Cl |
| $-C_6H_5$-p-$NH_2$ | 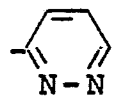 |
| $-C_6H_5$-m-$NH_2$ | |
| $-C_6H_5$-o-$CH_3$ | 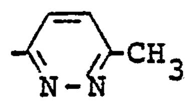 |
| $-C_6H_5$-p-$CH_3$ | |
| $-C_6H_5$-m-$CH_3$ | |
| $-C_6H_5$-p-$OCH_3$ | 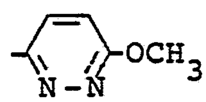 |
| $-C_6H_5$-o-$OCH_3$ | |
| | 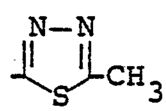 |
| 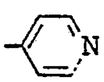 | |
| 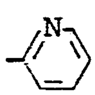 | 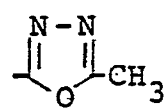 |
| 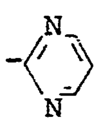 | 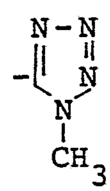 |
| 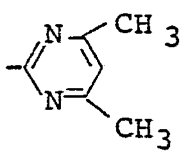 | 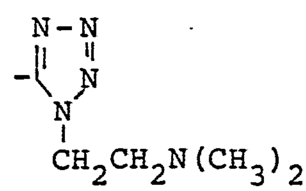 |
| $-CH_2C_6H_5$ | |
| $-CH_2C_6H_5$-p-Cl | 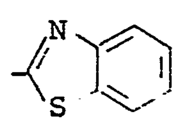 |

| $R^1$ | $R^1$ |
|---|---|
| 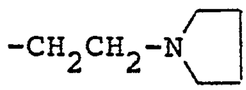 | 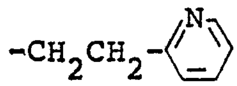 |
| 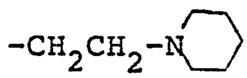 | 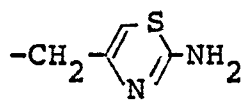 |
| 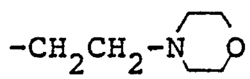 | 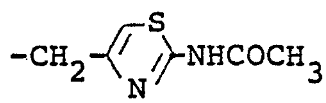 |
| 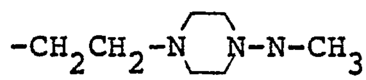 | 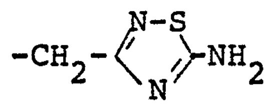 |
| 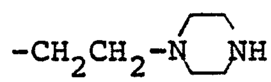 | 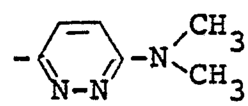 |
| 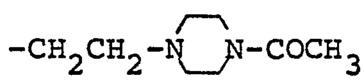 | 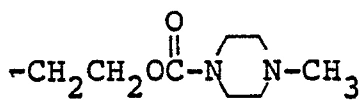 |
| $-CH_2CH_2CH_2NHCH_3$ | 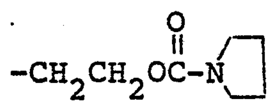 |
| $-CH_2CH_2CH_2N(CH_3)_2$ | 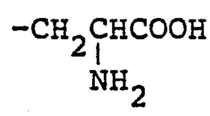 |
| 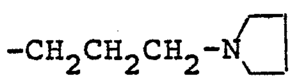 | 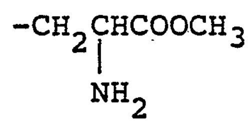 |
| 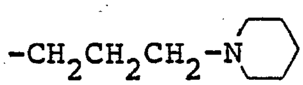 | 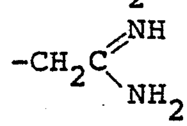 |
| 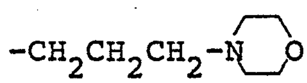 | 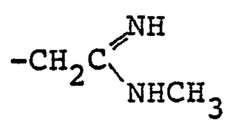 |
| 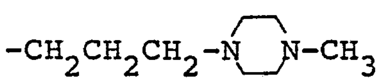 | 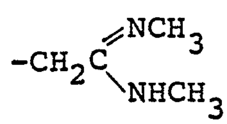 |
| 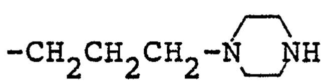 | 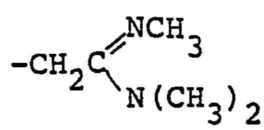 |
| 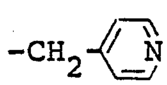 | |
| 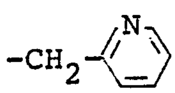 | |
| 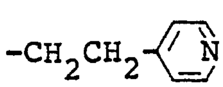 | |

| $R^1$ | $R^1$ |
|---|---|
| 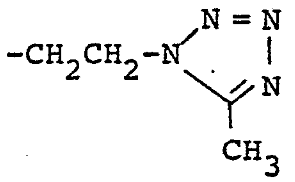 | $-(CH_2)_2NH$<br>$COCH_2NH_2$ |
| 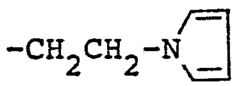 | $-(CH_2)_2N-SO_3H$<br>$CH_3$ |
| $CH_3$<br>$-CHCONHCH_2COOH$ | $-(CH_2)_2N-SO_3H$<br>H |
| $-(CH_2)_2NH$<br>$COCH_2NHCH_3$ | $-CH_2CH_2N(CH_3)_2$ |
| | 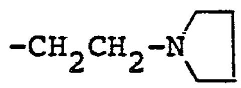 |

5,6-cis-Carbapenem compounds (I) or pharmaceutically acceptable salts thereof or esters thereof are the antibiotics that are active against gram-positive and gram-negative microorganisms and are valuable, and are utilized as a medicine for man and domestic animals and particularly used safely as an antimicrobial agent for the treatment of infections caused by gram-positive or gram-negative bacteria such as Staphylococcus aureus, Escherichia coli and Klebsiella pneumoniae. Further, the antimicrobial agents of the present invention are for example added to livestock feed as a bactericide for preservation of feed; for example, they can be used in aqueous compositions at concentrations within the range of 0.1 to 100 parts of the antibiotic per million parts of solution as a bactericide for destroying and preventing growth of detrimental bacteria on medicinal and dental devices and for preventing growth of hazardous bacteria in water-borne paints and white water discharged from paper mills.

The compounds (I) of the present invention of pharmaceutically acceptable salts thereof or esters thereof can be used solely or in combination with other active ingredients in the processing of either of various

pharmaceutical preparations, and can be for example employed as capsules, tablets, powders or solution, suspensions, or elixirs. These preparations can be administered orally, intravenously or intramuscularly.

Tablets to be employed for oral administration can contain ordinary excipients, binders such as syrup, gum arabic, gelatin, sorbitol, gum tragacanth or polyvinyl pyrrolidone, fillers such as lactose, sugars, corn starch, calcium phosphate, sorbitol or glycine, lubricants such as magnesium stearate, talc, polyethylene glycol or silica, disintegrating agents such as potato starch, or employable wetting agents such as sodium lauryl sulfate. Tablets can be coated by the methods known in the art. The oral liquid preparations may be in such dosage forms as aqueous or oil suspensions, solutions, syrups, elixirs, etc., or in the form of lyophilisates for extemporaneous dissolution in water or a suitable solvent.

Such liquid preparations can also be incorporated with suspending agents such as sorbitol syrup, methylcellulose, glucose/sugars syrups, gelatin, hydroxyethylcellulose, carboxymethylcellulose, aluminium stearate gel or hydrogenated edible oils exemplified by almond oil and fractional-distilled coconut oil, oil-formed esters, propylene glycol or ethyl alcohol, and preservatives such as methyl or propyl p-hydroxybenzoate or sorbic acid. Suppositories can be prepared by use of common suppository bases such as cocoa butter or other glycerides.

Injectable compositions made available in a ampoules or other can be supplied in the unit-dose containers with the addition of a preservative. Said compositions may be in the forms such as suspensions, solutions or emulsions in oily or aqueous solvents, and may be suitably incorporated with auxiliary agents such as suspending agents, stabilizers and/or dispersing agents. Furthermore, the active components can be converted into the form of powders that are reconstituted with appropriate solvent such as sterilized

water not containing any pyrogen before use.

The compounds can also be prepared into suitable dosage forms which can be absorbed by nasal and pharyngeal mocosae or bronchial tissue, such as powders, liquid sprays or inhalants, lozenges and throat paints. For ophthalmological or ortological application, they can be administered as liquid or semi-solid capsules or as drops for instillation. Further, hydrophobic or hydrophilic bases may be utilized to prepare the compounds into preparations for external application such as ointments, creams, lotions, paints and powders.

Furthermore, such dosage forms can contain, in addition to the carriers, other components such as stabilizers, binders, antioxidants, preservatives, lubricants, suspending agents, thickening agents or flavoring agents. Besides, the compositions can be incorporated with other active ingredients to provide a wider spectrum of antimicrobial activity.

For administration to domestic animals, the compound can be formulated into bases for long-acting or fast releasing preparations intended for intramammary use.

The compounds (I) of the present invention or pharmaceutically acceptable salts or esters thereof can be used as the agent for the treatment of bacterial infections, for example in the treatment of respiratory trast infections, urinary trast infections, suppurative diseases, infections in the bile duct, intraintestinal infections, gynecologic infections, surgical infections, etc. in mammals. Their daily dose depends upon the conditions of patients, or body weight of hosts, to be treated, the method and frequency of administration, the parenteral administration method suited for general infections and the oral administration method for intraintestinal infections. In general, their daily oral dose, in single or more applications per day, comprises about 15 to 600 mg of active constituent per 1 kg of body weight of a patient. The suitable daily dose for

adult human is about 10 to about 200 mg of active constituent per 1 kg of body weight, and it is appropriate to administer parenterally the compound in the quantity of about 2.5 to about 100 mg/kg body weight, two to four times daily.

Compositions containing the compound (I) or a pharmaceutically acceptable salt or a ester thereof can be administered for example in several, liquid or solid single-dose forms capable of being orally given. The liquid or solid compositions intended for single-dose use contain 0.5 to 99% of the active substance. The suitable range of the content is about 10 to 60%. The compositions normally contain about 15 to 1500 mg of the active constituent. However, it is generally suitable to use the dose in the range of about 250 to 1000 mg.

The compounds (I) or pharmaceutically acceptable salts thereof or esters thereof exhibit β-lactamase inhibitory activity. That is to say, the compounds of the present invention have inhibitory activity against various kinds of β-lactamase produced by gram-positive and gram-negative bacteria for example S. aureus, E. coli, E. cloacae. The compounds of the present invention may be employed in combination with β-lactam antibiotics, in addition to the above-mentioned applications. As examples of such β-lactam antibiotics, use can be made of penicillins such as benzylpenicillin, phenoxymethylpenicillin, carbenicillin, ampicillin, amoxicillin and sulbenicillin, cephalosporins such as cephaloridine, cephalothin, cephazolin, cephalexin, cephoxitin, cephacetrile, cephamandol, cephmenoxime, cephthrodine, cephothiam, cephotaxime, cephapirin, cephthizoxime, cepharadin and cephaloglycin, etc.

In addition to the above-mentioned various biological characteristics of the compounds of the present invention those compounds have remarkable stability in the body of human and animals particularly in kidney.

5,6-cis-Carbapenem derivatives (I), or their pharmaceutically acceptable salts or esters can be produced by reacting a compound (II) or its salt or its ester with a

compound (III) or its salt.

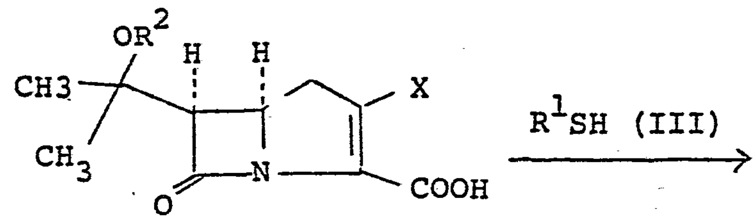


(I)

(II)

Referring to the above-mentioned formula (II), the group to be eliminated which is represented by X may be any of groups which can be substituted by a group of $R^1S$- (wherein $R^1$ is as defined above). For example, use is made of substituted sulfonyloxy groups such as p-toluenesulfonyloxy, p-nitrophenylsulfonyloxy, p-bromophenylsulfonyloxy and methanesulfonyloxy, halogens such as chlorine and bromine, and disubstituted phosphoryloxy groups such as diphenylphosphoryloxy and diethylphosphoryloxy.

As salts of esters of the compound (II), use is made of those as described with reference to the salts or esters of the compounds (I) mentioned above. The compound (III) may be used in the free form, but may be subjected to the reaction in the form of salts, such as salts with alkali metals such as lithium, sodium and potassium and salts with alkaline earth metals such as calcium and magnesium. 1 Mole of the compound (II) or its salts or its ester is reacted with 1 to 10 moles, preferably 1 to 5 moles, of the compound (III) or its salt. Normally, the reaction is carried out in a solvent. As the solvent, preferred use is made of halogenated hydrocarbons such as dichloromethane and chloroform, nitriles such as acetonitrile, ethers such as dimethoxyethane and tetrahydrofuran, dimethylformamide, dimethylsulfoxide, hexamethylphosphoramide, etc. Addition of a base favorably promotes the reaction. Preferred examples of such base include sodium hydrogen carbonate, potassium hydrogen carbonate, sodium carboante, potassium carbonate, sodium hydride, potassium hydride, sodium amide,

sodium methoxide, triethylamine, diisopropylethylamine, pyridine, etc. In this reaction, furthermore, the compound (III) may be converted to its salts with alakli metals, alkaline earth metals, etc. as described above to react with the compound (II) or its salt or its ester, instead of the use of a base. The amount of a base to be used varies depending upon the types of the compounds (II) and (III) and solvent being employed and other reaction conditions, and normally is 1 to 10 moles, preferably 1 to 5 moles, per 1 mole of the compound (II). The reaction temperature is in the range of -50°C to 40°C, preferably -30°C to 20°C. The reaction time varies with the type of the compound (II) or its salt or its ester, kind of the compound (III) or its salt, reaction temperature, etc., and is 1 to 72 hours, preferably 1 to 24 hours. The resultant compound (I) or its pharmaceutically acceptable salt or its ester can be isolated and purified by the means known per se, such as concentration, phase transfer, solvent extraction, crystallization, recrystallization, fractional distillation and chromatography. For example, the objective compound can be obtained by adding an organic solvent immiscible with water such as ethyl acetate as well as water to the reaction mixture, separating the organic solvent layer to wash with water, drying with a drying agent and distilling off the solvent. The compound [I] or its pharmaceitucally acceptable salt or its ester obtained in this manner can be further purified by the conventional procedures such as thin layer chromatography for fractionation, column chromatography and recrystallization method, if necessary.

When the compound (I) or its pharmaceutically acceptable salt or its ester thus obtained has the protective group, such protective group can be removed, if desired. As the procedure of removing said protective group, the conventional procedures such as removal with an acid, removal with a base, removal with hydrazine, removal

through reduction and the procedure of bringing the protected compound into contact with an iminohalogenating agent and then an iminoetherifying agent and conducting hydrolysis, if necessary, can be suitably selected and carried out, depending upon the type of such protective group. In the case of a procedure of removal with an acid, the useful acids which may vary depending upon the kind of protective groups and other conditions include inorganic acids such as hydrochloric acid, sulfuric acid and phosphoric acid, and organic acids such as formic acid, acetic acid, trifluoroacetic acid, propionic acid, benzenesulfonic acid and p-toluenesulfonic acid as well as acidic ion exchange resins, etc. In the event of the procedure of removal with a base, the useful bases, which vary with the type of protective groups and other conditions, include inorganic bases such as hydroxides and carbonates of alkali metals, e.g. sodium and potassium, or alkaline earth metals, e.g. calcium and magnesium, and organic bases such as metal alkoxides, organic amines and quaternary ammonium salts as well as basic ion exchange resins, etc. When a solvent is used in the above procedure of removal of the protecting group with acid or base, hydrophilic organic solvents, water or a mixed solvent thereof are often employed. In the case of the procedure of removal through reduction, the employable procedures vary with the type of protective groups and other conditions, and their examples include the procedures with use of metals such as tin and zinc or metal compounds such as chromium dichloride and chromium acetate and acids such as organic and inorganic acids, e.g. acetic acid, propionic acid and hydrochloric acid, the procedures of conducting reduction in the presence of metal catalysts for catalytic reduction, etc. Examples of the catalysts useful in the procedures through catalytic reduction include platinum catalysts such as platinum wire, platinum sponge, platinum black, platinum oxide and colloidal platinum, palladium catalysts such as palladium

sponge, palladium black, palladium oxide, palladium barium sulfate, palladium barium carbonate, palladium carbon, palladium silica gel and colloidal palladium, reduced nickel, nickel oxide, Raney nickel, Urushibara nickel, etc.; and in the case of the procedures through reduction by metal and acid, compounds of metals such as iron and chromium as well as inorganic acids such as hydrochloric acid and organic acids such as formic acid, acetic acid and propionic acid are employed. The procedures through reduction are normally carried out in a solvent, and in the procedures through catalytic reduction, frequent use is made of alcohols such as methanol, ethanol, propyl alcohol and isopropyl alcohol, ethyl acetate, and others. In the event of the procedures through reduction by metal and acid, frequent use is made of water, acetone, etc., but when the acid is liquid, such acid itself can also be employed as solvent. Throughout the procedures of removal with acid or base and through reduction, the reaction is normally carried out at temperaure lowered by cooling or raised by warming. As the iminohalogenating agents which are useful in the case of the procedure of removing protective groups by bringing the protected compound into contact with an iminohalogenating agent and then an iminoetherifying agent and conducting hydrolysis, if necessary, use is for example made of phosphorus trichloride, phosphorus pentachloride, phosphorus tribromide, phosphorus oxychloride, thionyl chloride and phosgene. The reaction temperature is not specifically limited, but the reaction is often carried out at room temperature or under cooling. As the iminoetherifying agents, use is made of alcohols or metal alkoxides; examples of the alcohols which are usable include alkanols such as methanol, ethanol, propanol, isopropanol, n-butanol and tert-butanol and compounds from such alkanols by substituting their alkyl moieties with alkoxy groups such as methoxy, ethoxy, propoxy, isopropoxy and butoxy, and examples of the metal alkoxides which are

employable include alkoxides of alkali metals such as sodium alkoxides, potassium alkoxides, etc. and alkoxides of alkaline earth metals such as calcium alkoxides, barium alkoxides, etc., which are all derived from the above alcohols. In the case of the protective group being a residue of an organic carboxylic acid where substituents such as free amino, hydroxyl, mercapto, carboxyl and sulfo are present in the carbon atom adjacent to its carbonyl group, for example, it produces preferred results and is advantageous to remove the protective group after conducting such treatment as may enhance the neighboring-group effect of these groups to increase the reactivity of the carbonyl group. As an illustration of such case with a protective group in which the substituent on the carbon atom adjacent to its carbonyl group is for example a free amino group, there may be mentioned the procedures of removing protective groups that comprise applying the known procedures employed for splitting the peptide linkage such as the procedure of converting the free amino group into a thioureido group and then conducting deacylation. The reaction temperature is not specifically restricted and is suitably selected according to the type of protective groups, the kind of procedures of removing protective groups, etc., but the reaction is desirably conducted under mild conditions under cooling to under warming.

In the case of the compound (I) its pharmaceutically acceptable salt or its ester where the carboxyl-protecting group is a halogenoalkyl or aralkyl (e.g. benzhydryl) group, etc., specifically, removal of the protective group is achieved by contacting the compound with a reducing agent. As the preferred examples of the reducing agents that are usable in this reaction, there may be mentioned zinc and acetic acid in the case of the carboxyl-protecting group being halogenoalkyl groups such as 2,2-dibromoethyl and 2,2,2-trichloroethyl, while, in the event of the carboxyl-protecting group being aralkyl groups such as

benzyl and p-nitrobenzyl or a benzhydryl group, there may be mentioned hydrogen and catalytic reduction catalysts such as platinum oxide, platinum black, platinum sponge, palladium-carbon, palladium black, palladium-barium sulfate, palladium-barium carbonate, reduced nickel, Raney nickel and Urushibara nickel or alkali metla sulfides such as sodium sulfide or potassium sulfide. Furthermore, removal by photo irradiation and removal by electrolytic reduction can be carried out with o-nitrobenzyl and p-methoxybenzyl groups, respectively. The reaction is conducted in the presence of a solvent. The solvent which is useful is not specifically restricted, unless it participates in this reaction, but preferred examples thereof include alcohols such as methanol and ethanol, ethers such as tetrahydrofuran and dioxane, fatty acids such as acetic acid and solvent mixtures of these organic solvents with water. The reaction temperature is normally in the neighborhood of 0° to 40°C, and the reaction time varies depending upon the types of starting compounds and reducing agents and is normally 5 minutes to 12 hours. When the protective group of the amino group in the compound (I) or its pharmaceutically acceptable salts or its ester is for example p-nitrobenzyloxycarbonyl, o-nitrobenzyloxycarbonyl, p-methoxybenzyloxycarbonyl, benzyloxycarbonyl, etc., further, such protective groups are removed by the above-mentioned reactions for removal of carboxyl-protecting groups. When the protective group of the hydroxyl group in the compound (I) or its pharmaceutically acceptable salts or its ester is lower aliphatic acyloxy groups such as acetoxy, the protective groups can be removed by treatment with a base in the presence of an aqueous solvent. The solvent which is useful is not specifically restricted, if it is such a solvent as may be employed in the normal hydrolysis reaction, but its suitable examples include water or solvent mixtures of water with organic solvents such as alcohols exemplified by methanol and ethanol or

ethers exemplified by tetrahydrofuran and dioxane. The bases which are useful are not specifically restricted, unless they exert adverse effect on the β-lactam ring, and the reaction is conducted with use of carbonates of alkali metals such as sodium carbonate and potassium carbonate. The reaction is carried out at temperatures of 0°C to room temperature. The reaction time varies with the type of starting compounds, reaction temperature, etc., and is normally 1 to 6 hours. In cases in which tri-lower-alkylsilyl groups such as tert-butyldimethylsilyl are used as the hydroxyl-protecting group, such groups are removed by treatment with fluorine ions such as ammonium tetrabutyl fluoride or potassium fluoride. As the solvent which is used, ethers such as tetrahydrofuran and dioxane are suitable. The reaction is carried out by treating in the neighborhood of room temperature for 10 to 18 hours.

In the above-mentioned reactions for removal of the protective groups in the compound (I) where $R^1$ is a group having substituent derived from carboxyl, the derivatives relative to said substituent in some instances, is converted into carboxyl group, and such case, needless to say, comes into the scope of this invention.

When an ester at 3-position of the compound (II) is reacted with compound (III) or its salt, the carboxyl at 3-position of the resulting compound (I) is esterified. In such a case the ester residue can be removed by a method mentioned above for the removal of the carboxyl-protecting group.

The compound (I) as obtained in this manner in free form can be converted to the desired salts by a *per se* conventional method.

The compound (I), having a carboxyl group, can normally be reacted with a base to form a salt. Therefore, the compound (I) is in some instances obtained as a salt, and the compound obtained as a salt may be converted into the free form or other salts. Further, the compound

obtained inthe free form may be converted into a salt. As the procedure of converting the compound (I) obtained as a salt into the free form, use is for example made of a procedure of utilizing an acid. The acids which are useful vary with the type of protective groups and other conditions, and as such acids, use is often made of inorganic acids such as hydrochloric acid, sulfuric acid and phosphoric acid and organic acids such as formic acid, acetic acid and p-toluenesulfonic acid. In addition to these, acidic ion exchange resins, etc. are employed. As the solvent, use is in many cases made of hydrophilic solvents such as acetone, tetrahydrofuran, methanol, ethanol and dioxane, water or a mixed solvent. This procedure is normally conducted at room temperature, but may also be carried out under cooling or under warming. The reaction time varies depending upon the types of acids and solvents and temperature, but it is preferable that the reaction be completed within a short period of time. The compound (I) in the free form as obtained in this manner can be isolated by the known procedure as described above.

When the compound (I) thus obtained has an amino group among the substituent groups represented by $R^1$, the amino group can be converted into amido, ureido, amidino or quanidino group, etc., and when the compound has a hydroxyl group instead, the hydroxyl group can also be converted into acyloxy group, halogen atom or azido group.

The reaction converting amino group into amido, ureido, amidino ($-NH-\overset{|}{C}=N-$ group) or guanidino group can be conducted by a variety of the known procedures. The conversion reaction into amido group can be carried out by contacting with an acylating agent in the presence of a solvent. The solvent which is used is not specifically restricted, but preferred examples thereof include halogenated hydrocarbons such as chloroform and methylene chloride and ethers such as tetrahydrofuran and dioxane. The acylating agent which is used is not specifically restricted, if it acylates

amino groups, and examples thereof include fatty acid anhydrides such as acetic anhydride and propionic anhydride, and halide derivatives of fatty acids such as acetyl chloride, propionyl chloride, n-butyl chloride, isobutyl bromide and methoxallyl chloride. This reaction is suitably conducted in the presence of a base, and preferred examples of the base which is usable include organic bases such as triethylamine and pyridine, and alkali metal salts of fatty acids such as sodium acetate and potassium acetate. The reaction temperature is not specifically restricted, but the temperatures in the range of 0°C to 40°C are preferable. The time required for the completion of the reaction varies with the type of acylating agent, reaction temperature, etc., and normally ranges from 0.5 to 5 hours. The conversion reaction into ureido group is conducted by reacting with substituted isocyanates or substituted isothiocyanates in the presence of such a solvent as may be employed for the conversion reaction into amido group. As the substituted isocyanate, use can for example be made of methyl isocyante, ethyl isocyanate, phenyl isocyanate, p-bromophenyl isocyanate, etc. and as the substituted isothiocyanate, use can be for example made of methyl isothiocyanate, phenyl isothiocyanate, etc. The reaction temperature is in the neighborhood of 0° to 40°C, and the reaction time is normally 0.5 to 5 hours. The conversion into amidino group is conducted by reacting with for example imido-esters in a solvent such as dioxane, tetrahydrofuran, dimethylformamide, chloroform, acetone, acetonitrile and water. As the imido-ester, use can for example be made of methyl formimidate, ethyl formimidate, methyl acetimidate, ethyl acetimidate, methyl phenylacetimidate, ethyl N-methyl-formimidate, methyl N-ethylformimidate, methyl N-isopropylformimidate, etc. The reaction temperature is in the neighborhood of 0° to 25°C, and the reaction time normally ranges from 1 to 6 hours. The conversion into guanidino group is conducted

by reacting with for example O-alkyl or O-aryl pseudourea or S-alkyl or S-arylpseudothioureas in a solvent such as water, dimethylformamide and hexamethylphosphoramide. As the pseudoureas, use can be made of O-methylpseudourea, S-methylpseudourea, O-2,4-dichlorophenylpseudourea, S-p-nitrophenylpseudothiourea, O-N,N-trimethylpseudourea, etc. The reaction temperature is in the neighborhood of 0° to 40°C, and the reaction time normally range from 1 to 24 hours. The compound (I) thus obtained can be isolated and purified by the procedures known per se.

The reaction of converting hydroxyl group into acyloxy group, halogen atom or azido group is performed by a variety of known procedures. The conversion reaction into acyloxy group can be carried out in the same manner as the above-mentioned reaction of converting amino group into amido group. The conversion into halogen atom is performed by halogenating in accordance with a conventional method. As the halogenating agent which is useful, use is for example made of thionyl chloride, thionyl bromide, oxalyl chloride, carbon tetrachloride-triphenylphosphine, carbon tetrabromidetriphenylphosphine, etc. Preferred examples of the solvent which is usable include ethers such as tetrahydrofuran and dioxane, aromatic hydrocarbons such as benzene and toluene, and others. The reaction temperature is desirably in the neighborhood of 0°C to room temperature, and the reaction time normally ranges from 15 minutes to 5 hours. The conversion reaction into azido group is performed by reacting with hydrogen azide or diphenylphosphoric acid azide in the presence of a phosphine derivative and an azodicarboxylic acid diester. Preferred examples of the phosphine derivative which is useful include triphenylphosphine and tri-n-butylphosphine, and as the azodicarboxylic acid diester, use is for example made of dimethyl azodicarboxylate, diethyl azodicarboxylate, etc. Preferred examples of the solvent which is used include halogenated hydrocarbons such as methylene chloride

and chloroform, and ethers such as tetrahydrofuran and dioxane. The reaction temperature is 0°C to 60°C, and the reaction time is preferably 5 minutes to 5 hours. The compound (I) thus obtained can be isolated and purified by the procedures known per se.

The sulfur atom in the compound (I) or its pharmaceutically acceptable salt or its ester can be oxidized by the procedures known per se. The reaction, for example, is carried out by use of a mild oxidizing agent which does not substantially act on the carbapenem skeleton, such as perbenzoic acid, ozone, phenyl dichloroiodide, hydrogen peroxide, sodium meta-periodate and sodium hypochlorite. Particularly, perbenzoic acid, m-chloroperbenzoic acid, etc. are for example preferable. When the oxidizing agent is used in 1 equivalent relative to the compound (I), normally, there is obtained a sulfoxide $(-\overset{\overset{O}{\uparrow}}{S}-)$, and when the oxidizing agent is employed in the proportions of 2 equivalents or more, a sulfone $(-\underset{\underset{O}{\downarrow}}{\overset{\overset{O}{\uparrow}}{S}}-)$ is obtained. The reaction is normally carried out in an inert solvent, such as dichloromethane, chloroform and carbon tetrachloride, at temperatures in the range of -30° to 25°C., and the reaction time ranges from about 3 minutes to 3 hours for the sulfoxide and from about 1 to 9 hours for the sulfone. The S-oxidation product can be isolated and purified by a variety of known procedure, though the sulfoxide in some instances produces two kinds of diastereomeric isomers having different configurations of the S-O bond. These isomers can be separated by the known procedures such as column chromatography and recrystallization.

The compounds (II) in the present invention can be produced by the following method or methods similar thereto.

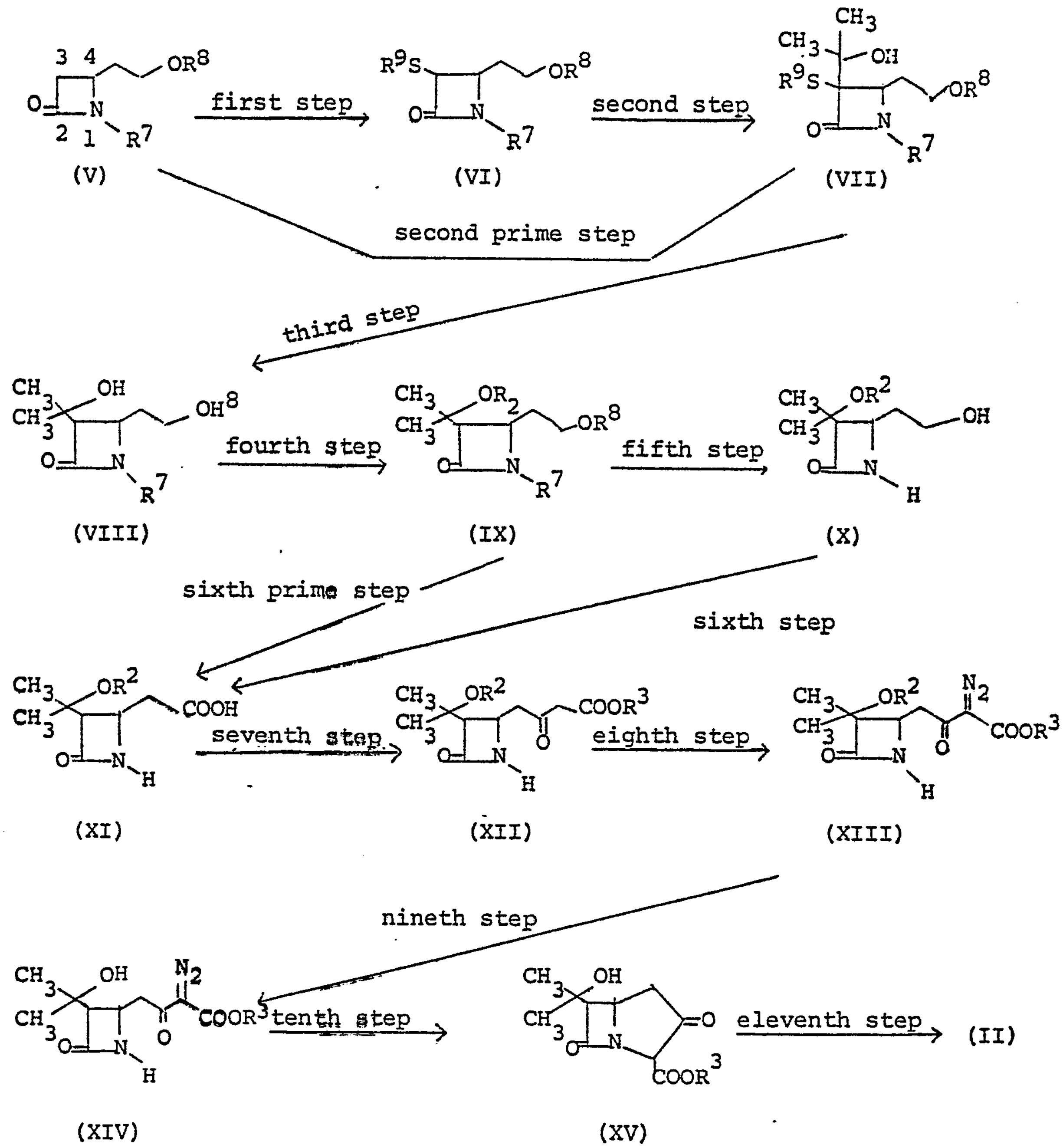


(V) (VI) (VII) (VIII) (IX) (X) (XI) (XII) (XIII) (XIV) (XV) (II)

[wherein $R^2$ and $R^3$ are as defined above; $R^7$ is hydrogen or an amido-protecting group; $R^8$ is hydrogen or hydroxy-protecting group; $R^7$ and $R^8$ are combined with each other to form a hydrocarbon group or a heterocyclic group; and $R^9$ is a hydrocarbon group or a heterocyclic group]

Referring to the above-described scheme, as the starting material, use is made of the compound (V) prepared by

the protection of the amide and hydroxyl groups of 4-(2-hydroxyethyl)azetidin-2-one known in the literature (B. G. Christensen et al.; Journal of Organic Chemistry, vol. 45, pp. 1130 (1980)). The protective groups, $R^7$ and $R^8$, are in no way restricted in the selection thereof, unless they interfere in the subsequent reactions, and are organosilyl groups such as trimethylsilyl and t-butyldimethylsilyl or cyclic ethers such as 2-tetrahydropyranyl. Furthermore, $R^7$ and $R^8$ can bond simultaneously to form a compound of the following formula:

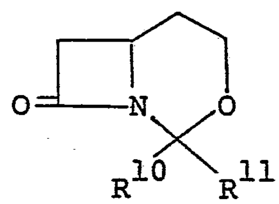

(XVI)

[wherein $R^{10}$ and $R^{11}$ are the same or different and each represent a hydrocarbon group]. As the hydrocarbon groups represented by $R^{10}$ and $R^{11}$, use is made of those described for the above $R^1$, and among others, frequent use may be made of lower alkyl groups such as methyl, ethyl and isopropyl and further groups comprising $R^{10}$ and $R^{11}$ linked through a alkylene group having 3 to 6 carbon atoms. A compound of (XVI) where $R^{10}$ and $R^{11}$ both are methyl groups has been already described in the literature (B.G. Christensen, et al.; Journal of Organic Chemistry, vol. 45, pp. 1130 (1980)), and is obtained by reacting 4-(2-hydroxyethyl)-azetidin-2-one with 2,2-dimethoxypropane in the presence of an acid catalyst such as boron trifluoride etherate and p-toluenesulfonic acid. Other compounds of (XVI) are easily obtained by employing ketones (e.g., diethyl ketone, methyl ethyl ketone, diisopropyl ketone, cyclopropanone, cyclobutanone, cyclopentanone, cyclohexanone, etc.) in place of 2,2-dimethoxypropane in the above-described

reaction.

It is preferable to use (XVI) in the subsequent reaction as the compound represented by the formula (V).

The first reaction step is the step where the 3-position of (V) is sulfenylated to give (VI).

In carrying out the reaction of this reaction step, 1 mole of (V) is preferably reacted with 1 mole of a sulfenylating agent, under the presence of 1 to 3 moles of a strong base such as lithium diisopropylamide, lithium isopropylcyclohexylamide, sodium amide and potassium amide, in a solvent such as tetrahydrofuran (THF), dimethoxyethane, ether, dimethylformamide (DMF) and dimethylsulfoxide (DMSO) at temperatures of -78°C to 0°C to yield (VI). As the sulfenylating agent, use can be made of disulfides such as those of the formula $(R^9S)_2$, substituted thiosulfonates such as those of the formula $R^9S-SO_2R^9$, and N-substituted thioimides such as those of the formula 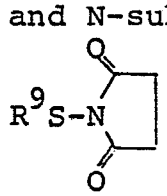 or

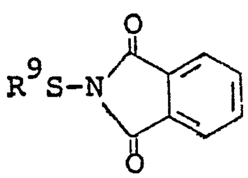 [wherein $R^9$ is as defined above].

As $R^9$, use is made of groups as described for the above-mentioned $R^1$, and particularly, frequent use can be made of aryl groups such as phenyl, lower-alkyl (e.g., methyl, ethyl, etc.) substituted phenyl and halogeno (e.g., chlorine, bromine, etc.) substituted phenyl groups, a 5 to 8 membered heterocyclic group containing 1 to 4 hetero-atoms such as 2-pyridyl and 2-benzothiazol groups, lower alkyl groups such as methyl, ethyl, isopropyl and n-propyl groups, and others. It is theoretically possible that (VI) exists in two kinds of diastereomers with relation to the substituent in the 3-position. These isomers can be isolated by the procedures such as column chromatography and recrystallization, and each of these can be used in the

subsequent reaction, though they can be used as such, without being isolated, in the subsequent reaction.

The second reaction step is the step where the 3-position of (VI) is alkylated to produce (VII).

In carrying out the reaction of this step, 1 mole of (VI) is treated with 1 mole to 20 moles of a ketone at temperatures ranging from -78°C to 0°C, with use of the same strong base and solvent as employed in the first reaction step, to give (VII). The above conversion reactions of (V) → (VI) → (VII) can be accomplished, without isolation of (VI), to effect the conversion of (V) → (VII) (indicated as the second prime reaction step in the above scheme) in the same reaction vessel. In such case, (V) is treated at temperatures ranging from -78°C to 0°C, with use of the same strong base and solvent as employed in the first reaction step, and 1 mole of a sulfenylating agent per 1 mole of (V) is added, followed by addition of 1 mole to 20 moles of acetone, to produce (VII). The amount of the strong base to be used is suitably 2 moles to 3 moles per 1 mole of (V). It is theoretically possible that the resultant (VII) exists in two kinds of diastereomers relative to the substituent in the 3-position. In this reaction, these isomers can be isolated by the procedures such as column chromatography and recrystallization, and each of them can be used in the subsequent reaction, though they can be used, without being isolated, in the form of the mixture, in the subsequent reaction.

The third reaction step is the step where (VII) is reduced to produce selectively 3,4-cis-substituted azetidinone (VIII).

In carrying out the reaction of this reaction step, as the reducing agent, use is for example made of organic tin compounds, nickel compounds, mercury compounds, zinc compounds, etc., and the reaction is preferably conducted by use of organotin compounds of the following formula:

$R^{12}SnH_3$, $R^{12}_2SnH_2$ or $R^{12}_3SnH$

[wherein $R^{12}$ is a hydrocarbon group]. As the hydrocarbon group represented by $R^{12}$, use is made of those as mentioned for $R^1$, and particularly, use can be made of lower alkyl groups having 1 to 6 carbon atoms such as methyl, ethyl, n-propyl, isopropyl, n-butyl, t-butyl and n-pentyl, phenyl and lower-alkyl (e.g., methyl, ethyl, etc.) substituted phenyl groups. Specifically, use can be made of triphenyltin hydride, tri-n-butyltin hydride, diphenyltin hydride, din-butyltin hydride, triethyltin hydride, trimethyltin hydride and n-butyltin hydride, and particularly, the compounds such as triphenyltin hydride and tri-n-butyltin hydride are preferable. The reducing agent is employed normally in the proportions of 1 mole to 10 moles, preferably 1.2 to 5 moles, per 1 mole of (VII). This reaction is suitably carried out in the presence of 0.1 mole to 0.5 mole of a free radical initiator such as azobis-isobutyronitrile or di-t-butyl peroxide. Also, light irradiation may be effected in place of the use of the free radical initiator. The reaction is normally carried out in a solvent not participating in the reaction, although the reducing agent itself may be used as solvent. As the solvent, use can be made of ketones such as acetone and methyl ethyl ketone, ethers such as dioxane and tetrahydrofuran, alcohols such as methanol and ethanol, aromatic hydrocarbons such as benzene, toluene and xylene, and others. The reaction temperature is normally 0°C to 130°C, especially suitably 10°C to 100°C, and the reaction can be conducted in an atmosphere of inert gas such as nitrogen or argon, if necessary. The reaction time varies with the type of starting compounds, reaction temperature, used amount of a reducing agent, etc., and is about 1 to 24 hours. This reaction can produce preferentially the objective compound (VIII) having the 3,4-cis configuration, but is accompanied with the production of a small amount of the by-product

compound having the 3,4-trans configuration. Isolation of (VIII) from the reaction mixture is effected in accordance with the conventional procedures. For example, (VIII) can be easily isolated by distilling off the solvent and subjecting the residue to recrystallization or column chromatography.

The fourth reaction step is the step where the hydroxyl group present in (VIII) is protected to produce (IX).

As the protective group represented by $R^2$, use is made of those described above, and protective groups capable of forming an ether linkage such as β-methoxyethoxymethyl, methoxymethyl and methylthiomethyl groups are suitable. Out of these, especially, the groups such as β-methoxyethoxymethyl group are preferable as the protective group for (VIII). Reaction of introducing a protective group is accomplished by the known procedures, and the above-described protective groups capable of forming an ether linkage are introduced, for example, by reacting an alkyl halide such as β-methoxyethoxymethyl chloride, methoxymethyl chloride and methylthiomethyl chloride with (VIII), under the presence of a base such as triethylamine, diisopropylethylamine, pyridine, n-butyllithium and sodium hydride, in a solvent such as dichloromethane, chloroform, carbon tetrachloride, THF, ether, dimethoxyethane, acetonitrile, DMF and DMSO at temperatures ranging from -20°C to 100°C for 0.5 to 72 hours. The amount of the alkylating agent and base to be used vary with their types, starting compounds, sovent, reaction temperature, etc., and each are 1 mole to 20 moles, suitably 1 mole to 5 moles, per 1 mole of (VIII).

(IX) thus obtained can also be obtained from (VII) via (XVII) as shown by the following reaction scheme.

(VII) → 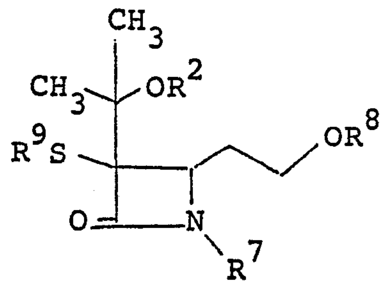 → (IX)

(XVII)

[wherein the symbols in the formula are as defined above]. In the above-described reaction, the hydroxyl group of (VII) is in the first place protected to give (XVII), and then reduction is effected to yield (IX). The conversion from (VII) to (XVII) is accomplished by substantially the same procedure as in the conversion from (VIII) to (IX). Also, the conversion from (XVII) to (IX) is achieved by the substantially the same procedure as in the conversion from (VII) to (VIII).

The fifth reaction step is the step where the protective groups, $R^7$ and $R^8$, for (IX) are removed to give (X). Removal of the protective groups $R^7$ and $R^8$ is conducted by applying a variety of known procedures of removing protective groups, and in the case of (IX) being the compound derived from (XVI), for example, the removal of the protective groups is carried out by the acid hydrolysis in a solvent such as aqueous acetic acid at temperatures of 0°C to 75°C for a reaction time of 5 minutes to 16 hours.

The sixth reaction step is the step where the hydroxyethyl group of (X) is converted by oxidation reaction into the carboxymethyl group to produce (XI).

The oxidation reaction can be conducted by employing various known oxidation systems such as Jones reagent, potassium permanganate and silver oxide in a solvent such as acetone, aqueous THF and aqueous dioxane over the temperature range of -10°C to 40°C for 10 minutes to 24 hours. Particularly, it is preferable to subject (X) to

an oxidation reaction with use of Jones reagent in a solvent such as acetone at a temperature of -10°C to 30°C for 10 minutes to 8 hours.

Or, alternatively, at first (X) is converted to a compound of the following formula:

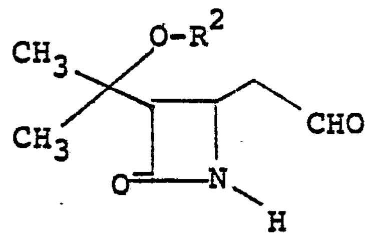

(XVIII)

[wherein the symbols are as defined above], which can then be converted into (XI). The conversion from (X) to (XVIII) is effected by the oxidation with an oxidizing agent such as DMSO-acetic anhydride, chromic anhydride-pyridine and cyclohexylcarbodiimide-DMSO in a solvent such as DMSO, pyridine, acetonitrile and dichloromethane at a temperature of about 0°C to 40°C. The conversion from (XVIII) to (XI) thus is accomplished by the substantially same procedure as in the above conversion from (X) to (XI).

(XI) can also be obtained by subjecting (IX) directly to an oxidation reaction (indicated as the sixth prime reaction step in the scheme). In the case of (IX) being the compound derived from (XVI), for example, (XI) is obtained by subjecting to the oxidation reaction with use of Jones reagent in a solvent such as acetone at a temperature of -10°C to 30°C for 10 minutes to 8 hours. In this reaction, (X) is produced as an intermediate, and the oxidation reaction then proceeds.

The objective compounds (I) of the present invention encompass each of the d-, the ℓ-, and the dl-forms, and although optically active (I) can be produced from optically active (V), it is preferable to effect resolution with use of (XI) in the case of the dl-form. Resolution can be conducted by the various known procedures. For example,

such racemate (d,ℓ-mixture) is resolved by crystallization, or fractional crystallization of diastereomers formed from (XI) and an optically active amine such as quinine, brucine, ephedrine, strychnine and morphine, and physical separation by means of chromatography, etc.

The seventh reaction step is the step where the carboxyl group of (XI) is activated and the chain is extended by two carbons to produce (XII).

In carrying out the reaction of this reaction step, the reaction is conducted by firstly reacting 1 mole of (XI) with 1 to 2 moles of a reagent such as 1,1'-carbonyldiimidazole or 1,1'-carbonylbis(2-methylimidazole) in a solvent such as THF and dimethoxyethane at 0°C to 50°C to give imidazolide, and reacting the imidazolide without being isolated with 1 to 3 moles per 1 mole of (XI) of a magnesium salt of a malonic acid derivative of the following formula:

$$(R^3OCOCH_2COO)_2Mg \qquad (XIX)$$

[wherein $R^3$ is as defined above] at 0°C to 50°C for 1 to 48 hours.

The eighth reaction step is the step where (XII) is diazotized to (XIII).

The diazotization reaction is carried out by reacting 1 mole of (XII) with 1 to 2 moles of an azide such as p-carboxybenzenesulfonyl azide, p-toluenesulfonyl azide and methanesulfonyl azide in a solvent such as acetonitrile, dichloromethane and THF in the presence of a base such as triethylamine, diethylamine and pyridine. The reaction temperature is -10°C to 40°C, and the reaction time is normally 1 to 48 hours.

The nineth reaction step is the step where the protective group ($R^2$) in (XIII) is removed to produce (XIV), and it is not essential to conduct the removal of the protective group in this reaction step; in other words, such protective group, if it is pharmaceutically acceptable, may remain, and can be removed in any reaction step

subsequent to this reaction step, if necessary. Such removal of the protective group is carried out by applying the various, known procedures of removing protective groups, and in the case of $R^2$ being a β-methoxyethoxymethyl group, the removal is effected by contacting with a Lewis acid such as titanium tetrachloride and zinc bromide in a solvent such as chloroform, dichloromethane and THF. The amount of the Lewis acid to be used is 1 to 30 moles per 1 mole of (XIII), and the reaction temperature is -10°C to 40°C, while the reaction time is 5 minutes to 10 hours.

The tenth reaction step is the step where (XIV) is cyclised to produce (XV).

The cyclisation reaction is conducted by cyclising (XIV) in a solvent such as benzene, toluene and THF in the presence of a catalyst such as copper sulfate, copper powder, rhodium acetate and palladium acetate. The reaction temperature is 50°C to 110°C, while the reaction time is 1 to 5 hours, and the reaction is normally carried out in an atmosphere of inert gas such as nitrogen or argon. Also, this cyclisation reaction can be conducted by irradiating (XIV) with light in a solvent such as benzene, THF, carbon tetrachloride and diethyl ether at -10°C to 40°C for 0.5 to 2 hours.

The eleventh reaction step is the step where (XV) is activated with an activating agent to produce (II).

In the case of X in (II) being a substituted sulfonyloxy group, the reaction is conducted by reacting 1 mole of (XV) with 1 to 3 moles of a sulfonylating agent such as p-toluenesulfonic acid anhydride, p-nitrophenylsulfonic acid anhydride, 2,4-triisopropyl-phenylsulfonic acid anhydride, methanesulfonic acid anhydride, p-toluenesulfonic chloride and p-bromophenylsulfonyl chloride in a solvent such as dichloromethane, chloroform, acetonitrile, dimethoxyethane and THF in the presence of a base such as triethylamine, diisopropylethylamine, pyridine and 4-dimethylaminopyridine. Normally, the reaction temperature is -20°C to

40°C, and the reaction time is 0.5 to 5 hours. In the case of X in (II) being halogen, the reaction is carried out under the same conditions with use of a halogenating agent (e.g., oxalyl chloride, $(C_6H_5)_3PCl_2$, $(C_6H_5)_3PBr_2$, $(C_6H_5O)_3PBr_2$, thionyl chloride, etc.) in place of the sulfonylating agent employed in the reaction in the case of the above X being a substituted sulfonyloxy group, and thus, (XV) can be converted into (II). In the case of X in (II) being a disubstituted phosphoryloxy group, also, the reaction is perfomed under the same conditions with use of a phosphorylating agent (e.g., diphenyl chlorophosphate (diphenylphosphoryl chloride), dimethyl chlorophosphate, diethyl chlorophosphate, etc.) in place of the sulfonylating agent employed in the reaction in the case of the above X being a substituted sulfonyloxy group, and (XV) can be converted into (II).

After the completion of the reaction, the objective compound (II) of this reaction step is recovered from the reaction mixture by the conventional procedures. The compound, for example, can be obtained by adding to the reaction mixture an organic solvent immiscible with water such as ethyl acetate and water, separating a organic solvent layer to wash with water, drying with a drying agent and distilling off the solvent. Also, (II) can be subjected, without the above described isolation procedure, as such to the reaction in the subsequent reaction step.

The above-described example is not intended to limit the starting material (II), and use can be made of any starting compound (II), if it is suited for the purpose of this invention. Specific examples of such compound are illustrated below as the refernece example.

The following examples, reference examples and test example are given to illustrate this invention in further detail. In these examples, nuclear magnetic resonance (NMR) spectra were measured with Varian XL-100 (100 MHz), EM-390 (90 MHz) or T-60 (60 MHz) instrument, with tetramethylsilane as reference standard, and the δ values are shown in ppm. In the chemical shift data, s means a singlet, br.s. a broad singlet, d a doublet, dd a double doublet, t a triplet, q a quartet, m a multiplet, ABq an AB pattern quartet, J a coupling constant, THF tetrahydrofuran, DMF dimethylformamide, DMSO dimethyl sulfoxide, br. or b. a broad, arom. aromatic, Ar: aromatic group. Infrared (IR) spectra are measured with a Hitachi 215 spectrophotometer; Ultraviolet (UV) spectra with a Perkin-Elmer 450 or a Hitachi EPS-3T spectrometer; and Mass spectra with a JEOL JMS-OISC mass spectrometer.

In silica gel column-chromatography, Kiesel Gel 60 (Art 9385, 230-400 Mesh, Merck Co., W. Germany) is employed. In Florisil column-chromatography, Florisil (100-200 mesh, produced by Wako Pure Chemical Co., Japan) is employed. The elution in the both chromatography is monitored by thin layer chromatography (TLC) with precoated silica gel plate (Kiesel gel $60F_{254}$, Merck, W. Germany). The TLC is developed with the solvent which is the same as the eluent employed in the column-chromatography and UV detector.

In Amberlite XAD-2 (produced by Rhom & Haas Co., U.S.A.) or Diaion HP20 (produced by Mitsubishi Chemical Ind. Ltd., Japan) column-chromatography, the elution in the chromatography is monitored by UVICORD (produced by LKB, Sweden).

Reference Example 1

- 7-Phenylthio-8-oxo-2,2-dimethyl-3-oxa-1-azabicyclo-[4.2.0]octane -

A 42 mℓ (0.067 mol) portion of 15% n-butyllithium/hexane is added with stirring to a solution of 8.82 mℓ (0.063 mole) of diisopropylamine in 140 mℓ of anhydrous tetrahydrofuran under nitrogen atomosphere at -78°C. A solution of 4.65 g (0.03 mol) of 8-oxo-2,2-dimethyl-3-oxa-1-azabicyclo[4.2.0]ocatane in 30 mℓ of anhydrous tetrahydrofuran is added to the solution over a 20-minute period under stirring. 10 minutes later, a solution of 6.54 g (0.03 mol) of diphenyl disulfide in 30 mℓ of anhydrous tetrahydrofuran is added to the mixture over a 25-minute period while maintaining the internal temperature at below -73°C. After the resultant mixture is stirred for 10 minutes, it is poured in saturated aqueous ammonium chloride (150 mℓ) under ice cooling, and the tetrahydrofuran layer is separated, while the water layer is extracted with ethyl acetate. The organic layers are combined, washed with 0.5N NaOH and water successively, dried ($Na_2SO_4$) and the solvent is distilled off. The residue is treated with isopropyl ether - hexane, and there is obtained the trans form of the subject compound as colorless crystals (5.72 g). The mother liquor of crystallization is concentrated, and the residue is chromatographed on a column

of silica gel (hexane - ethyl acetate = 4 : 1), thus giving further 1.33 g (combined yield of 7.05 g, 89.3%) of colorless crystals. m.p. 49 to 51°C.

IR $\nu_{max}^{Nujol}$ $cm^{-1}$: 1740

NMR (60MHz, $CDCl_3$)δ: 1.20, 1.69 (each 3H, s x 2, $-CH_3$ x 2), 1.9 (2H,m,$-CH_2-$), 3.4 (1H,m, CH-N<), 3.77 (2H,m,$-CH_2O-$), 3.95 (1H,d,J=2Hz,$^{-S}$>CHCO-), 7.1 - 7.5 (5H,m,arom.H)

Elemental analysis, for $C_{14}H_{17}NO_2S$

Calcd.: C, 63.85; H, 6.51; N, 5.32

Found : C, 64.00; H, 6.43; N, 5.18

The NMR spectrum of the oily material (0.16 g) obtained by concentrating the above-described mother liquor of crystallization for the additional trans-form revealed the absorption (in $CDCl_3$, δ 4.60, J=5Hz, $C_{(7)}H$) which is attributable to the cis-form of the subject compound, in addition to the above-mentioned absorptions ascribed to the trans-form.

Reference Example 2

- 7-(1-Hydroxy-1-methylethyl)-7-phenylthio-8-oxo-2,2-dimethyl-3-oxa-1-azabicyclo[4.2.0]octane -

(1) Production from 7-phenylthio-8-oxo-2,2-dimethyl-3-oxa-1-azabicyclo[4.2.0]octane.

A 15 mℓ (24.0 mmol) portion of 15% n-butyllithium/hexane is added with stirring to a solution of 3.16 mℓ (22.5 mmol) of diisopropylamine in 80 mℓ of anhydrous tetrahydrofuran under nitrogen atmosphere at -78°C. A solution of 3.95 g (15 mmol) of trans-7-phenylthio-8-oxo-2,2-dimethyl-3-oxa-1-azabicyclo[4.2.0]octane in 15 mℓ of anhydrous tetrahydrofuran is added to the solution at -78°C, and the mixture is stirred for 10 minutes. Then, after 5 mℓ of anhydrous acetone is added and stirring is continued for 30 minutes, the reaction solution is added under stirring to acetic acid (4 mℓ) while maintaining the internal temperature at below 5°C, and the organic layer is separated. After the water layer is extracted with ethyl acetate, the organic layers are combined, washed with water, dried ($Na_2SO_4$), and the solvent is distilled off. The residue is

treated with isopropyl ether and hexane, and there is thus obtained the subject compound as colorless crystals (3.50 g) of the mixture of its isomers A and B (A-B ratio = about 2.4:1 as determined from the NMR spectrum). The mother liquor of crystallization is concentrated and the residue is chromatographed on a column of silica gel (hexane-ethyl acetate ratio = 4:1, 1:1), giving the starting material (0.16 g as recovered) and 1.0 g (combined yield of 4.50 g, 97.2%, as determined on the basis of unrecovered starting material) of the subject compound (isomer A) as colorless crystals, respectively. Part of the mixture of the isomers A and B is chromatographed on a column of silica gel (hexane-ethyl acetate ratio = 4:1, 1:1), thus yielding the isomers A and B separated as colorless crystals, respectively. Their physical properties are as follows:

Isomer A (main product):

m.p. 95-97°C.

IR $\nu_{max}^{Nujol}$ $cm^{-1}$: 3470, 1725

NMR (100MHz, $CDCl_3$) δ: 1.33, 1.40(each 3H, s x 2,$-CH_2$ x 2), 1.56 (6H,s,$-CH_3$x2), 1.70(1H,m,$>C<\frac{H}{\underline{H}}$), 2.30(1H,m,$>C<\frac{H}{\underline{H}}$), 3.7-4.1 (3H,m,>CH-N< , $-CH_2O-$)

Elemental analysis, for $C_{17}H_{23}NO_3S$

Calcd.: C, 63.52; H, 7.21; N, 4.36

Found : C, 63.49; H, 7.25; N, 4.45

Isomer B (minor product):

m.p. 188-189°C

IR $\nu_{max}^{KBr}$ $cm^{-1}$: 3430, 1720

NMR (100MHz,$CDCl_3$) δ: 0.65, 1.42, 1.58, 1.68 (each 3H, s x 4, $-CH_3$x4), 1.75(1H,m,$>C<\frac{H}{\underline{H}}$), 2.92(1H,m,$>C<\frac{H}{\underline{H}}$), 3.4-3.8(3H,m >CH-N<,$-CH_2O-$).

Elemental analysis, for $C_{17}H_{23}NO_3S$

Calcd.: C, 63.52; H, 7.21; N, 4.36

Found : C, 63.35; H, 7.13; N, 4.46

(2) Production from 8-oxo-2,2-dimethyl-3-oxa-1-azabicyclo-[4.2.0]octane.

A 8.4 mℓ (13 mmol) portion of 15% n-butyllithium/hexane

is added to a solution of 1.74 mℓ (12 mmol) of diisopropylamine in 28 mℓ of anhydrous tetrahydrofuran under atmospheric pressure at -78°C. A solution of 0.93 g (6 mmol) of 8-oxo-2,2-dimethyl-3-oxa-1-azabicyclo[4.2.0]octane in 6 mℓ of tetrahydrofuran is added under stirring to the solution over a 10 minute period, and stirring is continued for 5 minutes. A solution of 1.31 g (6 mmol) of diphenyl disulfide in 6 mℓ of anhydrous tetrahydrofuran is added dropwise to the mixture over a 15-minute period, while maintaining the internal temperature at below -73°C, and stirring is continued for 5 minutes. Further, 2 mℓ of anhydrous acetone is added, followed by stirring for 5 minutes. This reaction mixture is gradually added to a mixed solution of saturated aqueous ammonium chloride (50 mℓ) and ethyl acetate (30 mℓ) under cooling (-10°C) with stirring, and the organic layer is separated, while the water layer is extracted with ethyl acetate. The organic layers are combined, washed with 0.5N NaOH and water successively, dried ($Na_2SO_4$) and the solvent is distilled off. The residue is treated with hexane, and there is thus obtained the subject compound (isomer mixture. ca. 20:1 mixture as determined from the NMR spectrum) as colorless crystals (1.36 g). The mother liquor of crystallization is concentrated, and the residue is chromatographed on a column of silica gel (hexane-ethyl acetate ratio = 4:1, 1:1), thus giving 0.04g of trans-7-phenylthio-8-oxo-2,2-dimethyl-3-oxa-1-azabicyclo[4.2.0]octane, 0.03 g of the isomer B and 0.13 g of the isomer A of the subject compound (combined yield of 1.52 g, 79.0%) separated as colorless crystals, respectively.

## Reference Example 3

- 7-Methylthio-8-oxo-2,2-dimethyl-3-oxa-1-azabicyclo[4.2.0]-octane -

A 1.0 mℓ (1.6 mmol) portion of 15% n-butyllithium/hexane is added stirring to a solution of 0.25 mℓ (1.4 mmol) with of diisopropylamine in 5 mℓ of anhydrous tetrahydrofuran under nitrogen atmosphere at -78°C. A solution of 155 mg

(1 mmol) of 8-oxo-2,2-dimethyl-3-oxa-1-azabicyclo[4.2.0]-octane in 1 mℓ of anhydrous tetrahydrofuran is added to the solution at -78°C, and stirring is continued for 10 minutes. Then, after 0.1 mℓ of dimethyl disulfide is added and stirring is continued for 15 minutes, the reaction solution is poured into saturated aqueous ammonium chloride under ice-cooling, and the mixture is extracted with ethyl acetate. The extract is washed with water, dried ($Na_2SO_4$) and the solvent is distilled off. The residue is chromatographed on a column of silica gel (hexane-ethyl acetate ratio = 3:1), giving the subject compound (isomer mixture) as a colorless oily substance (140 mg, 70%).

IR $\nu_{max}^{Neat}$ $cm^{-1}$: 1740

NMR (60MHz, $CDCl_3$) δ: 1.23, 1.75(each 3H, s x 2, $-CH_3$x2), 1.3 (2H,m,$-CH_2-$), 2.18(3H,s,$-CH_3$), 3.4-4.3(4H,m,-S-CH<,>CH-N<, $-CH_2O-$)

Reference Example 4

- 7-(1-Hydroxy-1-methylethyl)-7-methylthio-8-oxo-2,2-dimethyl-3-oxa-1-azabicyclo[4.2.0]octane -

A 1.0 mℓ (1.6 mmol) portion of 15% n-butyllithium/hexane is added with stirring to a solution of 0.25 mℓ(1.4 mmol) of diisopropylamine in 5 mℓ of anhydrous tetrahydrofuran under nitrogen atmosphere at -78°C. A solution of 140 mg (0.7 mmol) of 7-methylthio-8-oxo-2,2-diemthyl-3-oxa-1-azabicyclo[4.2.0]octane as obtained in Reference Example 3 in 1 mℓ of anhydrous tetrahydrofuran is added to the solution at -78°C and stirring is continued for 10 minutes. Then, after 0.5 mℓ of anhydrous acetone is added and stirring is continued for 15 minutes, the reaction solution is poured into saturated aqueous ammonium chloride under ice-cooling, and the mixture is extracted with ethyl acetate. The extract is washed with water, dried ($Na_2SO_4$) and the solvent is distilled off. The residue is chromatographed on a column of silica gel (hexane-ethyl acetate ratio = 3:1), thus giving two isomers (A and B) of the subject compound.

Isomer A: colorless crystals, yield, 95 mg (52.8%).

m.p. 95-97°C

IR $\nu_{max}^{Nujol} cm^{-1}$: 3410, 1720

NMR (60MHz, $CDCl_3$)δ: 1.27, 1.43, 1.48, 1.72 (each 3H, s x 4,$-CH_3x4$), 1.8(2H,m,$-CH_2-$), 2.35(3H,s,$-SCH_3$), 3.7-4.0 (3H,m,>CH-N<,$-CH_2O-$).

Elemental analysis, for $C_{12}H_{21}NO_3S$

Calcd.: C, 55.57; H, 8.16; N, 5.40

Found : C, 55.23; H, 8.00; N, 5.49

Isomer B: colorless, oily substance, yield, 10 mg (5.6%).

IR $\nu_{max}^{Neat} cm^{-1}$: 3450, 1740

NMR (60MHz, $CDCl_3$)δ: 1.37, 1.47, 1.63, 1.80(each 3H, s x 4, $-CH_3x4$), 1.9(2H,m,$-CH_2-$), 2.23(3H,s,$-SCH_3$), 3.7-4.1(3H,m, >CH-N<,$-CH_2O-$).

## Reference Example 5

- 7-(2-pyridylthio)-8-oxo-2,2-dimethyl-3-oxa-1-azabicyclo-[4.2.0]octane -

A 2.7 mℓ (4.3 mmol) portion of 15% n-butyllithium/hexane is added with stirring to a solution of 0.56 mℓ (4.0 mmol) of diisopropylamine in 10 mℓ of anhydrous tetrahydrofuran under nitrogen atmosphere at -78°C. A solution of 310 mg(2.0 mmol) of 8-oxo-2,2-dimethyl-3-oxa-1-azabicyclo-[4.2.0]octane in 2 mℓ of anhydrous tetrahydrofuran is added to the solution. Then, a solution of 440 mg (2.0 mmol) of di(2-pyridyl) disulfide in 2 mℓ of anhydrous tetrahydrofuran is added, and stirring is continued for 15 minutes. The reaction solution is poured into saturated aqueous ammonium chloride under ice-cooling, and the mixture is extracted with ethyl acetate. The extract is washed with 1N NaOH and water successively, dried ($Na_2SO_4$), and the solvent is distilled off. The residue is chromatographed on a column of silica gel (ethyl acetate-hexane, ratio = 1:2), thus giving the subject compound (the trans-isomer) as an oily substance (396 mg, 74.9%).

IR $\nu_{max}^{Neat}$ $cm^{-1}$: 1750

NMR (60MHz, $CDCl_3$)δ: 1.41, 1.80(each 3H,s x 4,$-CH_3x2$), 2.0(2H, m,$-CH_2-$), 3.6(1H,m,>CH-N<), 3.85(2H,m,$-CH_2O-$), 4.66(1H,d,

J=2Hz,>CH-CO-), 6.9-7.8(4H,m,arom.H).

Reference Example 6

- 7-(1-Hydroxy-1-methylethyl)-7-(2-pyridylthio)-8-oxo-2,2-dimethyl-3-oxa-1-azabicyclo[4.2.0]octane -

A 3 mℓ (4,8 mmol) portion of 15% n-butyllithium/hexane is added with stirring to a solution of 0.63 mℓ (4.5 mmol) of diisopropylamine in 17 mℓ of anhydrous tetrahydrofuran under nitrogen atmosphere at -78°C. A solution of 793 mg (3.0 mmol) of 7-(2-pyridylthio)-8-oxo-2,2-dimethyl-3-oxa-1-azabicyclo[4.2.0]octane in 4 mℓ of anhydrous tetrahydrofuran is added to the solution at -78°C, and then 1.0 mℓ of anhydrous acetone is added, followed by stirring for 30 minutes. The reaction solution is poured into an aqueous solution (30 mℓ) containing acetic acid (0.8 mℓ) under stirring. Sodium hydrogen carbonate is added to the mixture to adjust to pH 7, and the mixture is extracted with ethyl acetate. The extract is washed with saturated aqueous sodium chloride, dried ($Na_2SO_4$), and the solvent is distilled off. The residue is chromatographed on a column of silica gel (ethyl acetate-hexane ratio = 1:2, 2:1), thus giving the subject compound (isomer mixture) as slightly yellow crystals (773 mg, 80.0%).

m.p. 124-125°C (decomp.)

IR $\nu_{max}^{KBr}$ $cm^{-1}$: 1735

NMR (90MHz, $CDCl_3$)δ: 1.30, 1.4, 1.64, 1.70(each 0.9H, s x 4, $-CH_3$x4), 1.39, 1.41, 1.49, 1.71 (each 2.1H, s x 4, $-CH_3$x4), 1.0-1.8(1H,m,>C$\frac{H}{H}$), 1.8-2.3(1H,m,>C<$^H_H$), 3.72(2H,m,$-CH_2O-$), 4.15 1H,dd,J=10,5Hz,>CH-N<), 7.0-7.8(3H,m,arom.H), 8.3(1H,m,arom.H).

Elemental analysis, for $C_{16}H_{22}N_2O_3S$

Calcd.: C, 59.60; H, 6.87; N, 8.68

Found : C, 59.49; H, 7.17; N, 8.61

Reference Example 7

- 7-[1-(2-Methoxyethoxymethoxy)-1-methylethyl]-7-phenylthio-8-oxo-2,2-dimethyl-3-oxa-1-azabicyclo[4.2.0]octane -

To a solution of 369 mg (1.15 mmol) of 7-[1-hydroxy-1-methylethyl]-8-oxo-2,2-dimethyl-3-oxa-1-azabicyclo[4.2.0]-octane (the isomer A as obtained in (1) of Reference Example 2 in 6 mℓ of methylene chloride are added 0.60 mℓ (3.45 mmol) of diisopropylethylamine and 0.39 mℓ (3.42 mmol) of 2-methoxyethoxymethyl chloride, and stirring is continued under nitrogen atmosphere at room temperature for 5 hours. After 0.20 mℓ (1.15 mmol) of diisopropylethylamine and 0.13 mℓ (1.13 mmol) of 2-methoxyethoxymethyl chloride are further added and the mixture is allowd to stand at room temperature for 14 hours, the reaction solution is washed with water, 2% aqueous acetic acid and water successively, dried ($Na_2SO_4$) and the solvent is distilled off. The residue is chromatographed on a column of silica gel (ethylether-hexane ratio = 1:1, 3:1), thus giving 132 mg of the starting material (recovered) and 264 mg of the subject compound (87.4% as determined on the basis of unrecovered starting material; colorless, oily substance), respectively.

IR $\nu_{max}^{Neat}$ $cm^{-1}$: 1750

NMR (60 Hz, $CDCl_3$) δ: 1.40(6H,s,$-CH_3$x2), 1.51, 1.62 (each 3H, s x 2, $-CH_3$x2), ca.1.8(2H,m,$-CH_2-$), 3.41(3H,s,$-OCH_3$), 3.47-4.3 (7H,m,>CH-N<,$-OCH_2-$x3), 4.83(2H,s,$-OCH_2O-$), 7.1-7.9(5H,m, arom.H).

## Reference Example 8

- 7-(1-Hydroxy-1-methylethyl)-8-oxo-2,2-dimethyl-3-oxa-1-azabicyclo[4.2.0]octane -

(1) Production from 7-(1-hydroxy-1-methylethyl)-7-phenylthio-8-oxo-2,2-dimethyl-3-oxa-1-azabicyclo[4.2.0]octane (the isomer A) and tri-n-butyltin hydride.

A solution of 160 mg (0.5 mmol) of the isomer A as obtained in Reference Example 2, 16 mg (0.1 mmol) of azobis-isobutyronitrile and 0.435 mℓ (1.6 mmol) of tri-n-butyltin hydride in 5 mℓ of acetone is refluxed under nitrogen atmosphere for 16 hours. The solvent is distilled off, and the residue is chromatographed on a column of silica gel (hexane-ethyl acetate ratio = 1:1), thus giving 77 mg (72.2%) of the cis-form of, and 24 mg (22.5%) of the trans-form of, the subject compound separated as colorless crystals, respectively.

cis-form: m.p. 125-127°C.

IR $\nu_{max}^{Nujol}cm^{-1}$: 3450, 1720

NMR (60MHz, $CDCl_3$)δ: 1.30, 1.43, 1.50, 1.80(each 3H, s x 4, $-CH_3$x4), 1.9(2H,m,$-CH_2-$), 3.27(1H,d,J=6Hz,>CH-CO-), 3.7-4.1(3H,m,>CH-N<,$-CH_2O-$)

Mass spectrometry, m/e: 213($M^+$)

Elemental analysis, for $C_{11}H_{19}NO_3$

Calcd.: C, 61.94; H, 8.98; N, 6.57

Found : C, 61.68; H, 8.85; N, 6.73

trans-form: m.p. 102-104°C.

IR $\nu_{max}^{Nujol}cm^{-1}$: 3450, 1720

NMR (60MHz, $CDCl_3$)δ: 1.30, 1.37, 1.43, 1.77(each 3H, s x 4, $-CH_3$x4), 1.9(2H,m,$-CH_2-$), 2.91(1H,d,J=2Hz,>CH-CO<), 3.5-4.1 (3H,m,>CH-N<, $-CH_2O-$).

Elemental analysis, for $C_{11}H_{19}NO_3$

Calcd.: C, 61.94; H, 8.98; N, 6.57

Found : C, 62.07; H, 8.90; N, 6.52

(2) Production from 7-(1-hydroxy-1-methylethyl)-7-phenylthio-8-oxo-2,2-dimethyl-3-oxa-1-azabicyclo[4.2.0]octane(the isomer B) and tri-n-butyltin hydride.

A solution of 80 mg (0.25 mmol) of the isomer B as obtained in Reference Example 2, 8 mg (0.05 mmol) of azobisisobutyronitrile and 0.22 mℓ (0.8 mmol) of tri-n-butyltin hydride in 3 mℓ of acetone is heated and refluxed under nitrogen atomosphere for 5 hours. The solvent is distilled off, and the residue is chromatographed on a column of silica gel (hexane-ethyl acetate ratio = 1:1), thus giving 37 mg (70.0%) of the cis-form of, and 12 mg (22.6%) of the trans-from of, the subject compound obtained as colorless crystals, respectively.

(3) Production from 7-(1-hydroxy-1-methylethyl)-7-phenylthio-8-oxo-2,2-dimethyl-3-oxa-1-azabicyclo[4.2.0]octane (mixture of the isomers A and B) and tri-n-butyltin hydride.

A solution of 20.0g (62 mmol) of the mixture of the isomers A and B (ca.3.4:1 ratio) obtained in Reference Example 2, 2.0g (12 mmol) of azobis-isobutyronitrile and 56.4 mℓ (210 mmol)

of tri-n-butyltin hydride in 500 mℓ of acetone is heated and refluxed under nitrogen atmosphere for 16 hours. The solvent is distilled off, and chloroform (100 mℓ) is added to the residue, followed by filtering out insoluble matter with celite. The filtrate is concentrated, and the residue is treated with isopropyl ether, thus yielding 8.13 g of the cis-form of the subject compound as colorless crystals. The mother liquor of crystallization is concentrated, and the residue is chromatographed on a column of silica gel (hexane-ethyl acetate ratio = 1:1), giving further 1.70 g (combined yield of 9.83 g (74.1%)) of the cis-form and 2.61g (19.7%) of the trans-form obtained as colorless crystals, respectively.

(4) Production from 7-(1-hydroxy-1-methylethyl)-7-phenylthio-8-oxo-2,2-dimethyl-3-oxa-1-azabicyclo[4.2.0]octane (mixture of the isomers A and B) and triphenyltin hydride.

A solution of 150 mg (0.46 mmol) of the mixture of the isomers A and B (ca. 20:1 ratio) as obtained in Reference Example 2, 15 mg (0.09 mmol) of azobis-isobutyronitrile and 0.32 g (0.92 mmol) of triphenyltin hydride in 4 mℓ of acetone is heated and refluxed under nitrogen atmosphere for 22 hours. The solvent is distilled off, and a small amount of chloroform is added to the residue, followed by filtering out insoluble matter with celite. The filtrate is concentrated, and the residue is chromatographed on a column of silica gel (hexane-ethyl acetate ratio = 1:1), thus giving 81 mg (79.7%) of the cis-form, and 17 mg (16.3%) of the trans-form, of the subject compound obtained as colorless crystals, respectively.

(5) Production from 7-(1-hydroxy-1-methylethyl)-7-methylthio-8-oxo-2,2-dimethyl-3-oxa-1-azabicyclo[4.2.0]octane (the isomer A) and tri-n-butyltin hydride.

A solution of 95 mg (0.37 mmol) of the isomer A as obtained in Reference Example 4, 11 mg (0.07 mmol) of azobis-isobutyronitrile and 0.32 mℓ (1.2 mmol) of tri-n-butyltin hydride in 3 mℓ of acetone is heated and refluxed under nitrogen atmosphere for 17 hours. The solvent is distilled off, and the residue is chromatographed on a column of silica

gel (hexane-ethyl acetate ratio = 1:1), thus giving the starting material (recovered, 65 mg), and 17 mg (68.8%, as determined on the basis of unrecovered starting material) of the cis-form, and 4 mg (16.2%, as determined on the basis of unrecovered starting material) of the tras-form, of the subject compound separated as colorless crystals, respectively.

(6) Production from 7-(1-hydroxy-1-methylethyl)-7-(2-pyridylthio)-8-oxo-2,2-dimethyl-3-oxa-1-azabicyclo[4.2.0]octane and tri-n-butyltin hydride.

A solution of 500 mg (1.55 mmol) of 7-(1-hydroxy-1-methylethyl)-7-(2-pyridylthio)-8-oxo-2,2-dimethyl-3-oxa-1-azabicyclo[4.2.0]octane (isomer mixture) as obtained in Reference Example 6, 50 mg (0.3 mmol) of azobis-isobutyronitrile and a solution of 1.45 mℓ (5.3 mmol) of tri-n-butyltin hydride in 13 mℓ of acetone is heated and refluxed under nitrogen atmosphere for 16 hours. The solvent is distilled off, and the residue is chromatographed on a column of silica gel (benzene-acetone ratio = 10:1, 5:1), thus giving the starting material (one isomer; m.p. 162-164°C (decomp.), 130 mg), and 143 mg (67.1%, as determined on the basis of unrecovered starting material) of the cis-form, and 41 mg (19.2%, as determined on the basis of unrecovered starting material) of the trans-form, of the subject compound obtained as colorless crystals, respectively.

The physical properties (melting point, IR and NMR spectra) of the compounds as obtained under the above-described items (2) through (6) were in agreeement with those of the compound as obtained under the above-mentioned item (1).

Reference Example 9

- cis-7-[1-(2-Methoxyethoxymethoxy)-1-methylethyl]-8-oxo-2,2-dimethyl-3-oxa-1-azabicyclo[4.2.0]octane -

(1) Production from cis-7-(1-hydroxy-1-methylethyl)-8-oxo-2,2-dimethyl-3-oxa-1-azabicyclo[4.2.0]octane.

To a solution of 7.00 g (33 mmol) of cis-7-(1-hydroxy-1-methylethyl)-8-oxo-2,2-dimethyl-3-oxa-1-azabicyclo-[4.2.0]octane in 130 mℓ of methylene chloride are added 18.92 mℓ

(109 mmol) of diisopropylethylamine and 12.40 mℓ (109 mmol) of 2-methoxyethoxymethyl chloride, and the mixture is allowed to stand under nitrogen atmosphere at room temperature for 21 hours. The reaction solution is washed with water, 2% aqueous acetic acid, saturated aqueous sodium hydrogen carbonate and water, successively, dried ($Na_2SO_4$) and the solvent is distilled off, thus giving the subject compound quantitatively as a slightly yellow oily substance.

IR $\nu_{max}^{Neat}$ $cm^{-1}$: 1750

NMR (60MHz, $CDCl_3$)δ: 1.27, 1.35, 1.45, 1.65 (each 3H, s x 4, $-CH_3$x4), ca.1.9(2H,m,$-CH_2-$), 3.10(1H,d,J=5Hz,-CHCO-), 3.30 (3H,s,$-OCH_3$), 3.3-3.9(6H,m,$-CH_2O$-x3), 4.78(2H,s,$-OCH_2O-$).

(2) Production from 7-[1-(2-methoxyethoxymethoxy)-1-methylethyl]-7-phenylthio-8-oxo-2,2-dimethyl-3-oxa-1-azabicyclo-[4.2.0]octane.

A solution of 260 mg (0.64 mmol) of 7-[1-(2-methoxyethoxymethoxy)-1-methylethyl]-7-phenylthio-8-oxo-2,2-dimethyl-3-oxa-1-azabicyclo[4.2.0]octane, 21 mg (0.13 mmol) of azobisisobutyronitrile and 0.58 mℓ (2.18 mmol) of tri-n-butyltin hydride in 5 mℓ of acetone is heated and refluxed under nitrogen atmosphere for 17 hours. The solvent is distilled off, and the residue is chromatographed on a column of silica gel (hexane-ethyl acetate ratio = 2:1, 1:2), thus giving the subject compound in the form of its mixture with the trans-form (cis:trans = ca. 78:22, (as determined from the NMR spectrum); colorless oily substance; 164 mg).

Reference Example 10

-cis-4-(2-Hydroxyethyl)-3-[1-(2-methoxyethoxymethoxy)-1-methylethyl]azetidin-2-one -

In a mixed solution of acetic acid (2.4 mℓ) and water (1.2 mℓ) is dissolved 603 mg (2 mmol) of cis-7-[1-(2-methoxyethoxymethoxy)-1-methylethyl]-8-oxo-2,2-dimethyl-3-oxa-1-azabicyclo[4.2.0]octane, and stirring is continued at room temperature for 8 hours. Chloroform is added to the reaction solution, and the solvent is distilled off under reduced pressure.

The residue is treated with ethyl ether, thus giving the subject compound as colorless crystals (345 mg, 66.0%). m.p. 75-76°C.

IR $\nu_{max}^{Nujol}$ $cm^{-1}$: 3280, 3200, 1720.

NMR (90MHz, $CDCl_3$) δ: 1.36, 1.50 (each 3H, s x 2, $-CH_3$x2), 2.0-2.3 (2H,m,$-C\underline{H}_2-CH_2OH$), 2.6 (1H,b,-OH), 3.28 (1H,d,J=6Hz,>CHCO-), 3.37 (3H,s,$-OCH_3$), 3.4-4.0 (7H,m,$-CH_2C\underline{H}_2OH$,>C$\underline{H}$NH-,$-OCH_2CH_2O-$), 4.83 (2H,s,$-OCH_2O-$), 6.6 (1H,b,-NH-).

Elemental analysis, for $C_{12}H_{23}NO_5$

Calcd.: C, 55.15; H, 8.87; N, 5.36

Found : C, 54.75; H, 9.09; N, 5.54

Reference Example 11

- cis-3-[1-(2-Methoxyethoxymethoxy)-1-methylethyl]-4-(2-oxoethyl)azetidin-2-one -

To a solution of 0.32 mℓ of anhydrous pyridine in 6 mℓ of methylene chloride is added 200 mg of chromic anhydride, and the mixture is stirred at room temperature for 15 minutes. To the mixture is added a solution of 110 mg (0.42 mmol) of cis-4-(2-hydroxyethyl)-3-[1-(2-methoxyethoxymethoxy)-1-methylethyl]azetidin-2-one in 1 mℓ of methylene chloride, followed by stirring at room temperature for 15 minutes. The reaction solution is filtered with celite, and washed with methylene chloride. The filtrate is concentrated, and the residue is dissolved in ethyl acetate. Insoluble matter is filtered out again with celite, and the filtrate is concentrated. The residue is chromatographed on a column of silica gel (ethyl acetate), thus giving the subject compound as a colorless oily substance (70 mg, 64%).

IR $\nu_{max}^{Neat}$ $cm^{-1}$: 3280, 1750, 1720

NMR (60MHz, $CDCl_3$) δ: 1.33, 1.51 (each 3H, s x 2, $-CH_3$x2), 3.38 (3H,s,$-OCH_3$), 3.2-4.3 (8H,m,>CHCO-,>CH-N<,$-CH_2CO-$,$-CH_2O-$x2), 4.85 (2H,s,$-OCH_2O-$), 6.4 (1H,b,-NH-), 9.96 (1H,s,-CHO).

Reference Example 12

- 2,2-Cyclohexyl-8-oxo-3-oxa-1-azabicyclo[4.2.0]octane -

To a solution of 2.0 g (17.3 mmol) of 4-(2-hydroxyethyl)-2-azetidinone and 2.5 g (25.5 mmol) of cyclohexanone in 25 mℓ of anhydrous methylene chloride is added 0.2 mℓ (1.98 mmol) of $BF_3 \cdot Et_2O$ (47%), and the mixture is stirred at 25°C for 90 minutes. The solvent is distilled off, and the residue is chromatographed on a column of silica gel (hexane-ethyl acetate ratio = 1:2), thus giving the subject compound as colorless crystals (1.8 g, 53.3%). m.p. 71-72°C.

IR $\nu_{max}^{KBr}$ $cm^{-1}$: 1730

NMR (60MHz, $CDCl_3$) δ: 1.38-2.40(12H,m,$-CH_2$x6), 2.63(1H,dd,J=14,2Hz, $C_{(7)}H$), 3.01(1H,dd,J=14.5Hz,$C_{(7)}H$), 3.20-3.72(1H,m,$C_{(6)}H$), 3.74-3.79(2H, m,$-C_{(4)}H_2-$)

Elemental analysis, for $C_{11}H_{17}NO_2$

Calcd.: C, 67.66; H, 8.78; N, 7.17

Found : C, 67.57; H, 8.85; N, 7.06

## Reference Example 13

- 7-Phenylthio-2,2-spirocyclohexyl-8-oxo-1-azabicyclo[4.2.0]-octane -

A 9 mℓ (14.4 mmol) portion of 15% n-butyllithium/hexane is added under stirring to a solution of 1.89 mℓ (13,5 mmol) of diisopropylamine in 40 mℓ of anhydrous tetrahydrofuran under nitrogen atmosphere at -78°C. To the solution is added dropwise a solution of 1.76 g (9 mmol) of 2,2-spirocyclohexyl-8-oxo-3-oxa-1-azabicyclo[4.2.0]octane in 10 mℓ of anhydrous tetrahydrofuran, and the mixture is stirred for 10 minutes. Then, a solution of 1.96 g (9 mmol) of diphenyl disulfide in 8 mℓ of anhydrous tetrahydrofuran is added dropwise to the mixture, while maintaining the internal temperature at below -70°C, and the resultant mixture is stirred for 10 minutes. The mixture is poured into saturated aqueous ammonium chloride under ice-cooling, and the tetrahydrofuran layer is separated out, while the water layer is extracted with ethyl acetate. The organic layers are combined, washed with 0.5N NaOH and water, successively, dried ($Na_2SO_4$), and the solvent is distilled off. The residue is treated with isopropyl ether, thus giving the trans-form of the subject compound as colorless crystals (1.42 g). The mother liquor of crystallization

is concentrated, and the residue is chromatographed on a column of silica gel (hexane-ethyl acetate ratio = 4:1), thus giving further 0.55 g (combined yield of 1.97 g, 72.2%) of the trans form and 0.08 g (2.9%) of the cis-form of the subject compound as colorless crystals, respectively.

trans-form: m.p. 133-134°C.

IR $\nu_{max}^{Nujol}cm^{-1}$: 1730

NMR (90MHz, $CDCl_3$)δ: 0.8-2.4(12H,m,$-CH_2$-x6), 3.4(1H,m,>CH-N<), 3.77(2H,m,$-CH_2O-$), 3.88(1H,d,J=2Hz,$^{-S}$>CH-CO-), 7.2-7.6(5H, m,arom.H).

Elemental analysis, for $C_{17}H_{21}NO_2S$

Calcd.: C, 67.30; H, 6.98; N, 4.62

Found : C, 67.24; H, 6.77; N, 4.37

cis-form: m.p. 134-135°C.

IR $\nu_{max}^{Nujol}$ $cm^{-1}$: 1725

NMR (90MHz, $CDCl_3$)δ: 1.3-2.4(12H,m,$-CH_2$-x6), 3.8-4.0(3H,m, >CH-N<,$-CH_2O-$), 4.60(1H,d,J=5Hz,$^{-S}$>CH-CO-), 7.2-7.5(5H,m, arom.H).

Elemental analysis, for $C_{17}H_{21}NO_2S$

Calcd.: C, 67.30; H, 6.98; N, 4.62

Found : C, 66.94; H, 6.98; N, 4.69

Reference Example 14

- 7-(1-Hydroxy-1-methylethyl)-7-phenylthio-2,2-spirocyclohexyl-8-oxo-1-azabicyclo[4.2.0]octane -

A 4 mℓ (6.4 mmol) portion of 15% n-butyllithium/hexane is added under stirring to a solution of 0.83 mℓ (5.9 mmol) of diisopropylamine in 20 mℓ of anhydrous tetrahydrofuran under nitrogen atmosphere at -78°C. To the solution at -78°C is added a solution of 1.21 g (4.0 mmol) of trans-7-phenylthio-2,2-spirocyclohexyl-8-oxo-1-azabicyclo[4.2.0]-octane in 9 mℓ of anhydrous tetrahydrofuran , and the mixture is stirred for 15 minutes. Then, after 1.5 mℓ of anhydrous acetone is added and stirring continued for 30 minutes, the mixture is poured into saturated aqueous ammonium chloride under ice-cooling, and the tetrahydrofuran layer

is separated. After the water layer is extracted with ethyl acetate, the organic layers are combined, washed with water, dried ($Na_2SO_4$), and the solvent is distilled off. The residue is chromatographed on a column of silica gel (hexane-ethyl acetate ratio = 4:1, 1:1), thus giving the starting material (recovered, 0.13 g) and 1.03 g(79.8%, as determined on the basis of unrecovered starting material) of the subject compound (the isomer mixture) as colorless crystals.

m.p. 98-100°C

IR $\nu_{max}^{Nujol}$ $cm^{-1}$: 3430, 1740

NMR (90MHz, $CDCl_3$)δ: 1.3-2.3(18H,m,-$CH_3$x2,-$CH_2$-x6), 3.4-4.1 (3H,m,>CH-N<,-$CH_2$O-), 7.2-7.7(5H,m,arom.H).

Elemental analysis, for $C_{20}H_{27}NO_3S$

Calcd.: C, 66.45; H, 7.53; N, 3.87

Found : C, 66.53; H, 7.56; N, 4.09

## Reference Example 15

- 7-(1-Hydroxy-1-methylethyl)-2,2-spirocyclohexyl-8-oxo-1-azabicyclo[4.2.0]octane -

A solution of 870 mg (2.4 mmol) of 7-(1-hydroxy-1-methylethyl)-7-phenylthio-2,2-spirocyclohexyl-8-oxo-1-aza-bicyclo[4.2.0]octane as obtained in Reference Example 14, 87 mg (0.53 mmol) of azobis-isobutyronitrile and a solution of 2.10 mℓ (7.9 mmol) of n-tributyltin hydride in 25 mℓ of acetone is refluxed under nitrogen atmospherefor 14 hours. The solvent is distilled off, and residue is chromatographed on a column of silica gel (hexane-ethyl acetate ratio = 1:1), thus giving 450 mg (73.8%) of the cis-form, and 120 mg (19.7%) of the trans-form, of the subject compound obtained as colorless crystals, respectively.

cis-form: m.p. 153-154°C.

IR $\nu_{max}^{Nujol}$ $cm^{-1}$: 3500, 1720

NMR (90MHz, $CDCl_3$)δ: 1.27, 1.47(each 3H, s x 2, -$CH_3$x2), 1.3 -3.0(12H,m,-$CH_2$-x6), 3.18(1H,d,J=5Hz,>CHCO-), 3.6-3.9(3H,m, >CH-N<,-$CH_2$O-).

Elemental analysis, for $C_{14}H_{23}NO_3$

Calcd.: C, 66.37; H, 9.15; N, 5.53

Found : C, 65.95; H, 9.08; N, 5.50

trans-form: m.p. 83-84°C

IR $\nu_{max}^{Nujol}$ $cm^{-1}$: 3500, 1710

NMR (90MHz, $CDCl_3$)δ: 1.27, 1.33(each 3H, s x 2, $-CH_3$x2), 1.3-2.4(12H,m,$-CH_2$x6), 2.76(1H,d,J=2Hz,>CHCO-), 3.5(1H,m,>CH-N<), 3.8(2H,m,$-CH_2O-$).

Elemental analysis, for $C_{14}H_{23}NO_3$

Calcd.: C, 66.37; H, 9.15; N, 5.53

Found : C, 66.37; H, 9.02; N, 5.65

## Reference Example 16

- cis-7-[1-(2-Methoxyethoxymethoxy)-1-methylethyl]-2,2-spirocyclohexyl-8-oxo-3-oxa-1-azabicyclo[4.2.0]octane -

To a solution of 380 mg (1.5 mmol) of cis-7-(1-hydroxy-1-methylethyl)-2,2-spirocyclohexyl-8-oxo-3-oxa-1-azabicyclo[4.2.0]octane in 8 mℓ of methylene chloride are added 0.86 mℓ (5.0 mmol) of diisopropylethylamine and 0.56 mℓ (5.0 mmol) of 2-methoxyethoxymethyl chloride, and the mixture is allowed to stand at room temperature for 15 hours. After 0.086 mℓ (0.5 mmol) of diisopropylethylamine and 0.056 mℓ (0.5 mmol) of 2-methoxyethoxymethyl chloride are further added and the mixture is allowed to stand at room temperature for 9 hours, the reaction solution is washed with water, 2% aqueous acetic acid and water, successively, dried ($Na_2SO_4$), and the solvent is distilled off, thus giving the subject compound quantitatively as a pale yellow oily substance.

IR $\nu_{max}^{Neat}$ $cm^{-1}$: 1740

NMR (60MHz, $CDCl_3$)δ: 1.27, 1.47(each 3H, s x 2, $-CH_3$x2), 1.2-2.7 (12H,m,$-CH_2-$x6), 3.13(1H,d,J=5Hz,-CH-CO-), 3.32(3H,s,$-OCH_3$), 3.4-3.9(7H,m,$-CH_2O-$x3,>CH-N<), 4.80(2H,s,$-OCH_2O-$)

## Reference Example 17

- cis-4-Carboxymethyl-3-[1-(2-methoxyethoxymethoxy)-1-methylethyl]azetidin-2-one -

(1) Production from cis-4-hydroxyethyl-3-[1-(2-methoxyethoxymethoxy)-1-methylethyl]azetidin-2-one.

To a solution of 260 mg (1 mmol) of cis-4-hydroxyethyl-3-[1-(2-methoxyethoxymethoxy)-1-methylethyl]azetidin-2-one in 10 mℓ of acetone is added, under ice-cooling, 1.5 mℓ of a Jones reagent (a solution prepared by adding 26.7 g of chromic anhydride to a mixed solution of 23 mℓ of concentrated sulfuric acid and 40 mℓ of water, and adding water to make a total volume of 100 mℓ; the reagent prepared in this manner is employed in the subsequent procedures under the items (2) through (4)), and stirring is continued for 3 hours. After isopropanol is added to the reaction solution and stirring is effected for 5 minutes, the solution is diluted with methylene chloride, and filtered with celite. The filtrate is concentrated, and the residue is dissolved in chloroform. Then, the solution is washed with a small amount of saturated aqueous sodium chloride, dried ($Na_2SO_4$), and the solvent is distilled off. The residue is treated with isopropyl ether, thus giving the subject compound as colorless crystals (210 mg, 76.4%). m.p. 87-89°C.

IR $\nu_{max}^{KBr}cm^{-1}$: 3500-2800, 1765, 1730

NMR (90MHz, $CDCl_3$)δ: 1.33, 1.51(each 3H, s x 2, $-CH_3$x 2), 2.7-3.4(3H,m,$-CH_2$-CO-,>CH-CO-), 3.37(3H,s,$OCH_3$), 3.4-3.8(4H,m, $-OCH_2CH_2O-$), 4.0(1H,m,>C<u>H</u>-NH-), 4.83(2H,$-OCH_2O-$), 7.2(1H,b, -NH-), 8.6(1H,b,-COOH).

Elemental analysis, for $C_{12}H_{21}NO_6$

Calcd.: C, 52.35; H, 7.69; N, 5.09

Found : C, 52.50; H, 7.48; N, 5.21

(2) Production from cis-3-[1-(2-methoxyethoxymethoxy)-1-methylethyl]-4-(2-oxoethyl)azetidin-2-one.

To a solution of 40 mg (0.15 mmol) of cis-3-[1-(2-methoxyethoxymethoxy)-1-methylethyl]-4-(2-oxoethyl)azetidin-2-one in 1 mℓ of acetone is added, under ice-cooling, 0.08 mℓ of the Jones reagent, and stirring is effected for 10 minutes. After 0.1 mℓ of isopropanol is added to the reaction solution and stirring is effected for 5 minutes, methylene chloride is added to the mixture and filtered with celite. The filtrate is concentrated, and the residue is dissolved in methylene

chloride. The solution is washed with a small amount of saturated aqueous sodium chloride, dried ($Na_2SO_4$) and the solvent is distilled off. The residue is treated with isopropyl ether, thus giving the subject compound as colorless crystals (33 mg, 80%).

(3) Production from cis-7-[1-(2-methoxyethoxymethoxy)-1-methylethyl]-8-oxo-2,2-dimethyl-3-oxa-1-azabicyclo[4.2.0]octane.

To a solution of 10.0 g (33 mmol) of cis-7-[1-(2-methoxyethyxymethoxy)-1-methylethyl]-8-oxo-2,2-dimethyl-3-oxa-1-azabicyclo[4.2.0]octane in 330 mℓ of acetone is added, under ice-cooling, 33 mℓ of the Jones reagent , and stirring is continued for 4 hours. After 6.6 mℓ of the Jones reagent is further added and stirring is effected for 1.5 hours, 16 mℓ of isopropanol is gradually added to the reaction solution, and stirring is effected for 15 minutes. After the dilution of the reaction solution with methylene chloride (150 mℓ), insoluble matter is filtered out with celite, and the filtrate is concentrated. Chloroform (200 mℓ) is added to the residue, and the mixture is washed with saturated aqueous sodium chloride, (8 mℓ), dried ($Na_2SO_4$) and the solvent is distilled off, thus giving the subject compound as colorless crystals (7.24 g, 80.1%).

(4) Production from cis-7-[1-(2-methoxyethoxymethoxy-1-methylethyl]-2,2-spirocyclohexyl-8-oxo-3-oxa-1-azabicyclo[4.2.0]-octane.

To a solution of 510 mg (1.5 mmol) of cis-7-[1-(2-methoxyethoxymethoxy-1-methylethyl]-2,2-spirocyclohexyl-8-oxo-3-oxa-1-azabicyclo[4.2.0]octane in 15 mℓ of acetone is added, unde ice-cooling, 2.3 mℓ of the Jones reagent, and stirring is continued for 3 hours. After 1.5 mℓ of isopropanol is added to the reaction solution and stirring is effected for 5 minutes, the mixture is diluted with methylene chloride, and insoluble matter is filtered out with celite. After the filtrate is concentrated, the residue is dissolved in chloroform, and the solution is washed with a small amount of saturated aqueous sodium chloride, dried ($Na_2SO_4$) and the solvent is distilled off The residue is treated with isopropyl ether, thus giving the

subject compound as colorless crystals (270 mg, 65.6%).

The IR and NMR spectra of the compounds as obtained under the items (2) through (4) are found to be in agreement with those of the compound as obtained under the above-mentioned item (1).

Reference Example 18

- cis-3-[1-(2-Methoxyethoxymethoxy)-1-methylethyl]-4-[3-(4-nitrobenzyloxycarbonyl)-2-oxopropyl]azetidin-2-one -

To a solution of 7.78 g (28 mmol) of cis-4-carboxymethyl-3-[1-(2-methoxyethoxymethoxy)-1-methylethyl]azetidin-2-one in 230 mℓ of anhydrous tetrahydrofuran is added, at room temperature, 6.37 g (39 mmol) of 1,1'-carbonyldiimidazole, and stirring is continued for 6 hours. 14.0 g (28 mmol) of a magnesium salt of mono-4-nitrobenzyl malonate is added to the solution, and the mixture is stirred at room temperature for 17 hours. Ethyl acetate is added to the reaction solution, which is washed with diluted hydrochloric acid, water, saturated aqueous sodium hydrogen carbonate and water, successively, dried ($Na_2SO_4$) and the solvent is distilled off. The residue is chromatographed on a column of silica gel (ethyl acetate), thus giving the subject compound as a pale yellow, oily substance (10.50 g, 82.1%).

IR $\nu_{max}^{Neat}$ $cm^{-1}$: 3300, 1760-1720

NMR (60MHz, $CDCl_3$)δ: 1.30, 1.48(each 3H, s x 2, $-CH_3$x2), 3.33 (3H,s,$-OCH_3$), 3.63(2H,s,$-COCH_2CO-$), 3.2-4.1(3H,m,>CHCO-, >CH$\underline{H_2}$CO-), 4.2(1H,m,>C$\underline{H}$-NH-), 4.80(2H,s,$-OCH_2O-$), 5.27(2H, s,$-OCH_2Ar$), 6.52(1H,bs,>NH), 7.53(2H,d,J=11Hz,arom.H), 8.20 (2H,d,J=11Hz,arom.H).

Reference Example 19

- cis-4-[3-Diazo-3-(4-nitrobenzyloxycarbonyl)-2-oxopropyl]-3-[1-(2-methoxyethoxymethoxy)-1-methylethyl]aztidin-2-one -

To a solution of 10.5 g (23.2 mmol) of cis-3-[1-(2-methoxyethoxymethoxy)-1-methylethyl]-4-[3-(4-nitrobenzyloxycarbonyl)-2-oxopropyl]azetidin-2-one in 300 mℓ of anhydrous acetonitrile are added, at 0°C, a solution of 5.48 g (27.8 mmol) of p-toluenesulfonyl azide in 30 mℓ of anhydrous acetonitrile,

and then 11.6 mℓ (83.4 mmol) of triethylamine. After cooling is suspended, stirring is effected at room temperature for 30 minutes, ethyl acetate is added to the reaction solution, which is then washed with satuated aqueous sodium chloride, dried ($Na_2SO_4$) and the solvent is distilled off. The residue is chromatographed on a column of silica gel (ethyl acetate), thus giving the subject compound as colorless crystals (9.80 g, 88.3%). m.p. 97-99°C.

IR $\nu_{max}^{Nujol}$ $cm^{-1}$: 3360, 3210, 2140, 1730, 1705, 1640

NMR (90MHz, $CDCl_3$)δ: 1.37, 1.53 (each 3H,s x 2,$-CH_3$x2), 3.37 (3H,s,$-OCH_3$), 3.3-4.0(6H,m,>CHCO-,$-CH_2CO$,$-OCH_2CH_2O-$), 4.2 (1H,m,>C<u>H</u>-NH-), 4.85(2H,s,$-OCH_2O-$), 5.40(2H,s,$-OCH_2Ar$), 6.33 (1H,bs,-NH-), 7.57(2H,d,J=11Hz,arom.H), 8.27(2H,d,J=11Hz,arom.H).

Elemetal analysis, for $C_{21}H_{26}N_4O_9$

Calcd.: C, 52.71; H, 5.48; N, 11.71

Found : C, 52.29; H, 5.36; N, 11.64

## Reference Example 20

- cis-4-[3-Diazo-3-(4-nitrobenzyloxycarbonyl)-2-oxopropyl]-3-(1-hydroxy-1-methylethyl)azetidin-2-one-

To a solution of 1.00 g (2.1 mmol) of cis-4-[3-diazo-3-(4-nitrobenzyloxycarbonyl)-2-oxopropyl]-3-[1-(2-methoxyethoxymethoxy)-1-methylethyl]azetidin-2-one in 42 ml of dried methylene chloride is added, under nitrogen atmosphere at 0°C, 1.45 mℓ (13.2 mmol) of titanium tetrachloride, and stirring is effected for 1 hour. The reaction solution is poured into a mixed solution of saturated aqueous potassium carbonate (50 mℓ) and methylene chloride (50 mℓ) under stirring with the internal temperature maintained at 0°C. The mixture is filtered with celite, and the methylene chloride layer is separated, while the water layer is extracted with methylene chloride. Then, the organic layers are combined, washed with water, diluted hydrochloric acid, aqueous sodium hydrogen carbonate and water, successively, dried ($Na_2SO_4$) and the solvent is distilled off. The residue is treated with benzene, thus giving the subject compound as pale yellow crystals of its solvate

with benzene (0.80 g, 81.7%). m.p. 90°C (once molten, then solidified), 154-155°C (decomp.).
IR $\nu_{max}^{Nujol} cm^{-1}$: 3400, 3310, 2130, 1740, 1680
NMR (90MHz, $CDCl_3$) δ: 1.30, 1.51 (each 3H, s x 2,$-CH_3$x2), 3.2-3.7(3H,m,>CHCO-,$-CH_2$-CO-), 4.1(1H,m,>C<u>H</u>NH-), 5.36(2H,s,$-OCH_2$Ar), 6.2(1H,b,>NH), 7.55(2H,d,J=9Hz,arom.H), 8.27(2H,d,J=9Hz,arom.H).
Elemental analysis, for $C_{17}H_{18}N_4O_7 \cdot C_6H_6$
Calcd.: C, 58.97; H, 5.16; N, 11.96
Found : C, 58.64; H, 5.23; N, 11.82

## Reference Example 21

- 4-Nitrobenzyl 5,6-cis-6-(1-hydroxy-1-methylethyl)-1-azabicyclo[3.2.0]heptane-3,7-dione-2-carboxylate -

Nitrogen is introduced for 10 minutes into a suspension of 1.17 g (2.5 mmol) of cis-4-[3-diazo-3-(4-nitrobenzyloxycarbonyl)-2-oxopropyl]-3-(1-hydroxy-1-methylethyl)azetidin-2-one(benzene-solvation product) and 60 mg of Rhodium (II) acetate in 125 mℓ of anhydrous benzene so that deoxygenation is conducted. Then, the mixture is heated with stirring under nitro gen atmosphere at 78°C for 45 minutes. The reaction solution is cooled, then filterd with celite and the solvent is distilled off under reduced pressure, thus giving the subject compound as colorless foamy substance (0.91 g, 100%).
IR $\nu_{max}^{KBr} cm^{-1}$: 3500, 1780-1720
NMR (90MHz, $CDCl_3$) δ: 1.28, 1.53(each 3H,s x 2,$-CH_3$x2), 1.9(1H,b,-OH), 2.63(1H,dd,J=19, 7Hz,>C<$\frac{H}{H}$), 3.67(1H,d,J=6Hz,>CHCO-), 3.96(1H,dd,J=19, 10Hz,>C<$\frac{H}{H}$), 4.25(1H,m,>C<u>H</u>-NH-), 4.70(1H,s,>NCH<$\frac{CO-}{CO-}$), 5.30(2H,ABq,J=16, 13Hz,$-OCH_2$Ar), 7.55(2H,d,J=9Hz,arom.H), 8.27(2H,d,J=9Hz,arom.H).

This compound, when being treated with benzene, changes into colorless crystals.(containing 1/3 moles of benzene). Its physical properties is as follows: m.p. 55-60°C (softened).
IR $\nu_{max}^{KBr} cm^{-1}$: 3550, 1780-1720
Elemental analysis, for $C_{17}H_{18}N_2O_7 \cdot 1/3C_6H_6$
Calcd.: C, 58.76; H, 5.19; N, 7.21
Found : C, 58.74; H, 5.17; N, 7.02

In the reaction of the subsequent reaction step, is employed the above-described crude product as obtained by distilling off the solvent.

Reference Example 22

- 4-Nitrobenzyl 5,6-cis-6-[1-(2-methoxyethoxymethoxy)-1-methylethyl-1-azabicyclo[3.2.0]heptane-3,7-dione-2-carboxylate -

Nitrogen is introduced for 10 minutes into a suspension of 320 mg (0.67 mmol) of 5,6-cis-4-[3-diazo-3-(4-nitrobenzyloxycarbonyl)-2-oxopropyl]-3-[1-(2-methoxyethoxymethoxy)-1-methylethyl]azetidin-2-one and 16 mg of Rhodium (II) acetate in anhydrous benzene to carry out deoxygenation. Then, the mixture is heated with stirring under nitrogen atmosphere, at 78°C for 50 minutes. The reaction solution is cooled, filtered with celite, and the solvent is distilled off under reduced pressure, thus giving the subject compound as a slightly yellow, oily material (0.30 g, 100%).

IR $\nu_{max}^{Neat}$ $cm^{-1}$ : 1780-1740

NMR (60 MHz, $CDCl_3$) δ: 1.30, 1.51(each 3H,s x 2, $-CH_3$ x 2), 2.60 (1H,dd,J=18,7Hz,$>C<^{H}_{H}$), 3.30(3H,s,$-OCH_3$), 3.4-3.7(5H,m,>CH-CO, $-OCH_2CH_2O-$), 3.8-4.4(2H,m,$>C<^{H}_{H}$,>CH-N<), 4.65(1H,s,$>N-CH<^{CO-}_{CO-}$) 4.71(2H,s,$-OCH_2O-$), 5.20(2H,s,$-OCH_2-Ar$), 7.42(2H,d,J=9Hz, arom.H), 8.10(2H,d,J=9Hz,arom.H)

This product is employed as such in the reaction of the subsequent reaction step.

## Reference Example 23

- 7-(1-Hydroxy-1-methylethyl)-8-oxo-2,2-dimethyl-3-oxa-1-azabicyclo[4.2.0]octane -

A solution of 3.14 g (9.76 mmol) of 7-(1-hydroxy-1-methylethyl)-7-phenylthio-8-oxo-2,2-dimethyl-3-oxa-1-azabicyclo-[4.2.0]octane (the isomer A) and 7.02 g (20.0 mmol) of triphenyltin hydride in 100 mℓ of acetone is irradiated under nitrogen atmosphere with light by use of a high-pressure mercury lamp for 6 hours (the internal temperature of 38°C to 42°C). The precipitate separated out is filtered out, and the filtrate is concentrated. The residue is chromatographed on a column of silca gel (hexane-ethyl acetate ratio = 1:2), thus giving 1.567 g (75.3%) of the cis-form and 0.363 g (17.5%) of the trans-form as colorless crystals, respectively. The physical properties (melting point, IR and NMR spectra) each of the two isomers are found to be in agreement with those of the isomers as obtained in Reference Example 8 (1).

0074599

## Reference Example 24

- 7-(N,N-Dimethylaminothiocarbonylthio)-8-oxo-2,2-dimethyl-3-oxa-1-azabicyclo[4.2.0]octane -

A 3 mℓ portion of 15% n-butyllithium/hexane is added with stirring to a solution of 0.59 mℓ (4.2 mmol) of diisopropylamine in 9 mℓ of anhydrous tetrahydrofuran under nitrogen atmosphere at -78°C. To the solution is added at, -78°C, a solution of 310 mg (2 mmol) of 8-oxo-2,2-dimethyl-3-oxa-1-azabicyclo[4.2.0]octane in 3 mℓ of anhydrous tetrahydrofuran, followed by stirring for 5 minutes. Then, a solution of 240 mg (2 mmol) of bis-[dimethylamino(thiocarbonyl)]disulfide in 3 mℓ of anhydrous tetrahydrofuran is added over a period of 10 minutes. The reaction solution is stirred for 10 minutes, poured in a saturated aqueous solution of ammonium chloride under ice-cooling and extracted with ethyl acetate. The extract is washed with water, dried ($Na_2SO_4$) and the solvent is distilled off. The residue is treated with hexane, thus giving the subject compound as light yellow crystals (360 mg, 66%).

m.p. 128 - 129°C.

IR $\nu_{max}^{Nujol}$ $cm^{-1}$: 1740

NMR (60 MHz, $CDC\ell_3$) δ: 1.40, 1.75 (each, 3H,s x 2, $-CH_3$x2), 2.1 (2H,m,$-CH_2-$), 3.37, 3.75 (each, 3H,s x 2, $-CH_3$x2), 3.3-3.5 (1H, m,⟩CH-N<), 3.8 (2H,m,$-CH_2O-$), 4.68 (1H,d,J=1.5Hz,>CH-CO-)

Elemental analysis, for $C_{11}H_{18}N_2O_2S$

Calcd.: C, 48.15; H, 6.61; N, 10.21

Found : C, 48.31; H, 6.75; N, 10.23

## Reference Example 25

- 7-(1-Hydroxy-1-methylethyl)-7-(N,N-dimethylaminothiocarbonylthio)-8-oxo-2,2-dimethyl-3-oxa-1-azabicyclo[4.2.0]octane -

A. From 7-(N,N-dimethylaminothiocarbonylthio)-8-oxo-2,2-dimethyl-3-oxa-1-azabicyclo[4.2.0]octane;

A 0.9 mℓ portion of 15% n-butyllithium/hexane is added to a solution of 0.17 mℓ (1.2 mmol) of diisopropylamine in 4 mℓ of anhydrous tetrahydrofuran under nitrogen atmosphere at -78°C. To the solution is added at -78°C a solution

of 274 mg (1 mmol) of 7-(N,N-dimethylaminothiocarbonylthio)-8-oxo-2,2-dimethyl-3-oxa-1-azabicyclo[4.2.0]octane in 2 mℓ of anhydrous tetrahydrofuran, followed by stirring for 10 minutes. Then, 0.3 mℓ of anhydrous acetone is added, and stirring is continued for 30 minutes. The reaction solution is poured in an aqueous ammonium chloride solution under cooling and extracted with ethyl acetate. The extract is washed with water, dried ($Na_2SO_4$) and the solvent is distilled off. The residual, oily substance is chromatographed on a column of silica gel (hexane-ethyl acetate = 4:1, 2:1), thus affording the starting material (as recovered, 20 mg) and the subject compound (199 mg, 60%). The subject compound is obtained as a mixture of two forms of isomers, whose ratio is found to be 3:1 as indicated by an analysis of the NMR spectrum. The two isomers were separated by the crystallization procedure with use of isopropyl ether.

Isomer A (minor product):

m.p. 166-167°C

IR $\nu_{max}^{Nujol}$ $cm^{-1}$: 1740

NMR (60 MHz, $CDCℓ_3$) δ: 1.37, 1.42, 1.52, 1.72 (each, 3H,s x 4, $-CH_3$x4), 2.2 (2H,m,$-CH_2-$), 3.50 (6H,s,$-NCH_3$x2), 3.8 (2H, m,$-OCH_2-$), 4.2 (1H,m,>CH-N<), 5.75 (1H,s,-OH)

Elemental analysis, for $C_{14}H_{24}N_2O_3S_2$

Calcd.: C, 50.59; H, 7.28; N, 8.43

Found : C, 50.66; H, 7.39; N, 8.31

Isomer B (main product):

m.p. 124-126°C

IR $\nu_{max}^{Nujol}$ $cm^{-1}$: 1750

NMR (60 MHz, $CDCℓ_3$) δ: 1.45 (6H,s,$-CH_3$x2), 1.60, 1.72 (each, 3H,s x 2, $-CH_3$x2), 2.2 (2H,m,$-CH_2-$), 3.47 (6H,s,$-NCH_3$x2), 3.9 (2H,m,$-OCH_2-$), 4.3 (1H,m,>CH-N<), 4.5 (1H,s,-OH).

Elemental analysis, for $C_{14}H_{24}N_2O_3S_2$

Calcd.: C, 50.59; H, 7.28; N, 8.43

Found : C, 50.66; H, 7.40; N, 8.55

B. From 8-oxo-2,2-dimethyl-3-oxa-1-azabicyclo[4.2.0]octane;

A 15 mℓ portion of 15% n-butyllithium/hexane is added

with stirring to a solution of 2.9 mℓ (21 mmol) of diisopropylamine in 50 mℓ of anhydrous tetrahydrofuran under nitrogen atmosphare at -78°C. To the solution is added at -78°C a solution of 1.55 g (10 mmol) of 8-oxo-2,2-dimethyl-3-oxa-1-azabicyclo[4.2.0]octane in 15 mℓ of anhydrous tetrahydrofurane, followed by stirring for 5 minutes. A solution of 1.20 g (10 mmol) of bis-[dimethylamino(thiocarbonyl)]disulfide in 30 mℓ of anhydrous tetrahydrofuran is added to the mixture over a period of 15 minutes, and the resultant mixture is stirred at -78°C for 5 minutes, followed by gradual addition of 2 mℓ of anhydrous acetone. After the reaction mixture is stirred for 40 minutes at -78°C, it, by utilization of the pressure of nitrogen, is gradually poured with stirring in an aqueous ammonium chloride solution under cooling and extracted with ethyl acetate. The extract is washed with water, dried ($Na_2SO_4$) and the solvent is distilled off. The residue is chromatographed on a column of silica gel (hexane-ethyl acetate = 4:1, 2:1), giving 7-(N,N-dimethylaminothiocarbonylthio)-8-oxo-2,2-dimethyl-3-oxa-1-azabicyclo-[4,2,0]octane (light yellow crystals, 0.14 g) and the subject compound [the isomer mixture (whose ratio is 3:1 as indicated by an analysis of the NMR spectrum), colorless crystals (as treated with isopropyl ether - hexane), m.p. 116-117°C, 1.17 g (35%)].

Reference Example 26

- 7-(1-Hydroxy-1-methylethyl)-8-oxo-2,2-dimethyl-3-oxa-1-azabicyclo[4.2.0]octane -

A solution of 160 mg (0.48 mmol) of 7-(1-hydroxy-1-methylethyl)-7-(N,N-dimethylaminothiocarbonylthio)-8-oxo-2,2-dimethyl-3-oxa-1-azabicyclo[4.2.0]octane (the 3:1 isomer mixture) as obtained in Reference Example 25, 16 mg (0.1 mmol) of azobisisobutyronitrile and 0.33 mℓ (1.2 mmol) of tri-n-butyltin hydride in 5 mℓ of acetone is refluxed under nitrogen atmosphere for 5 hours. The solvent is distilled off, and the residue is chromatographed on a column of silica gel (hexane - ethyl acetate = 1:1), thus giving 76 mg (74.2%, m.p. 125-127°C) of the cis-form, and 22 mg (21.6%,

m.p. 102-104°C) of the trans form, of the subject compound as colorless crystals, respectively.

Example 1

- 4-Nitrobenzyl 5,6-cis-3-[2-(4-nitrobenzyloxycarbonyl)amino-ethylthio]-6-(1-hydroxy-1-methylethyl)-7-oxo-1-azabicyclo-[3.2.0]hept-2-ene-2-carboxylate -

To a solution in 25 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 250 mg (0.52 mmol) of 5,6-cis-4-[3-diazo-3-(4-nitrobenzyloxycarbonyl)-2-oxopropyl]-3-(1-hydroxy-1-methylethyl)azetidin-2-one (benzene-solvation product) are added, under nitrogen atmosphere at 0°C, 0.12 mℓ (0.7 mmol) of diisopropylethylamine and then 0.14 mℓ (0.7 mmol) of diphenylphosphoryl chloride, and stirring is effected for 90 minutes. 0.20 mℓ (1.1 mmol) of diisopropylethylamine and 250 mg (1.0 mmol) of N-4-nitro-benzyloxycarbonylcysteamine are added to this mixture, which is stirred at 0°C for 2 hours and then allowed to stand at -20°C for 16 hours. The reaction solution is diluted with ethyl acetate, and the mixture is poured into ice-water, followed by separating the organic layer. The organic layer is washed with water, dried ($Na_2SO_4$) and the solvent is distilled off. The residue is chromatographed on a column of Florisil (100 to 200 mesh) (acetone-hexane ratio = 1:2), thus giving the subject compound (170 mg) as a pale yellow, foamy substance.

IR $\nu_{max}^{KBr}$ $cm^{-1}$: 3400, 1770, 1710

UV $\lambda_{max}^{EtOH}$ nm($E_{1cm}^{1\%}$) : 264(303), 313(165).

NMR (100 MHz, $CDCl_3$)$\delta$: 1.28, 1.52(each 3H,s x 2,$-CH_3$x2), 3.02 (3H,m,$-SC\underline{H}_2CH_2N$<,>C$\langle{}^{H}_{\underline{H}}$), 3.46(2H,m,$-SCH_2CH_2NH-$), 3.60(1H ,d,J=6Hz, >C$\underline{H}$CO-), 4.0-4.4(2H,m,>C$\underline{H}$N<,>C<${}^{H}_{\underline{H}}$), 5.18(2H,s,$-OCH_2Ar$), 5.36 (2H,ABq,J=29, 14Hz,$-OCH_2Ar$), 7.48(2H,d,J=9Hz,arom.H), 7.65 (2H,d,J=9Hz,arom.H), 8.20(4H,d,J=9Hz,arom.H.).

Example 2

- 5,6-cis-3-(2-Aminoethylthio)-6-(1-hydroxy-2-methylethyl)-7-oxo-1-azabicyclo[3.2.0]hept-2-ene-2-carboxylic acid -

A 140 mg of 10% Pd-C is added to a mixed solution of 140 mg (0.23 mmol) of 4-nitrobezyl 5,6-cis-3-[2-(4-nitrobenzyloxycarbonyl)aminoethylthio]-6-(1-hydroxy-1-methylethyl)-7-oxo-1-azabicyclo[3.2.0]hept-2-ene-2-carboxylate in 28 mℓ of tetrahydrofuran, 14 ml of a pH 7.0 phosphate buffer and 14 mℓ of water, and hydrogenation is carried out at room temperature under atmospheric pressure for 2 hours. After 140 mg of 10% Pd-C is further added and hydrogenation is conducted for additional 3.5 hours, the catalyst is filtered out, and washed with a small amount of water. The filtrate and washings are combined, and the tetrahydrofuran is distilled off under reduced pressure. The water layer is washed with ethyl acetate and concentrated under reduced pressure. The concentrate is chromatographed on a column of Amberlite XAD-2 ($H_2O$, 5% EtOH), and the fractions, which show the UV spectrum having the absorption at 295 nm, are collected and lyophilized, thus giving the subject compound as colorless powder (30 mg).

IR $\nu_{max}^{KBr}$ $cm^{-1}$: 1750

UV $\lambda_{max}^{H_2O}$ $nm(E_{1cm}^{1\%})$ : 295 (219)

NMR (100 MHz, $D_2O$) δ: 1.32, 1.45(each 3H,s x 2,-$CH_3$x2), 2.9-3.3 (5H,m,$>C<^{H}_{H}$, -$SCH_2CH_2N<$), 3.76(1H,d,J=6Hz,>CH-CO-), 3.9(1H,m, $>C<^{H}_{\underline{H}}$), 4.4(1H,m,>CH-N<)

Example 3

- 4-Nitrobenzyl 5,6-cis-3-(2-acetamidoethylthio)-6-(1-hydroxy-1-methylethyl)-7-oxo-1-azabicyclo[3.2.0]hept-2-ene-2-carboxylate -

To a solution in 20 mℓ of anhydrous actonitrile of 4-nitrobenzyl 5,6-cis-6-(1-hydroxy-1-methylethyl)-1-aza-bicyclo[3.2.0]heptane-3,7-dione-2-carboxylate prepared in accordance with the procedure of Reference Example 21 from 250 mg(0.52 mmol) of 5,6-cis-4-[3-diazo-3-(4-nitrobenzyloxycarbonyl)-2-oxopropyl]-3-(1-hydroxy-1-methylethyl)azetidin-2-one (benzene-solvate) are added, under nitrogen atmosphere at 0°C,

0.12 mℓ (0.7 mmol) of diisopropylethylamine and then 0.14 mℓ (0.7 mmol) of diphenylphosphoryl chloride, and stirring is effected for 90 minutes. After 0.13 mℓ (0.75 mmol) of diisopropylethylamine and 90 mg (0.75 mmol) of N-acetylcysteamine are added to the mixture at 0°C and stirring is continued for 2 hours, 0.13 mℓ (0.75 mmol) of diisopropylethylamine and 90 mg (0.75 mmol) of N-acetylcysteamine are further added, followed by stirring for 4 hours. The reaction solution is allowed to stand at -20°C for 14 hours, diluted with ethyl acetate, washed with water, dried ($Na_2SO_4$) and the solvent is distilled off. The residue is treated with a small amount of ethyl acetate, thus giving the subject compound as colorless crystals (110 mg).

m.p. 185-187°C.

IR $\nu_{max}^{Nujol}$ $cm^{-1}$: 3380, 1760, 1700, 1650

UV $\lambda_{max}^{EtOH}$ nm ($E_{1cm}^{1\%}$) : 265 (241), 315 (262)

NMR (90MHz, DMSO-$d_6$) δ: 1.19, 1.35(each 3H,s x 2, $-CH_3$x2), 1.82 (3H,s,$CH_3CO-$), 2.8 - 3.4(5H,m,$>C<^H_H$,$-S-CH_2CH_2N<$), 3.59(1H,d, J=6Hz,>CH-CO-), 4.0-4.3(2H,m,>CH-N<,$>C<^H_H$), 4.70(1H,s,-OH), 5.37(2H,ABq,J=21,14Hz,$-OCH_2Ar$), 7.77(2H,d,J=8Hz,arom.H), 8.10(1H,b,-NH-), 8.27(1H,d,J=8Hz,arom.H).

Elemental analysis, for $C_{21}H_{25}N_3O_7S\cdot1/2H_2O$

Calcd.: C, 53.38; H, 5.55; N, 8.89

Found : C, 53.58; H, 5.43; N, 8.89

## Example 4

- Sodium 5,6-cis-3-(2-acetamidoethylthio)-6-(1-hydroxy-1-methylethyl)-7-oxo-1-azabicyclo[3.2.0]hept-2-ene-2-carboxylate -

(1) Production through catalytic reduction with Pd-C of 4-nitrobenzyl 5,6-cis-3-(2-acetamidoethylthio)-6-(1-hydroxy-1-methylethyl)-7-oxo-1-azabicyclo[3.2.0]hept-2-ene-2-carboxylate.

A 100 mg of 10% Pd-C is added to a mixed solution of 73 mg (0.15 mmol) of 4-nitrobenzyl 5,6-cis-3-(2-acetamidoethylthio)-6-(1-hydroxy-1-methylethyl)-7-oxo-1-azabicyclo[3.2.0]hept-2-ene-2-carboxylate in 14 mℓ of tetrahydrofuran, 7 mℓ of a pH 7 phosphate buffer

and 7 mℓ of water, and hydrogenation is carrried out at room temperature under atmospheric pressure for 1 hour. After 50 mg of 10% Pd-C is further added and hydrogenation is conducted for additional 2 hours, the catalyst is filtered out and washed with a small amount of water. The filtrate and washings are combined, and after the addition of 20 mg of sodium hydrogen carbonate, the tetrahydrofuran is distilled off. The water layer is washed with ethyl acetate and concentrated under reduced pressure. The concentrate is chromatographed on a column of Amberlite XAD-2 ($H_2O$), and the fractions which show the UV spectrum having the absorption at 298 nm are collected and lyophilized, thus giving the crude subject compound as grayish powder (50 mg). This product is purified by chromatographing on a column of Diaion HP-20 (produced by Mitsubishi Chemical Industries Limited) ($H_2O$, 5% EtOH), thus giving the subject compound as colorless powder (30 mg).

IR $\nu_{max}^{KBr}$ $cm^{-1}$: 1745, 1640

UV $\lambda_{max}^{H_2O}$ nm ($E_{1cm}^{1\%}$): 298 (265)

NMR (100 MHz, $D_2O$)δ: 1.33, 1.44(each 3H,s x 2,$-CH_3$x2), 2.02(3H, s,$-COCH_3$), 2.9-4.0(6H,$-SCH_2CH_2N$<,$-CH_2-$), 3.74(1H,d,J=6Hz, >CH-CO-), 4.3(1H,m,>CHN<).

(2) Production through reaction of 5,6-cis-3-(2-aminoethylthio)-6-(1-hydroxy-1-methylethyl)-7-oxo-1-azabicyclo[3.2.0]-hept-2-ene-2-carboxylic acid with acetic anhydride.

To a solution of 12 mg of 5,6-cis-3-(2-aminoethylthio)-6-(1-hydroxy-1-methylethyl)-7-oxo-1-azabicyclo[3.2.0]hept-2-ene-2-carboxylic acid in 4 ml of water and 4 ml of tetrahydrofuran are added, at 0°C, a solution of 8.8 mg of sodium hydrogen carbonate in 2.5 mℓ of water, and then a solution of 5.2 μℓ of acetic anhydride in 1 mℓ of tetrahydrofuran, and stirring is effected for 20 minutes. The reaction solution is concentrated under reduced pressure, and the residue is chromatographed on a column of Diaion HP-20 (produced by Mitsubishi Chemical Industries Limited) ($H_2O$, 5% EtOH). The fractions which show the UV spectrum having the absorption at 298 nm are collected and lyophilized, thus giving the subject compound as colorless

powder (12 mg).

The physical properties (IR and UV spectra) of this product are found to be in agreement with those of the compound as obtained under the item (1).

## Example 5

- 4-Nitrobenzyl 5,6-cis-3-[2-(4,6-dimethylpyrimidinyl)thio]-6-(1-hydroxy-1-methylethyl)-7-oxo-1-azabicyclo[3.2.0]hept 2-ene-2-carboxylate -

To a solution in 8 mℓ of anhydrous acetonitrile of 4-nitrobenzyl 5,6-cis-6-(2-hydroxy-2-propyl)-1-azabicyclo-[3.2.0]heptane-3,7-dione-2-carboxylate as prepared in accordance with the procedure of Reference Example 21 from 94 mg (0.2 mmol) of 5,6-cis-4-[3-diazo-3-(4-nitrobenzyloxycarbonyl)-2-oxopropyl]-3-(1-hydroxy-1-methylethyl)azetidin-2-one (benzene-solvation product) are added, under nitrogen atmosphere at 0°C, 0.044 mℓ (0.25 mmol) of diisopropylethylamine and then 0.052 mℓ (0.25 mmol) of diphenylphosphoryl chloride, and stirring is effected for 90 minutes. Then, 116 mg (0.8 mmol) of lithium salt of 4,6-dimethyl-2-mercaptopyrimidine is added to the mixture, which is thereafter stirred at 0°C for 5 hours and allowed to stand at -20°C for 14 hours. The reaction solution is diluted with ethyl acetate, and the mixture is poured into ice-water. The organic layer is separated, washed with water, dried ($Na_2SO_4$) and the solvent is distilled off. The residue is chromatographed on a column of silica gel (hexane-ethyl acetate ratio = 1:1), thus giving the subject compound as colorless crystals (46 mg).

m.p. 164-166°C.(decomp.)

IR $\nu_{max}^{Nujol}$ $cm^{-1}$: 3300, 1780, 1700

UV $\lambda_{max}^{EtOH}$ nm ($E_{1cm}^{1\%}$): 263 (154), 320 (162).

NMR (90MHz, $CDCl_3$) : 1.27, 1.51(each 3H,s. x 2,$-CH_3$x2), 1.83(1H, s,OH), 2.43(6H,s,$-CH_3$x2), 3.4-3.8(1H,m,$>C<^H_H$), 3.65(1H,d,J= 6Hz,>CH-CO-), 4.2-4.5(2H,m,$>C<^H_H$,>CH-N<), 5.40(2H,ABq,J=25, 14Hz,$-OCH_2Ar$), 6.80(1H,s,arom.H), 7.69(2H,d,J=9Hz,arom.H), 8.25(2H,d,J=9Hz,arom.H).

Elemental analysis, for $C_{23}H_{24}N_4O_6S$

Calcd.: C, 57.02; H, 4.99; N, 11.56

Found : C, 57.14; H, 4.97; N, 11.73

Example 6

- Sodium 5,6-cis-3-[2-(4,6-dimethylpyrimidinyl)thio]-6-(1-hydroxy-1-methylethyl)-7-oxo-1-azabicyclo[3.2.0]hept-2-ene-2 carboxylate -

90 mg of 10% Pd-C is added to 90 mg (0.2 mmol) of 4-nitrobenzyl ester of the subject carboxylic acid dissolved in 18 ml of tetrahydrofuran, 9 mℓ of pH 7.0 phosphate buffer and 9 mℓ of water, and hydrogenation is conducted at room temperature under atmospheric pressure for 60 minutes. After 90 mg of 10% Pd-C is further added and hydrogenation is carried out for additional 75 minutes, the catalyst is filtered out, and washed with a small amount of water. The filtrate and washings are combined, and the tetrahydrofuran is distilled off under reduced pressure. The water layer is washed with ethyl acetate and concentrated under reduced pressure. The concentrate is chromatographed on a column of Amberlite XAD-2 ($H_2O$, 5% EtOH), and the fractions which show the UV spectrum having the absorption at 300 nm are collected and lyophilized, thus giving the crude subject compound as grayish powder (43 mg). This product is purified by chromatographing again on a column of Amberlite XAD-2 ($H_2O$, 5% EtOH), thus giving the subject compound as colorless powder (38 mg).

IR $\nu_{max}^{KBr}$ $cm^{-1}$ : 1750

UV $\lambda_{max}^{H_2O}$ ($E_{1cm}^{1\%}$) : 300 (338)

NMR (100MHz,$D_2O$) δ: 1.25, 1.42(each 3H,s x 2,-$CH_3$x2), 2.45(6H,s, -$CH_3$x2), 3.15(1H,dd,J=17,10Hz,$>C<^H_H$), 3.68(1H,dd,J=17,9Hz,$>C<^H_H$), 3.76(1H,d,J=6Hz,>CHCO-), 4.4(1H,m,>CHN<), 7.12(1H,s,arom.H).

Elemental analysis, for $C_{16}H_{18}N_3NaO_4S \cdot 2H_2O$

Calcd.: C, 47.17; H, 5.44; N, 10.31; Na, 5.64

Found : C, 46.82; H, 5.39; N, 10.09; Na, 5.60

Example 7

0074599

- 4-Nitrobenzyl 5,6-cis-3-(2-pyridylthio)-6-(1-hydroxy-1-methylethyl)-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one(benzene-solvate) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. After 117 mg (1.0 mmol) of lithium salt of 2-mercaptopyridine is added to the mixture and stirring is continued at 0°C for 2.5 hours, 59 mg (0.5 mmol) of lithium salt of 2-mercaptopyridine is further added, and stirring is continued for additional 2 hours. The reaction solution is diluted with ethyl acetate, and the mixture is poured into an aqueous sodium chloride solution under ice-cooling. The organic layer is separated, washed with an aqueous sodium chloride solution, dried ($Na_2SO_4$) and the solvent is distilled off. The residue is chromatographed on a column of silica gel (hexane-ethyl acetate ratio = 1:1), thus giving the subject compound as colorless crystals (94 mg).

m.p. 159-160°C.

IR $\nu_{max}^{KBr}$ $cm^{-1}$ : 1770

UV $\lambda_{max}^{EtOH}$ nm($E_{1cm}^{1\%}$): 263 (293), 320 (332).

NMR (90MHz, $CDCl_3$)δ: 1.25, 1.53(each 3H,s x 2,$-CH_3$x2), 3.09 (1H,dd,J=17,9Hz,$>C<^H_H$), 3.58(1H,d,J=6Hz,>CH-CO-), 3.95(1H,dd, J=17,8Hz,$>C<^H_H$), 4.31(1H,m,>CH-N<), 5.37(2H,ABq,J=24,15Hz, $-OCH_2Ar$), 7.14-8.65(8H,m,arom.H).

Elemental analysis, for $C_{22}H_{21}N_3O_6S$

Calcd.: C, 58.01; H, 4.64; N, 9.22

Found : C, 57.54; H, 4.58; N, 9.27

Example 8

- Sodium 5,6-cis-3-(2-pyridylthio)-6-(1-hydroxy-1-methylethyl)-7-oxo-1-azabicyclo[3.2.0]hept-2-ene-2-carboxylate -

90 mg of 10% Pd-C is added to 90 mg (0.20 mmol) of 4-nitrobenzyl ester of the subject carboxylic acid dissolved in 18 mℓ of tetrahydrofuran, 9 mℓ of pH 7.0 phosphate buffer and 9 mℓ of water, and hydrogenation is carried out at room temperature under atmospheric pressure for 1 hour. After 90 mg of 10% Pd-C is further added and hydrogenation is coducted for additional 1 hour, the catalyst is filtered off and washed with a small volume of water. The filtrate and washings are combined, and the tetrahydrofuran is distilled off under reduced pressure. The resultant aqueous solution is washed with ethyl acetate and concentrated under reduced pressure. The concentrate is chromatographed on a column of Amberlite XAD-2 ($H_2O$, 5% EtOH), and the fractions which show the UV spectrum having the absorption at 304 nm are combined and lyophilized, thus giving the subject compound as colorless powder (32 mg).

IR $\nu_{max}^{KBr}$ $cm^{-1}$: 1750

UV $\lambda_{max}^{H_2O}$ nm ($E_{1cm}^{1\%}$): 304 (353)

NMR (100 MHz, $D_2O$) δ: 1.20, 1.42(each 3H, s x 2,-$CH_3$x2), 2.70 (1H,dd,J=17,10Hz,>C<$\frac{H}{H}$), 3.58(1H,dd,J=17,9Hz,>C<$\frac{H}{H}$), 3.71(1H, d,J=6Hz,>CH-CO-), 4.30(1H,m,>CH-N<), 7.4-8.5(4H,m,arom.H).

## Example 9

- 4-Nitrobenzyl 5,6-cis-3-(2-pyrimidinylthio)-6-(1-hydroxy-1-methylethyl)-7-oxo-1-azabicyclo[3.2.0]hept-2-ene-2-carboxylate -

To a solution, in 20 mℓ of anhydrous acetonitrile, of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one(benzene-solvation product) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. 140 mg (1.2 mmol) of lithium salt of mercaptopyrimidine is added to the mixture, which is stirred at 0°C for 4 hours. The reaction solution is diluted with ethyl

acetate, and the mixture is poured into ice-water. The organic layer is separated, washed with water, dried ($Na_2SO_4$) and the solvent is distilled off. The residue is chromatographed on a column of silica gel (hexane-ethyl acetate ratio = 1:2, 1:3), thus giving the subject compound as colorless crystals (125mg).

m.p. 160-162°C. (decomp.)

IR $\nu_{max}^{Nujol}$ $cm^{-1}$:

UV $\lambda_{max}^{EtOH}$ nm ($E_{1cm}^{1\%}$): 262 (164), 315 (165)

NMR (90MHz,$CDCl_3$) : 1.24, 1.50(each 3H,s x 2,$-CH_3$x2), 1.80(1H,s, -OH), 3.5-3.8(1H,m,$>C<^{H}_{H}$), 3.63(1H,d,J=6Hz,>CH-CO-), 4.0-4.5 (2H,m,$>C<^{H}_{H}$,>CH-N<), 5.39(2H,ABq,J=25,14Hz,$-OCH_2Ar$), 7.07(1H, t,J=5Hz,arom.H), 7.65(2H,d,J=9Hz,arom.H), 8.22(2H,d,J=9Hz, arom.H), 8.58(2H,d,J=5Hz,arom.H).

Elemental analysis, for $C_{21}H_{20}N_4O_6S$

Calcd.: C, 55.26; H, 4.42; N, 12.27

Found : C, 55.49; H, 4.49; N, 12.18

Example 10

- Sodium 5,6-cis-3-(2-pyrimidinylthio)-6-(1-hydroxy-1-methylethyl)-7-oxo-1-azabicyclo[3.2.0]hept-2-ene-2-carboxylate -

A 122 mg quantity of 10% Pd-C is added to a mixed solution of 122 mg (0.27 mmol) of 4-nitrobenzyl ester of the subject carboxylic acid in 25 mℓ of tetrahydrofuran, 12 mℓ of a phosphate buffer with a pH of 7.0 and 12 mℓ of water, and hydrogenation is carried out at room temperature under atmospheric pressure for 1.5 hours. The catalyst is filtered out and washed with a small amount of water. The filtrate and washings are combined, and the tetrahydrofuran is distilled off under reduced pressure. The resultant aqueous solution is washed with ethyl acetate, and concentrated under reduced pressure. The concentrate is chromatographed on a column of Amberlite XAD-2 ($H_2O$, 5% EtOH), and the fractions which show the UV spectrum having the absorption at 295 nm are collected and lyophilized, thus giving the subject compound as colorless powder (62 mg).

IR $\nu_{max}^{KBr}$ $cm^{-1}$ : 1750

UV $\lambda_{max}^{H_2O}$ ($E_{1cm}^{1\%}$): 295 (336)

NMR (100 MHz, $D_2O$) : 1.26, 1.42(each 3H,s x 2, $-CH_3$ x 2), 3.19 (1H,dd,J=17,10Hz,$>C<^H_H$), 3.68(1H,dd,J=17,9Hz,$>C<^H_H$), 3.78 (1H, d,J=6Hz,>CH-CO-), 4.40(1H,m,>CH-N<), 7.34(1H,t,J=5Hz,arom.H), 8.66(2H,d,J=5Hz,arom.H)

## Example 11

- 4-Nitrobenzyl 5,6-cis-3-[3-(6-methylpyridazinyl)thio]-6-(1-hydroxy-1-methylethyl)-7-oxo-1-azabicyclo[3.2.0]hept-2-ene-2-carboxylate -

To a solution, in 20 mℓ of anhydrous acetonitrile, of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one(benzene-solvate) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. After 131 mg (1.0 mmol) of lithium salt of 3-mercapto-6-methylpyridazine is added to the mixture and stirring is continued for 2 hours at 0°C, 66 mg (0.5 mmol) of lithium salt of 3-mercapto-6-methylpyridazine is further added, and stirring is continued for additional 18 hours. The reaction solution is diluted with ethyl acetate, and the mixture is poured into an aqueous sodium chloride solution under ice-cooling. The organic layer is separated, washed with an aqueous sodium chloride solution, dried ($Na_2SO_4$) and the solvent is distilled off. The residue is treated with ehtyl ether, thus giving the subject compound as slightly yellow crystals (83 mg).

m.p. 187-188°C.

IR $\nu_{max}^{KBr}$ $cm^{-1}$: 1750

UV $\lambda_{max}^{MeOH}$ nm ($E_{1cm}^{1\%}$) : 262 (294), 313 (294)

Elemental analysis, for $C_{22}H_{22}N_4O_6S$

Calcd.: C, 56.16; H, 4.71; N, 11.91

Found : C, 55.71; H, 4.90; N, 11.39

## Example 12

- Sodium 5,6-cis-3-[3-(6-methylpyridazinyl)thio]-6-(1-hydroxy-1-methylethyl)-7-oxo-1-azabicyclo[3.2.0]hept-2-ene-2-carboxylate-

A 80 mg quantity of 10% Pd-C is added to a mixed solution of 80 mg (0.17 mmol) of 4-nitrobenzyl ester of the subject carboxylic acid in 16 mℓ of tetrahydrofuran, 4 mℓ of methanol, 10 mℓ of a phosphate buffer with a pH of 7.0 and 10 mℓ of water, and hydrogenation is carried out at room temperature under atmospheric pressure for 70 minutes. The catalyst is filtered out, and washed with a small amount of water. The filtrate and washings are combined and the organic solvent is distilled off under reduced pressure. The resultant aqueous solution is washed with ethyl acetate and concentrated under reduced pressure. The concentrate is chromatographed on a column of Amberlite XAD-2 ($H_2O$, 5% EtOH), and the fractions which show the UV spectrum having the absorption at 296 nm are collected and lyophilized, thus giving the subject compound as colorless powder (34 mg).

IR $\nu_{max}^{KBr}$ $cm^{-1}$: 1750

UV $\lambda_{max}^{H_2O}$ nm ($E_{1cm}^{1\%}$): 296 (286)

NMR (100 MHz, $D_2O$)δ: 1.20, 1.41(each 3H,s x 2, $-CH_3$ x 2), 2.67 (3H,s,$-CH_3$), 2.72(1H,dd,J=17,10Hz,$>C<^H_H$), 3.61(1H,dd,J=17,9Hz, $>C<^H_H$), 3.74(1H,d,J=6Hz,>CH-CO-), 4.35(1H,m,>CH-N<), 7.60(1H, d,J=8Hz,arom.H), 7.81(1H,d,J=8Hz,arom.H).

Example 13

- 4-Nitrobenzyl 5,6-cis-3-(2-acetamidoethylthio)-6-[1-(2-methoxyethoxymethoxy)-1-methylethyl]-7-oxo-1-azabicyclo[3.2.0]-hept-2-ene-2-carboxylate -

To a solution of 0.30 g (0.67 mmol) of 4-nitrobenzyl 5,6-cis-6-[1-(2-methoxyethoxymethoxy)-1-methylethyl]-1-azabicyclo[3.2.0]heptane-3,7-dione-2-carboxylate as prepared Reference Example 22 in 25 mℓ of anhydrous acetonitrile are added, under nitrogen atmosphere at 0°C, 0.145 mℓ (0.83 mmol) of diisopropylethylamine and then 0.172 mℓ (0.83 mmol) of diphenylphosphoryl chloride, and stirring is effected for 75 minutes. After 0.17 mℓ (1.0 mmol) of diisopropylethylamine and 120 mg (1.0 mmol) of N-acetylcysteamine are added to the mixture and stirring is conducted at 0°C for 2 hours under nitrogen atmosphere, 0.17 mℓ (1.0 mmol) of diisopropylethylamine and 120 mg (1.0 mmol) of N-acetylcysteamine are further added and stirring is continued at 0°C for 17 hours. The reaction solution is diluted with ethyl acetate, and the mixture is poured into ice-water. The organic layer is separated, washed with water, dried ($Na_2SO_4$) and the solvent is distilled off. The residue is chromatographed on a column of silica gel (ethyl acetate, ethyl acetate-methanol ratio = 95:5), thus giving the subject compound as colorless crystals (270 mg).

m.p. 128-130°C

IR $\nu_{max}^{Nujol}$ $cm^{-1}$: 3310, 1775, 1700, 1650

UV $\lambda_{max}^{EtOH}$ ($E_{1cm}^{1\%}$): 265 (209), 315 (239)

NMR (60MHz, $CDCl_3$) δ: 1.35, 1.52 (each 3H, s x 2, $-CH_3$x2), 1.96 (3H, s, $-COCH_3$), 2.8-3.7 (10H, m, $-SCH_2CH_2N<$, $>CH-CO-$, $>C<^{H}_{H}$, $-OCH_2CH_2O-$),

3.33(3H,s,$-OCH_3$), 3.9-4.4(2H,m,>C<$^H_H$,>CH-N<), 4.77(2H,s,$-OCH_2O-$), 5.35(2H,ABq,J=20,14Hz,$-OCH_2Ar$), 6.2(1H,b,-NHCO-), 7.61(2H,d, J=9Hz,arom.H), 8.20(2H,d,J=9Hz,arom.H)

Elemental analysis, for $C_{25}H_{33}N_3O_9S$

Calcd.: C, 54.44; H, 6.03; N, 7.61

Found : C, 54.40; H, 5.78; N, 7.61

Example 14

- Sodium 5,6-cis-3-(2-acetamidoethylthio)-6-[1-(2-methoxyethoxymethoxy)-1-methylethyl]-7-oxo-1-azabicyclo[3.2.0]hept-2-ene-2-carboxylate -

A 130 mg quantity of 10% Pd-C is added to a mixed solution of 130 mg (0.24 mmol) of 4-nitrobenzyl ester of the subject carboxylic acid and 40 mg of sodium hydrogen carbonate in 13 mℓ of tetrahydrofuran and 13 mℓ of water, and hydrogenation is carried out at room temperature under atmospheric pressure for 2 hours. After the catalyst is filtered out and washed with a small amount of water, the filtrate and washings are combined, and the tetrahydrofuran is distilled off under reduced pressure. The resultant aqueous solution is washed with ethyl acetate and concentrated, and the concentrate is chromatographed on a column of Diaion HP-20 (produced by Mitsubishi Chemical Ind. Ltd.) ($H_2O$, 5% EtOH, 10% EtOH). The fractions which show the UV spectrum having the absorption at 298 nm are collected and lyophilized, thus giving the subject compound as colorless powder (90 mg).

IR $\nu_{max}^{KBr}$ $cm^{-1}$; 1750, 1640

UV $\lambda_{max}^{EtOH}$ nm ($E_{1cm}^{1\%}$): 298 (212)

NMR (100MHz, $D_2O$)δ: 1.36, 1.46(each 3H, s x 2, $-CH_3$x2), 2.01(3H, s,$-COCH_3$), 2.8-3.9(11H,m,$-SCH_2CH_2N<$, $-CH_2-$,>CHCO-,$-OCH_2CH_2O-$), 3.33(3H,s,$-OCH_3$), 4.30(1H,m,>CH-N<), 4.83(2H,s,$-OCH_2O-$)

Example 15

- 4-Nitrobenzyl 5,6-cis-3-ethoxycarbonylmethylthio-6-(1-hydroxy-1-methylethyl)-7-oxo-1-azabicyclo[3.2.0]hept-2-ene-2-carboxylate -

To a solution in 10 mℓ of anhydrous actonitrile of

4-nitrobenzyl 5,6-cis-6-(1-hydroxy-1-methylethyl)-1-azabicyclo-[3.2.0]heptane-3,7-dione-2-carboxyalte as prepared in accordance with the procedure of Reference Example 21 from 117 mg (0.25 mmol) of 5,6-cis-4-[3-diazo-3-(4-nitrobenzyloxycarbonyl)-2-oxopropyl]-3-(1-hydroxy-1-methylethyl)azetidin-2-one (benzene-solvate) are added, under nitrogen atmosphere at 0°C, 0.055 mℓ(0.31 mmol) of diisopropylethylamine and then 0.065 mℓ (0.31 mmol) of diphenylphosphoryl chloride, and stirring is effected for 90 minutes. To the mixture are added 0.064 mℓ (0.36 mmol) of diisopropylethylamine and then 0.041 mℓ (0.36 mmol) of methyl mercaptoacetate, and stirring is continued at 0°C for 3 hours. The reaction solution is diluted with ethyl acetate, and the mixture is poured into ice-water. The organic layer is separated, dried ($Na_2SO_4$) and the solvent is distilled off. The residue is chromatographed on a column of Florisil (100 to 200 mesh) (ethyl acetate-hexane ratio = 2:3), thus giving the subject compound as colorless crystals (26 mg).

m.p. 120-121°C

IR $\nu_{max}^{KBr}$ cm$^{-1}$: 1770

UV $\lambda_{max}^{EtOH}$ nm ($E_{1cm}^{1\%}$) : 266 (260), 315 (275)

NMR (90MHz, $CDCl_3$) δ: 1.28(3H,t,J=7.5Hz,$-CH_2CH_3$), 1.29, 1.53 (each 3H, s x 2, $-CH_3$x2), 1.68(1H,s,-OH), 3.09(1H,q,J=17,9Hz,$>C<^H_H$), 3.51-3.63(3H,m,>CH-CO-,$-S-C\underline{H}_2-CO-$), 4.06-4.38(4H,m,>CH-N<, $>C<^H_{\underline{H}}$,$-C\underline{H}_2CH_3$), 5.37(2H,ABq,J=24,15Hz,$-OCH_2Ar$), 7.68(2H,d,J=8Hz,arom.H), 8.26(2H,d,J=8Hz,arom.H).

Elemental analysis, for $C_{21}H_{24}N_2O_8S$

Calcd.: C, 54.30; H, 5.21; N, 6.03

Found : C, 54.05; H, 5.05; N, 5.94

Example 16

- Sodium 5,6-cis-3-ethoxycarbonylmethylthio-6-(1-hydroxy-1-methylethyl)-7-oxo-1-azabicyclo[3.2.0]hept-2-ene-2-carboxylate -

A 60 mg quantity of 10% Pd-C is added to a mixed solution of 60 mg (0.13 mmol) of 4-nitrobenzyl 5,6-cis-3-ethoxycarbonylmethylthio-6-(1-hydroxy-1-methylethyl)-7-oxo-1-azabicyclo[3.2.0]hept-2-ene-2-carboxylate and 16 mg (0.19 mmol) of sodium hydrogen carbonate in 6 mℓ of tetrahydrofuran and 6 mℓ of water, and hydrogenation is carried out at room temperature under atmospheric pressure for 2 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$, 5% EtOH). The fractions which show the UV spectrum having the absorption at 296 nm are collected and lyophilized, thus giving the subject compound as colorless powder (28 mg).

IR $\nu_{max}^{KBr}$ $cm^{-1}$: 1750

UV $\lambda_{max}^{H_2O}$ nm ($E_{1cm}^{1\%}$): 296 (256)

NMR (90MHz,$D_2O$) : 1.42(3H,t,J=7.5Hz,$-CH_2C\underline{H}_3$), 1.43, 1.53(each 3H,s x 2,$-CH_3$x2),3.20(1H,q,J=17,9Hz,$>C<\frac{H}{\underline{H}}$), 3.84(2H,s,$-SC\underline{H}_2CO-$), 3.86(1H,d,J=6Hz,>CH-CO-), 3.98-4.57(2H,m,>CH-N<,$>C<\frac{H}{\underline{H}}$), 4.38 (2H,q,$-C\underline{H}_2CH_3$).

Example 17

- 4-Nitrobenzyl 5,6-cis-3-benzylthio-6-(1-hydroxy-1-methylethyl)-7-oxo-1-azabicyclo[3.2.0]hept-2-ene-2-carboxylate -

To a solution in 20 mℓ of anhydrous actonitrile of 4-nitrobenzyl 5,6-cis-6-(1-hydroxy-1-methylethyl)-1-azabicvclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene-solvate) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. To the mixture are added 0.17 mℓ (0.97 mmol) of diisopropylethylamine and then 0.11 mℓ (0.97 mmol) of benzylmercaptane, and stirring is continued at 0°C for 24 hours. The reaction solution is diluted with ethyl acetate, and the mixture is poured into ice-water. The organic

layer is separated out, washed with water, dried ($Na_2SO_4$) and the solvent is distilled off. The residue is chromatographed on a column of Florisil (100-200 mesh) (ethyl acetate-hexane ratio = 1:2), thus giving the subject compound as colorless crystals (96 mg).

m.p. 180-181°C

IR $\nu_{max}^{KBr}$ $cm^{-1}$: 1760

UV $\lambda_{max}^{EtOH}$ nm($E_{1cm}^{1\%}$): 265 (255), 320 (312).

NMR (60MHz,$CDCl_3$)$\delta$: 1.20, 1.49(each 3H, s x 2, $-CH_3$x2), 1.67(1H, s,-OH), 2.96(1H,q,J=17,9Hz,>C<$\frac{H}{H}$), 3.58(1H,d,J=6Hz,>CH-CO-), 4.13(2H,s,$-SCH_2Ar$), 3.9-4.3(2H,m,>C<$^H_H$,>CH-N<), 5.36(2H,ABq, J=24,15Hz,$-OCH_2Ar$), 7.28(5H,s,arom.H), 7.57(2H,d,J=8Hz,arom.H), 8.18(2H,d,J=8Hz,arom.H).

Elemental analysis, for $C_{24}H_{24}N_2O_6S$

Calcd.: C, 61.53; H, 5.16; N, 5.98

Found : C, 61.82; H, 5.09; N, 5.75

## Example 18

- Sodium 5,6-cis-3-benzylthio-6-(1-hydroxy-1-methylethyl)-7-oxo-1-azabicyclo[3.2.0]hept-2-ene-2-carboxylate -

A 90 mg quantity of 10% Pd-C is added to a mixed solution of 90 mg (0.19 mmol) of 4-nitrobenzyl ester of the subject carboxylic acid and 24 mg (0.29 mmol) of sodium hydrogen carbonate in 9 mℓ of tetrahydrofuran and 9 mℓ of water, and hydrogenation is carried out at room temperature under atmospheric pressure for 2 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$, 10% EtOH). The fractions which show the UV spectrum having the absorption at 300 nm are collected and lyophilized, thus giving the subject compound as colorless powder (30 mg).

IR $\nu_{max}^{KBr}$ $cm^{-1}$: 1740

UV $\lambda_{max}^{H_2O}$ nm ($E_{1cm}^{1\%}$): 300 (275).

NMR (90Mhz,$D_2O$)$\delta$: 1.39, 1.51(each 3H,s x 2, $-CH_3$ x 2), 3.17(1H,dd, J=17,10Hz,>C<$\frac{H}{H}$), 3.78(1H,d,J=6Hz,>CH-CO-), 3.80-4.43(2H,m,>C<$^H_H$,

>CH-N<), 4.28(2H,s,$-CH_2$-Ar), 7.60(5H,s,arom.H).

Example 19

- 4-Nitrobenzyl 5,6-cis-3-(3-pyridazinylthio)-6-(1-hydroxy-1-methylethyl)-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene-solvate) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. After 118 mg (1.0 mmol) of lithium salt of 3-mercaptopyridazine is added to the mixture and stirring is conducted at 0°C for 2 hours, 59 mg (0.5 mmol) of lithium salt of 3-mercaptopyridazine is further added, and stirring is continued for additional 18 hours. The reaction solution is diluted with ethyl acetate, and the mixture is poured into an aqueous sodium chloride solution under ice-cooling. The organic layer is separated out, washed with an aqueous sodium chloride solution, dried ($Na_2SO_4$) and the solvent is distilled off. The residue is treated with ethyl ether, thus giving the subject compound as slightly orange crystals (109 mg).

m.p. 159-160°C.

IR $\nu_{max}^{KBr}$ $cm^{-1}$ : 1750

UV $\lambda_{max}^{MeOH}$ nm ($E_{1cm}^{1\%}$): 264 (274), 310 (222)

Example 20

- Sodium 5,6-cis-3-(3-pyridazinylthio)-6-(1-hydroxy-1-methylethyl)-7-oxo-1-azabicyclo[3.2.0]hept-2-ene-2-carboxylate -

A 95 mg quantity of 10% Pd-C is added to a mixed solution of 95 mg (0.20 mmol) of 4-nitrobenzyl ester of the subject carboxylic acid in 20 mℓ of tetrahydrofuran, 5 mℓ of methanol, 12 mℓ of a phosphate buffer with a pH of 7.0 and 12 mℓ of water, and hydrogenation is carried out at room

temperature under atmospheric pressure for 90 minutes. After the catalyst is filtered out and washed with a small amount of water, the filtrate and washings are combined, and the organic solvent is distilled off. The resultant aqueous solution is washed with ethyl acetate and concentrated under reduced pressure. The concentrate is chromatographed on a column of Amberlite XAD-2 ($H_2O$, 5% EtOH), and the fractions which show the UV spectrum having the absorption at 296 nm are collected and lyophilized, thus giving the subject compound as colorless powder (31 mg).

IR $\nu_{max}^{KBr}$ $cm^{-1}$ : 1750

UV $\lambda_{max}^{H_2O}$ nm ($E_{1cm}^{1\%}$): 296 (276)

NMR (90MHz,$D_2O$)δ: 1.32, 1.53(each 3H,s x 2,-$CH_3$x2), 2.90(1H,dd, J=17, 10Hz,>C<$\frac{H}{H}$), 3.71(1H,dd,J=17,9Hz,>C<$\frac{H}{H}$), 3.86(1H,d,J=6Hz, >CH-CO-), 4.47(1H,m,>CH-N<), 7.76-9.26(3H,m,arom.H).

## Example 21

- 4-Nitrobenzyl 5,6-cis-3-phenylthio-6-(1-hydroxy-1-methylethyl)-7-oxo-1-azabicyclo[3.2.0]hept-2-ene-2-carboxylate -

To a solution in 16 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 188 mg (0.4 mmol) of 5,6-cis-4-[3-diazo-3-(4-nitrobenzyloxycarbonyl)-2-oxopropyl]-3-(1-hydroxy-1-methylethyl)azetidin -2-one (benzene-solvate) are added, under nitrogen atmosphere at 0°C, 0.08 mℓ (0.45 mmol) of diisopropylethylamine and then 0.093 mℓ (0.45 mmol) of diphenylphosphoryl chloride, and stirring is effected for 90 minutes.0.14 mℓ (0.8 mmol) of diisopropylethylamine and 0.082 mℓ (0.8 mmol) of thiophenol are added to the mixture, which is then stirred at 0°C for 3 hours and allowed to stand for 16 hours. The reaction solution is diluted with ethyl acetate, and the mixture is washed with cooled water, dried ($Na_2SO_4$) and the solvent is distilled off. The residue is chromatographed on a column of silica gel (hexane-ethyl acetate ratio = 2:1), thus giving the subject compound as colorless crystals (100 mg).

m.p. 165-167°C (decomp.).

IR $\nu_{max}^{Nujol}$ $cm^{-1}$: 3460, 1770, 1750, 1690

UV $\lambda_{max}^{EtOH}$ nm ($E_{1cm}^{1\%}$): 265 (282), 315 (333)

NMR (60MHz, $CDCl_3$)δ: 1.12, 1.48(each 3H,s x 2, $-CH_3$ x 2), 2.39 (1H,dd,J=16,8Hz,>C<$\frac{H}{H}$), 3.53(1H,d,J=6Hz,>CHCO-), 3.4-4.4(2H, m,>CHN<,>C<$\frac{H}{H}$), 5.41(2H,ABq,J=22,14Hz,$-OCH_2Ar$), 7.4(5H,m,arom.H), 7.67(2H,d,J=9Hz,arom.H), 8.22(2H,d,J=9Hz,arom.H).

Elemental analysis, for $C_{23}H_{22}N_2O_6S$

Calcd.: C, 60.78; H, 4.88; N, 6.16

Found : C, 61.24; H, 4.87; N, 6.00

Example 22

- Sodium 5,6-cis-3-phenylthio-6-(1-hydroxy-1-methylethyl)-7-oxo-1-azabicyclo[3.2.0]hept-2-ene-2-carboxylate -

A 100 mg quantity of 10% Pd-C is added to a mixed solution of 100 mg (0.22 mmol) of 4-nitrobenzyl ester of the subject carboxylic acid and 28 mg (0.33 mmol) of sodium hydorgen carbonate in 10 mℓ of tetrahydrofuran and 10 mℓ of water, and hydrogenation is carried out at room temperature under atmospheric pressure for 1 hour. After 100 mg of 10% Pd-C is further added and hydrogenation is conducted for additional 1.5 hours, the catalyst is filtered out and washed with a small amount of water. The filtrate and washings are combined, and the tetrahydrofuran is distilled off under reduced pressure. The resultant aqueous solution is washed with ethyl acetate and concentrated under reduced pressure. The concentrate is chromatographed on a column of Amberlite XAD-2 ($H_2O$, 5% EtOH), and the fractions which show the UV spectrum having the absorption at 300 nm are collected and lyophilized, thus giving the subject compound as colorless crystals (35 mg).

IR $\nu_{max}^{KBr}$ $cm^{-1}$: 1750

UV $\lambda_{max}^{H_2O}$ nm ($E_{1cm}^{1\%}$) : 300 (329)

NMR (90MHz, $D_2O$)δ: 1.25, 1.50(each 3H,s x 2, $-CH_3$ x 2), 2.53(1H, dd,J=17,10Hz,>C<$\frac{H}{H}$), 3.60(1H,dd J=17,9Hz,>C<$\frac{H}{H}$), 3.75(1H,d, J=6Hz,>CH-CO-), 4.25(1H,m,>CH-N<), 7.6(5H,m,arom.H).

Example 23

- 4-Nitrobenzyl 5,6-cis-3-[2-(N,N-dimethylaminoethyl)thio]-6-(1-hydroxy-1-methylethyl)-7-oxo-1-azabicyclo[3.2.0]hept-2-ene-2-carboxylate -

To solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene-solvation product) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. 0.35 mℓ (2.0 mmol) of diisopropylethylamine and 142 mg (1.0 mmol) of N-dimethyl-cysteamine hydrochloride are added to the mixture, which is then stirred at 0°C for 4 hours. The reaction solution is diluted with ethyl acetate, and the mixture is poured into an aqueous sodium chloride solution under ice-cooling. The organic layer is separated out, washed with water, dried ($Na_2SO_4$) and the solvent is distilled off. The residue is treated with a small amount of ethyl acetate, thus giving the subject compound as colorless crystals (84 mg).

m.p. 148-150°C (decomp.)
IR $\nu_{max}^{KBr}$ $cm^{-1}$: 1770
UV $\lambda_{max}^{EtOH}$ nm ($E_{1cm}^{1\%}$): 266 (265), 317.5 (291)
NMR (60MHz, $CDCl_3$)δ: 1.27, 1.52(each 3H, s x 2, $-CH_3$ x 2), 2.26 (6H,s,$-CH_3$x2), 2.2-3.3(5H,m,$>C<^{H}_{H}$,$-S-CH_2CH_2-N<$), 3.57(1H,d, J=6Hz,>CH-CO-), 4.0-4.4(2H,m,>CH-N ,$>C<^{H}_{H}$), 5.33(2H,ABq,J=23, 14Hz,$-CH_2Ar$), 7.60(2H,d,J=8Hz,arom.H), 8.17(2H,d,J=8Hz,arom.H).
Elemetnal analysis, for $C_{21}H_{27}N_3O_6S$
Calcd.: C, 56.11; H, 6.05; N, 9.35
Found : C, 55.62; H, 5.91; N, 9.52

## Example 24

- 4-Nitrobenzyl 5,6-cis-3-[3-(6-methoxypyridazinyl)thio]-6-(1-hydroxy-1-methylethyl)-7-oxo-1-azabicyclo[3.2.0]hept-2-ene-2-carboxylate -

To a solution in 20 mℓ of anhydrous actonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene-solvate) are added, under nitrogen atmosphere at 0°C, 0.11 mℓ (0.63 mmol) of diisopropylethylamine and then 0.13 mℓ (0.63 mmol) of diphenylphosphoryl chloride, and stirring is effected for 90 minutes. After 164 mg (1.1 mmol) of lithium salt of 6-methoxy-3-mercaptopyridazine is added to the mixture and stirring is conducted at 0°C for 2 hours, 82 mg (0.55 mmol) of lithium salt of 6-methoxy-3-mercaptopyridazine is further added and stirring is continued for additional 18 hours. The reaction solution is diluted with ethyl acetate, and the mixture is poured into an aqueous sodium chloride solution under ice-cooling. The organic layer is separated out, washed with an aqueous sodium chloride solution, dried ($Na_2SO_4$) and the solvent is distilled off. The residue is chromatographed on a column of Florisil (100-200 mesh) (ethyl acetate-hexane ratio=3:2), thus giving the subject compounds as yellow crystals (46 mg). m.p. 169-171°C.

IR $\nu_{max}^{KBr}$ $cm^{-1}$: 1770

UV $\lambda_{max}^{MeOH}$ nm ($E_{1cm}^{1\%}$) : 264 (310), 308 (283)

## Example 25

- 4-Nitrobenzyl 5,6-cis-3-(4-pyridylthio)-6-(1-hydroxy-1-methylethyl)-7-oxo-1-azabicyclo[3.2.0]hept-2-ene-2-carboxylate -

To a solution in 4 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared in accordance with the procedure of Reference Example 21 from 47 mg (0.1 mmol) of 5,6-cis-4-[3-diazo-3-(4-nitrobenzyloxycarbonyl)-2-oxopropyl]-3-(1-hydroxy-1-methylethyl)azetidin-2-one (benzene solvate) are added, under nitrogen atmosphere at 0°C, 0.029 mℓ (0.17 mmol) of diisopropylethylamine and then 0.036 mℓ

(0.17 mmol) of diphenylphosphoryl chloride, and stirring is effected for 90 minutes. After 23 mg (0.2 mmol) of lithium salt of 4-mercaptopyridine is added to the mixture and stirring is conducted at 0°C for 1 hour, 23 mg (0.2 mmol) of lithium salt of 4-mercaptopyridine is further added, and stirring is continued for 5 hours. The reaction solution is diluted with ethyl acetate, and the mixture is poured into ice-water. The organic layer is separated out, washed with water, dried ($Na_2SO_4$) and the solvent is distilled off. The residue is chromatographed on a column of silica gel (ethylacetate-hexane ratio = 7:3), thus giving the subject compound as colorless crystals (9 mg).

m.p. 175-178°C (decomp.)

IR $\nu_{max}^{Nujol}$ cm$^{-1}$: 1760

UV $\lambda_{max}^{EtOH}$ nm ($E_{1cm}^{1\%}$): 264 (318), 318 (172)

## Example 26

- 5,6-cis-3-[2-(N,N-Dimethylaminoethyl)thio]-6-(1-hydroxy-1-methylethyl)-7-oxo-1-azabicyclo[3.2.0]hept-2-ene-2-carboxylic acid -

A 100 mg quantity of 10% Pd-C is added to a mixed solution of 90 mg (0.20 mmol) of 4-nitrobenzyl ester of the subject carboxylic acid in 18 mℓ of tetrahydrofuran, 9 mℓ of water and 9 mℓ of a phosphate buffer with a pH of 7.0, and hydrogenation is conducted under atmospheric pressure and at room temperature for 2 hours. After the catalyst is filtered out and washed with a small amount of water, the filtrate and washings are combined and the organic solvent is distilled off under reduced pressure. The resultant aqueous solution is washed with ethyl acetate and concentrated under reduced pressure. The concentrate is chromatographed on a column of Amberlite XAD-2 ($H_2O$, 5% EtOH), and fractions which show the UV spectrum having the absorption at 294 nm are collected and lyophilized, thus giving the subject compound as colorless powder (17 mg).

IR $\nu_{max}^{KBr}$ cm$^{-1}$: 1750

UV $\lambda_{max}^{H_2O}$ nm ($E_{1cm}^{1\%}$) : 294 (177).

NMR (90MHz,$D_2O$)$\delta$: 1.42, 1.55(each 3H,s x 2, $-CH_3$ x 2), 2.75(6H,s,

$-CH_3$ x 2),3.19(4H,s,$-SCH_2CH_2N$<), 3.2(1H,m,>C<$\frac{H}{H}$), 3.85(1H,d, J=6Hz,>CH-CO-), 3.7-4.2(1H,m,>C<$\frac{H}{\underline{H}}$), 4.45(1H,m,CH-N<).

Example 27

- Sodium 5,6-cis-3-[3-(6-methoxypyridazinyl)thio]-6-(1-hydroxy-1-methylethyl)-7-oxo-1-azabicyclo[3.2.0]hept-2-ene-2-carboxylate -

A 71 mg quantity of 10% Pd-C is added to a mixed solution of 71 mg (0.146 mmol) of 4-nitrobenzyl ester of the subject carboxylic acid in 15 mℓ of tetrahydrofurane, 4 mℓ of methanol, 9 mℓ of a phosphate buffer with a pH of 7.0 and 9 mℓ of water, and hydrogenation is conducted at room temperature under atmospheric pressure for 2 hours. After 71 mg of 10% Pd-C is further added to the reaction solution and hydorgenation is carried out for additional 1 hour, the catalyst is filtered out and washed with a small amount of water. The filtrate and washings are combined, and the organic solvent is distilled off under reduced pressure. The resultant aqueous solution is washed with ethyl acetate and concentrated under reduced pressure. The concentrate is chromatographed on a column of Amberlite XAD-2 ($H_2O$, 5% EtOH). The fractions which show the UV spectrum having the absorption at 294 nm are collected and lyophilized, thus giving the subject compound as colorless powder (26 mg).

IR $\nu_{max}^{KBr}$ $cm^{-1}$: 1750

UV $\lambda_{max}^{H_2O}$ nm ($E_{1cm}^{1\%}$): 294 (282)

NMR (90MHz,$D_2O$) δ: 1.30, 1.54(each 3H,s x 2,$-CH_3$ x 2), 2.77(1H,dd, J=17,10Hz,>C<$\frac{H}{\underline{H}}$), 3.68(1H,dd,J=17,9Hz,>C<$\frac{\underline{H}}{H}$), 3.72(1H,d,J=6Hz, >CH-CO-), 4.21(3H,s,$-OCH_3$), 4.38(1H,m,>C$\underline{H}$-N<), 7.39(1H,d,J=8Hz, arom.H), 7.93(1H,d,J=8Hz,arom.H)

Example 28

- 4-Nitrobenzyl 5,6-cis-3-ethylthio-6-(1-hydroxy-1-methylethyl)-7-oxo-1-azabicyclo[3.2.0]hept-2-ene-2-carboxylate -

To a solution, in 25 ml of anhydrous acetonitrile, of 5,6-cis-6-(1-hydroxy-1-methylethyl)-1-azabicyclo[3.2.0]-heptane-3,7-dione-2-carboxylic acid 4-nitrobenzyl ester prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidine-2-one(benzene solvate) in a manner as described in Reference Example 21 is added 0.11 ml (0.63) mmol) of diisopropylethylamine at 0°C under nitrogen atmosphere, followed by addition of 0.13 ml (0.63 mmol) of diphenylphosphoryl chloride and stirring for 90 minutes. To this mixture is added 0.22 ml (1.3 mmol) of diisopropylethylamine, followed by addition of 0.1 ml (1.3 mmol) of ethylmercaptane, and the whole mixture is stirred at 0°C for 17 hours. The solvent is evaporated off under reduced pressure, and the residue is dissolved in ethyl acetate. The solution is washed with water and dried on anhydrous sodium sulfate, followed by removal of the solvent by evaporation. The residue is subjected to column chromatography (hexane-ethyl acetate=1:1) using Florisil to thereby afford the titled compound (100 mg) as coloress crystals, m.p. 77-80°C (softening), 133-135°C (re-melting).

IR $\nu_{max}^{KBr} cm^{-1}$: 1770, 1740, 1690

UV $\lambda_{max}^{EtOH} nm (E_{1cm}^{1\%})$: 262(247), 317.5(244)

NMR (60MHz, $CDCl_3$)$\delta$: 1.27, 1.47 (each 3H, s x 2, $-CH_3$ x 2), 1.30(3H, t, J=7Hz, $-CH_2C\underline{H}_3$), 2.87(2H, q, J=7Hz, $-C\underline{H}_2CH_3$), 2.8-3.2(1H, m, $>C<^{H}_{\underline{H}}$), 3.57(1H, d, J=6Hz, $>CH-CO-$),3.9-4.4(2H, m, $>C<^{H}_{\underline{H}}$, $>CH-N<$), 5.30(2H, ABq, J=22, 14Hz, $-OCH_2Ar$), 7.57(2H, d, J=8Hz, arom. H), 8.10(2H, d, J=8Hz, arom. H)

Elemental Analysis:

Calcd. for $C_{19}H_{22}N_2O_6S \cdot H_2O$: C 53.76; H 5.69; N 6.59

Found: C 54.12; H 5.36; N 6.27

Example 29

- Sodium 5,6-cis-3-ethylthio-6-(1-hydroxy-1-methylethyl)-7-oxo-1-azabicyclo[3.2.0]hept-2-ene-2-carboxylate -

To a mixture of 70 mg (0.17 mmol) of 4-nitrobenzylester of the titled carboxylic acid, 14 mg of sodium hydrogen carbonate, 7 ml of tetrahydrofuran and 7 ml of phosphate buffer of pH 7.0 is added 70 mg of 10% Pd-C. The whole mixture is subjected to hydrogenation for 1.5 hour at room temperature under atmospheric pressure. To the resultant is further added 70 mg of 10% Pd-C, and the mixture is subjected to further hydrogenation for three hours, followed by removal of the catalyst by filtration and washing with a small volume of water. The filtrate and the washing are combined, and tetrahydrofuran is distilled off under reduced pressure. The resulting aqueous solution is washed with ethyl acetate and concentrated under reduced pressure. The concentrate is subjected to column chromatography ($H_2O$) using Amberlite XAD-2. Fractions showing UV spectrum having absorption bands at 297 nm are collected, followed by lyophilization to afford the titled compound as colorless powder (21 mg).

IR $\nu_{max}^{KBr} cm^{-1}$: 1750

UV $\lambda_{max}^{H_2O} nm (E_{1cm}^{1\%})$: 297(268)

NMR (90MHz, $D_2O$)$\delta$: 1.38(3H, t, J=7Hz, $-CH_2C\underline{H}_3$), 1.43, 1.53(each 3H, s x 2, $-CH_3$ x 2), 2.98(2H, q, J=7Hz, $-C\underline{H}_2CH_3$), 3.23(1H, dd, J=17, 10Hz, $>C<^{\underline{H}}_{H}$), 3.83(1H, d, J=6Hz, $>CH-CO-$), 3.98(1H, dd, J=17, 9Hz, $>C<^{H}_{\underline{H}}$), 4.3-4.6(1H, m, $>CH-N<$)

Example 30

- 4-Nitrobenzyl 5,6-cis-3-(2-hydroxyethylthio)-6-(1-hydroxy-1-methylethyl)-7-oxo-1-azabicyclo[3.2.0]hept-2-ene-2-carboxylate -

To 4-nitrobenzyl 5,6-cis-6-(1-hydroxy-1-methylethyl)-1-azabicyclo[3.2.0]heptane-3,7-dione-2-carboxylate prepared from 351 mg (0.75 mmol) of 5,6-cis-4-[3-diazo-3-(4-nitrobenzyloxycarbonyl)-2-oxopropyl]-3-(1-hydroxy-1-methylethyl)-azetidin-2-one (benzene solvate) after the manner as described in Reference Example 21, which is dissolved in 30 ml of anhydrous acetonitrile, is added 0.165 ml (0.93 mmol) of diisopropylethylamine at 0°C under nitrogen atmosphere, followed by addition of 0.195 ml (0.93 mmol) of diphenylphosphoryl chloride and stirring for 90 minutes. To this mixture is added 0.255 ml (1.44 mmol) of diisopropylethylamine, followed by further addition of 0.105 ml (1.44 mmol) of 2-mercapto ethanol and stirring for 16 hours. The reaction mixture is diluted with ethyl acetate, which is poured into an aqueous sodium chloride solution under ice-cooling, then the organic layer thus formed is separated. The organic layer is washed with water and dried over $Na_2SO_4$, then the solvent is distilled off. The residue is subjected to column chromatography (hexane-ethyl acetate=3:1) using Florisil to thereby afford 142 mg of the titled compound as colorless crystals.

m.p. 129-133°C (decomp.)

IR $\nu_{max}^{KBr}cm^{-1}$: 1770

UV $\lambda_{max}^{EtOH}nm(E_{1cm}^{1\%})$: 265(262), 318(280)

NMR (60MHz, $CDCl_3$)$\delta$ : 1.27, 1.49(each 3H, s x 2, $-CH_3$ x 2); 1.95(2H, b.s., -OH x 2), 2.9-3.1(3H, m, $-SC\underline{H}_2CH_2-$, $>C<^{H}_{\underline{H}}$), 3.55(1H, d, J=6Hz, $>CH-CO-$ ), 3.6-4.3(4H, m, $-SCH_2C\underline{H}_2-OH$, $>CH-N<$, $>C<^{\underline{H}}_{H}$ ), 5.32(2H, ABq, J=24, 15Hz, $-OCH_2Ar$), 7.60(2H, d, J=8Hz, arom. H), 8.21(2H, d, J=8Hz, arom. H)

Elemental Analysis:

Calcd. for $C_{19}H_{22}N_2O_7S$: C 54.02; H 5.25; N 6.63

Found: C 53.77; H 5.16; N 6.20

Example 31

- Sodium 5,6-cis-3-(2-hydroxyethylthio)-6-(1-hydroxy-1-methylethyl)-7-oxo-1-azabicyclo[3,2,0]hept-2-ene-2-carboxylate -

To a mixture of 91 mg (0.29 mmol) of 4-nitrobenzyl ester of the titled carboxylic acid, 37 mg (0.44 mmol) of sodium hydrogen carbonate, 9 ml of tetrahydrofuran and 9 ml of water is added 91 mg of 10% Pd-C, followed by hydrogenation for two hours under atmospheric pressure at room temperature. The catalyst is separated by filtration and washed with a small volume of water. The filtrate combined with the washing is subjected to distillation under reduced pressure to remove tetrahydrofuran. The remaining aqueous solution is washed with ethyl acetate, and concentrated under reduced pressure. The concentrate is subjected to a column chromatography ($H_2O$) using Amberlite XAD-2. Fractions showing UV spectrum having absorption at 298 nm are collected and lyophilised to afford the titled compound as colorless powder (33 mg).

IR $\nu_{max}^{KBr}cm^{-1}$: 1750

UV $\lambda_{max}^{H_2O}nm(E_{1cm}^{1\%})$: 298(248)

NMR (90MHz, $D_2O$)$\delta$: 1.45, 1.60 (each 3H, s x 2, $-CH_3$ x 2), 3.11(2H, t, $-SC\underline{H}_2CH_2OH$), 3.20(1H, dd, J=17, 10Hz, $>C<^{\underline{H}}_{H}$), 3.88(1H, d, J=6Hz, $>CH-CO-$ ), 3.8-4.1(3H, m, $>C<^{H}_{\underline{H}}$, $-SCH_2C\underline{H}_2OH$), 4.45(1H, m, $>CH-N<$ )

Example 32

- 4-Nitrobenzyl 5,6-cis-3-[3-(4-nitrobenzyloxycarbonyl)aminopropylthio]-6-(1-hydroxy-1-methylethyl)-7-oxo-1-azabicyclo-[3,2,0]hept-2-ene-2-carboxylate -

To a solution of 4-nitrobenzyl 5,6-cis-6-(1-hydroxy-1-methylethyl)-1-azabicyclo[3.2.0]heptane-3,7-dione-2-carboxylate, which is prepared from 250 mg (0.53 mmol) of 5,6-cis-4-[3-diazo-3-(4-nitrobenzyloxycarbonyl)-2-oxopropyl]-3-(1-hydroxy-1-methylethyl)azetidin-2-one(benzene solvate) after the

manner of Reference Example 21 in 25 ml of anhydrous acetonitrile is added 0.11 ml (0.63 mmol) of diisopropylethylamine at 0°C under nitrogen atmosphere, followed by further addition of 0.13 ml (0.63 mmol) of diphenyl phosphoryl chloride and stirring for 90 minutes. To this mixture is added 0.14 ml (0.8 mmol) of diisopropylethylamine, followed by further addition of 179 mg (0.64 mmol) of 3-(4-nitrobenzyloxycarbonyl)aminopropanethiol, and the whole mixture is stirred at 0°C for 3 hours. To this mixture are added 0.07 ml (0.4 mmol) of diisopropylethylamine and 89 mg (0.32 mmol) of aminopropanethiol, and the whole mixture is stirred for further 16 hours. The reaction mixture is diluted with ethyl acetate, which is poured into an aqueous sodium chloride solution under ice-cooling, then the organic layer is separated. The organic layer is washed with water and dried over $Na_2SO_4$. The solvent is evaporated off, and the residue is subjected to column chromatography(ethyl acetate-hexane=2:1) using Florisil to afford the titled compound as pale yellow foam-like substance (138 mg).

IR $\nu_{max}^{KBr} cm^{-1}$: 1770

UV $\lambda_{max}^{EtOH} nm (E_{1cm}^{1\%})$: 264(280), 318(164)

NMR (90MHz, $CDCl_3$)$\delta$: 1.29, 1.51 (each 3H, s x 2, $-CH_3$ x 2), 1.8-2.0(2H, m, $-SCH_2C\underline{H}_2CH_2-$), 2.01(1H, s, -OH), 2.7-3.4(5H, m, $-SC\underline{H}_2CH_2C\underline{H}_2N<$, $>C<^{H}_{H}$), 3.59(1H, d, J=6Hz, $>CH-CO-$), 4.0-4.5(2H, m, $>C<^{H}_{\underline{H}}$, $>CH-N<$), 5.18(2H, s, $-OC\underline{H}_2Ar$), 5.31(2H, ABq, J=24, 15Hz, $-OC\underline{H}_2Ar$), 7.2-8.3(8H, m, arom. H)

<u>Example 33</u>

- 5,6-cis-3-(3-Aminopropylthio)-6-(1-hydroxy-1-methylethyl)-7-oxo-1-azabicyclo[3.2.0]hept-2-ene-2-carboxylic acid -

To 134 mg (0.22 mmol) of 4-nitrobenzylester of the titled carboxylic acid dissolved in a mixture of 25 ml of tetrahydrofuran, 12 ml of a pH 7.0 phosphate buffer solution and 12 ml of water is added 134 mg of 10% Pd-C,

followed by hydrogenation under atmospheric pressure at room temperature for 90 minutes. 100 mg of 10% Pd-C is supplemented, and hydrogenation is then conducted for further 2 hours, followed by removing the catalyst by filtration and washing with a small volume of water. The filtrate and the washing are combined, which is subjected to distillation under reduced pressure to remove tetrahydrofuran. Thus obtained aqueous solution is washed with ethyl acetate, concentrated and subjected to column-chromatography ($H_2O$, 5%EtOH) using Amberlite XAD-2. Fractions showing UV spectrum having absorption at 298 nm are combined and lyophilised to afford the titled compound as colorless powder (28 mg).

IR $\nu_{max}^{KBr} cm^{-1}$: 1740

UV $\lambda_{max}^{H_2O} nm(E_{1cm}^{1\%})$: 298(246)

NMR (90MHz, $D_2O$)$\delta$: 1.44, 1.57(each 3H, s x 2, $-CH_3$ x 2), 1.9-2.3(2H, m, $-SCH_2C\underline{H}_2CH_2-$ ), 2.9-3.4(5H, m, $-SC\underline{H}_2CH_2C\underline{H}_2N\lt$, $\gt C\lt^{\underline{H}}_{H}$ ), 3.86(1H, d, J=6Hz, $\gt CH-CO-$ ), 3.8-4.2(1H, m, $\gt C\lt^{H}_{\underline{H}}$), 4.3-4.6(1H, m, $\gt CH-N\lt$ )

Example 34

- 4-Nitrobenzyl 5,6-cis-3-(2-morpholinoethylthio)-6-(1-hydroxy-1-methylethyl)-7-oxo-1-azabicyclo[3.2.0]hept-2-ene-2-carboxylate -

To a solution, in 20 ml of anhydrous acetonitrile, of 4-nitrobenzyl 5,6-cis-6-(1-hydroxy-1-methyl)-1-azabicyclo-[3.2.0]heptane-3,7-dione-2-carboxylate, which was prepared, after the manner of Reference Example 21, from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvate) is added 0.11 ml (0.63 mmol) of diisopropylethylamine under nitrogen atomosphere at 0°C, followed by addition of 0.13 ml (0.63 mmol) of diphenyl chlorophosphate and stirring for 90 minutes. To this mixture is added 0.14 ml (0.8 mmol) of diisopropylethylamine, followed by addition of 94 mg (0.64 mmol) of 2-morpholinoethyl mercaptane and stirring for 6.5 hours. To the resultant are added

0.07 ml (0.4 mmol) of diisopropylethylamine and 47 mg (0.32 mmol) of 2-morpholinoethyl mercaptane, and the mixture is stirred for 15 hours. The reaction mixture is diluted with ethyl acetate, which is added to an aqueous sodium chloride solution under ice-cooling, then the organic layer is separated. The organic layer is washed with water and dried over $Na_2SO_4$. The solvent is then evaporated off, and the residue is subjected to column-chromatography (ethyl acetate) using Florisil to give the titled compound as coloress crystals (100 mg), m.p. 172-173°C.

IR $\nu_{max}^{KBr}cm^{-1}$: 1760

UV $\lambda_{max}^{EtOH}nm$ ($E_{1cm}^{1\%}$): 265 (228), 320(260)

NMR (60 MHz, $CDCl_3$)$\delta$ : 1.26, 1.51 (each 3H, s x 2, $-CH_3$ x 2), 178 (1H, s, OH), 2.32-308 (11H, m,$-SC\underline{H}_2CH_2-$, $>C<^H_H$, 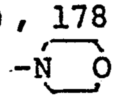 ), 3.51-4.38 (5H, m, $-SCH_2C\underline{H}_2N<$, $>C<^H_{\underline{H}}$, $>CH-N<$, $>CH-CO-$) 5.32 (2H, ABq, J=24, 15Hz, $-OCH_2Ar$), 7.59 (2H, d, J=8Hz, arom. H), 8.19 (2H, d, J=8Hz, arom. H)

Elemental Analysis

Calcd. for $C_{23}H_{29}N_3O_7S \cdot 1/2\ H_2O$: C 55.18; H 6.04; N 8.39

Found: C 54.73; H 6.03; N 8.17

Example 35

- 5,6-cis-3-(2-morpholinoethylthio)-6-(1-hydroxy-1-methylethyl)-7-oxo-1-azabicyclo[3.2.0]hept-2-ene-2-carboxylic acid -

To a mixture of 105 mg (0.2 mmol) of 4-nitro-benzylester of the titled carboxylic acid, 20 ml of tetrahydrofuran, 10 ml of pH 7.0 phosphate buffer and 10 ml of water is added 105 mg of 10% Pd-C, followed by hydrogenation for two hours under atmospheric pressure at room temperature. The catalyst is separated by filtration and washed with a small volume of water. The washing is combined with the filtrate, which is subjected to distillation under

reduced pressure to remove tetrahydrofuran. The resultant aqueous solution is washed with ethyl acetate, followed by concentration under reduced pressure. The concentrate is subjected to column chromatography ($H_2O$, 10% EtOH) using Amberlite XAD-2. The fractions showing UV spectrum having absorption at 294 nm are collected, followed by lyophilization to give the titled compound as colorless powder (12 mg).

IR $\nu_{max}^{KBr} cm^{-1}$: 1760

UV $\lambda_{max}^{H_2O}$ nm ($E_{1cm}^{1\%}$): 294 (165)

NMR (90 MHz, $D_2O$)$\delta$ : 1.44, 1.57 (each 3H, s x 2, $-CH_3$ x 2), 2.91-3.62 (11H, m, $-SC\underline{H}_2CH_2-$, >C<$\underline{H}$/H , 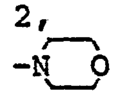 ), 3.79-4.24 (5H, m, $-SCH_2C\underline{H}_2-N<$ , >C<H/$\underline{H}$, >CH-CO-), 4.51 (1H, m, >CH-N< )

## Example 36

- 4-Nitrobenzyl 5,6-cis-3-(4-pyridylmethylthio)-6-(1-hydroxy-1-methylethyl)-7-oxo-1-azabicyclo[3.2.0]hept-2-ene-2-carboxylate -

4-Nitrobenzyl 5,6-cis-6-(1-hydroxy-1-methylethyl)-1-azabicylco[3.2.0]heptane-3,7-dione-2-carboxylate is prepared from 234 mg (0.5 mmol) of 5,6-cis-4-[3-diazo-3-(4-nitrobenzyloxycarbonyl)-2-oxypropyl]-3-(1-hydroxy-1-methylethyl)azetidin-2-one(benzene solvale) after the manner in Reference Example 21, which is dissolved in 20 ml of anhydrous acetonitrile. To the solution is added 0.11 ml (0.63 mmol) of diispropylethylamine at 0°C under nitrogen atmosphere, followed by addition of 0.13 ml (0.65 mmol) of diphenyl phosphoryl chloride and stirring for 90 minutes.

To this mixture is added 0.07 ml (0.4 mmol) of diisopropylethylamine, followed by addition of 80 mg (0.64 mmol) of 4-mercaptomethylpyridine and stirring for 90 minutes. To the resultant are further added 0.07 ml (0.4 mmol) of diisopropylethylamine and 40 mg (0.32 mmol) of 4-mercaptomethylpyridine, and the mixture is stirred for 18 hours. The reaction mixture is diluted with ethyl acetate, which

is poured into an aqueous sodium chloride solution under ice-cooling. The organic layer is separated, washed with water and dried over $Na_2SO_4$. The solvent is then removed by distillation, and the residue is subjected to a column-chromatography (ethyl acetate-hexane = 2:1) using Florisil to afford the titled compound as colorless crystals (104 mg), m.p. 181-182°C.

IR $\nu_{max}^{KBr} cm^{-1}$: 1770

UV $\lambda_{max}^{EtOH}$ nm ($E_{1cm}^{1\%}$): 262 (291), 315 (293)

NMR (90MHz, DMSO-$d_6$)$\delta$ : 1.13, 1.20 (each 3H, s x 2, $-CH_3$ x 2), 3.0-3.29 (1H, m, $>C<^{H}_{H}$), 3.57 (1H, d, J=6Hz, $>CH-CO-$) 3.94-4.37 (2H, m, $>C<^{H}_{H}$, $>CH-N<$ ), 4.17 (2H, s, $-SCH_2-$), 4.63 (1H, s, -OH), 5.31 (2H, ABq, J=21, 15Hz, $-OCH_2Ar$), 7.30-8.69 (8H, m, arom. H)

Elemental Analysis:

Calcd for $C_{23}H_{23}N_3O_6S$: C 58.83; H 4.93; N 8.95

Found: C 58.76; H, 4.98; N 8.94

Example 37

- Sodium 5,6-cis-3-(4-pyridylmethylthio)-6-(1-hydroxy-1-methylethyl)-7-oxo-1-azabicyclo[3.2.0]hept-2-ene-2-carboxylate -

To a solution of 200 mg (0.42 mmol) of 4-nitrobenzylester of the titled carboxylic acid in a mixture consisting of 40 ml of tetrahydrofuran, 20 ml of pH 7.0 phosphate buffer and 20 ml of water is added 200 mg of 10% Pd-C, followed by hydrogenation for three hours under atmospheric pressure at room temperature. The catalyst is removed by filtration and washed with a small volume of water. The filtrate and washing are combined, which is subjected to distillation under reduced pressure to remove tetrahydrofuran. The resultant aqueous solution is washed with ethyl acetate and concentrated under reduced pressure. The concentrate is subjected to column-chromatography ($H_2O$) using Amberlite XAD-2, and the eluate is lyophilized to afford the titled compound as colorless powder.

IR $\nu_{max}^{KBr}cm^{-1}$: 1750

UV $\lambda_{max}^{H_2O}nm$ ($E_{1cm}^{1\%}$) 262 (144), 298 (252)

NMR (90 MHz, $D_2O$)$\delta$: 1.35, 1.49 (each 3H, s x 2, $-CH_3$ x 2), 3.07 (1H, dd, J=17, 10Hz, $>C<^H_H$ ), 3.75 (1H, dd, J=17 ,9 Hz, $>C<^H_H$ ), 3.76 (1H, d, J=6Hz, >CH-CO-), 4.25 (2H, s, $-SCH_2-$), 4.30 (1H, m, >CH-N< ), 7.62 (1H, d, J=6Hz, arom H), 8.61 (1H, d, J=6Hz, arom. H)

Elemental Analysis:

Calcd. for $C_{16}H_{17}N_2O_4SNa\cdot3H_2O$: Na 5.60

Found: Na 5.40

Example 38

- 4-Nitrobenzyl 5,6-cis-3-(2-pyridylmethylthio)-6-(1-hydroxy-1-methylethyl)-7-oxo-1-azabicyclo[3.2.0]hept-2-ene-2-carboxylate -

4-Nitrobenzyl 5,6-cis-6-(1-hydroxy-1-methylethyl)-1-azabicyclo[3.2.0]-heptane-3,7-dione-2-carboxylate is prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one(benzene solvate) after the manner of Reference Example 21, which is then dissolved in 20 ml of anhydrous acetonitrile. To the solution is added 0.11 ml (0.63 mmol) of diisopropylethylamine under nitrogen atmosphere, followed by addition of 0.13 ml (0.65 mmol) of diphenyl chlorophosphate and the mixture is stirred for 90 minutes. To this mixture is added 0.14 ml (0.8 mmol) of diisopropylethylamine, followed by addition of 80 mg (0.64 mmol) of 2-mercaptomethylpyridine and stirring for 90 minutes. To the resultant are added 0.07 ml (0.4 mmol) of diisopropylethylamine and 40 mg (0.32 mmol) of 2-mercaptomethylpyridine. The mixture is stirred for four hours, and then the reaction mixture is diluted with ethyl acetate, which is poured into an aqueous sodium chloride solution under ice-cooling, followed by recovering the organic layer. The organic layer is washed with water and dried over $Na_2SO_4$, followed by removal of the solvent

by evaporation. The residue is subjected to column-chromatography (ethylacetate-hexane = 1:1) using Florisil to yield the titled compound as colorless crystals (102 mg), m.p. 87-88°C.

IR $\nu_{max}^{KBr} cm^{-1}$: 1750

UV $\lambda_{max}^{EtOH} nm(E_{1cm}^{1\%})$: 263(307), 318(279)

NMR (60MHz, $CDCl_3$)$\delta$: 1.25, 1.52(each 3H, s x 2, $-CH_3$ x 2), 1.93(1H, s, -OH), 3.24(1H, dd, J=17, 9Hz, $>C<^{H}_{H}$ ), 3.59(1H, d, J=6Hz, $>CH-CO-$ ), 3.86-4.40(4H, m, $>C<^{H}_{H}$ , $>CH-N<$, $-SCH_2-$), 5.37(2H, ABq, J=24, 15Hz, $-OCH_2Ar$), 7.18-8.39(8H, m, arom. H)

Elemental Analysis:

Calcd. for $C_{23}H_{23}N_3O_6S\cdot1/2\ H_2O$: C 57.73; H 5.05; N 8.78

Found: C 57.81; H 5.05; N 8.80

Example 39

- Sodium 5,6-cis-3-(2-pyridylmethylthio)-6-(1-hydroxy-1-methylethyl)-7-oxo-1-azabicyclo[3.2.0]hept-2-ene-2-carboxylate -

To a mixture of 90 mg (0.19 mmol) of 4-nitrobenzylester of the titled carboxylic acid, 18 ml of tetrahydrofuran, 9 ml of pH 7.0 phosphate buffer and 9 ml of water is added 90 mg of 10% Pd-C, which is subjected to hydrogenation under atmospheric pressure for four hours at room temperature. The catalyst is separated by filtration and washed with a small volume of water. The filtrate and the washing are combined, and tetrahydrofuran is removed by distillation under reduced pressure. The resulting aqueous solution is washed with ethyl acetate and concentrated under reduced pressure. The concentrate is subjected to column chromatography ($H_2O$, 10% EtOH) using Diaion HP-20 (manufactured by Mitsubishi Chemical Industries Ltd.), and fractions eluted with 10% EtOH are lyophilized to yield the titled compound as colorless powder (23 mg).

IR $\nu_{max}^{KBr}cm^{-1}$: 1750

UV $\lambda_{max}^{H_2O}nm(E_{1cm}^{1\%})$: 264(172), 298(256)

NMR (90MHz, $D_2O$) $\delta$: 1.33, 1.42(each 3H, s x 2, $-CH_3$ x 2), 3.13(1H, dd, J=17, 10Hz, $>C<^{H}_{H}$ ), 3.74(1H, dd, J=17, 9Hz, $>C<^{H}_{H}$ ), 3.75(1H, d, J=6Hz, $>$CH-CO- ), 4.30(2H, s, $-SCH_2-$), 4.31(1H, m, $>$CH-N$<$ ), 7.40-8.67(4H, m, arom. H)

Example 40

– 4-Nitrobenzyl 5,6-cis-3-[3-(6-dimethylaminopyridazinyl)thio]-6-(1-hydroxy-1-methylethyl)-7-oxo-1-azabicyclo[3·2·0]hept-2-ene-2-carboxylate –

4-Nitrobenzyl 5,6-cis-6-(1-hydroxy-1-methylethyl)-1-azabicyclo[3·2·0]heptane-3,7,-dione-carboxylate is prepared from 234 mg (0.5 mmol) of 5,6-cis-4-[3-diazo-3-(4-nitrobenzyloxycarbonyl)-2-oxopropyl]-3-(1-hydroxy-1-methylethyl) azetidin-2-one(benzene solvate) after the manner of Reference Example 21, which is dissolved in 20 ml of anhydrous acetonitrile. To the solution is added 0.11 ml (0.63 mmol) of diisopropylethylamine at 0°C, followed by addition of 0.13 ml (0.63 mmol) of diphenyl chlorophosphate and stirring for 90 minutes. To this mixture is added 161 mg (1.04 mmol) of lithium salt of 6-dimethylamino-3-mercaptopyridazine, which is stirred for two hours at 0°C. The reaction mixture is diluted with ethyl acetate, which is poured into an aqueous sodium chloride solution under ice-cooling, and the organic layer is separated. The organic layer is washed with water, dried over $Na_2SO_4$, and the solvent is removed by evaporation. The residue is subjected to column-chromatography using Florisil (ethyl acetate) to yield the titled compound as pale yellow crystals (60 mg), m.p. 178-179°C (decomp.)

IR $\nu_{max}^{KBr}cm^{-1}$: 1760

UV $\lambda_{max}^{MeOH}nm(E_{1cm}^{1\%})$: 267(482), 312.5(356)

Elemental Analysis:

Calcd. for $C_{23}H_{25}N_5O_6S$: C 55.30; H 5.04 N 14.02

Found: C 55.14; H 4.96: N 13.65

Example 41

- 5,6-cis-3-[3-(6-dimethylaminopyridazinyl)thio]-6-(1-hydroxy-1-methylethyl)-7-oxo-1-azabicyclo[3.2.0]hept-2-ene-2-carboxylic acid -

To a mixture of 105 mg (0.21 mmol) of 4-nitrobenzylester of the titled carboxylic acid, 20 ml of tetrahydrofuran, 5 ml of methanol, 12 ml of pH 7 phosphate buffer and 12 ml of water is added 105 mg of 10% Pd-C, which is subjected to hydrogenation for two hours under atmospheric pressure at room temperature. The catalyst is separated by filtration, which is washed with a small volume of water, followed by removal of the organic solvent under reduced pressure. The resulting aqueous solution is washed with ethyl acetate and concentrated under reduced pressure. To concentrate is subjected to column-chromatography ($H_2O$, 5% EtOH) using Amberlite XAD-2, and fractions showing UV spectrum having absorption at 292 nm are collected and lyophilised to yield the titled compound as colorless powder (41 mg).

IR $\nu_{max}^{KBr} cm^{-1}$: 1750

UV $\lambda_{max}^{H_2O} nm (E_{1cm}^{1\%})$: 292(382)

NMR (90MHz, $D_2O$)$\delta$: 1.30, 1.50(each 3H, s x 2, $-CH_3$ x 2), 2.58(1H, dd, J=17, 10Hz, $>C<^{H}_{H}$ ), 3.26(6H, s, $-N(CH_3)_2$), 3.61(1H, dd, J=17, 9Hz, $>C<^{H}_{H}$ ), 3.79(1H, d, J=6Hz, >CH-CO-), 4.3(1H, m, >CH-N< ), 7.22(1H, d, J=8Hz, arom. H). 7.70(1H, d, J=8Hz, arom. H)

Example 42

- 4-Nitrobenzyl 5,6-cis-3-(E-2-acetamidovinylthio)-6-(1-hydroxy-1-methylethyl)-7-oxo-1-azabicyclo[3.2.0]hept-2-ene-2-carboxylate -

4-Nitrobenzyl 5,6-cis-6-(1-hydroxy-1-methylethyl)-1-azabicyclo[3.2.0]heptane-3,7-dione-2-carboxylate is prepared from 234 mg (0.5 mmol) of 5,6-cis-4-[3-diazo-3-(4-nitrobenzyloxycarbonyl)-2-oxopropyl]-3-(1-hydroxy-1-methylethyl)-

azetidin-2-one(benzene solvate) after the manner of Reference Example 21, which is dissolved in 25 ml of anhydrous acetonitrile. To the solution is added 0.12 ml (0.69 mmol) of diisopropylethylamine at 0°C under nitrogen atmosphere, followed by addition of 0.14 ml (0.68 mmol) of diphenyl chlorophosphate and stirring for 90 minutes. To the mixture are added 750 mg (5 mmol) of NaI and 167 mg (0.75 mmol) of silver salt of E-2-acetamidoethylenethiol, which is stirred for two hours, followed by removal of the resulting precipitates by filtration. The solvent is evaporated off. To the residue are added ethyl acetate and an aqueous sodium chloride solution, and insoluble matters are removed. The ethyl acetate layer is separated, washed with water and dried over $Na_2SO_4$. The solvent is evaporated off. The residue is subjected to column-chromatography(ethylacetate-hexane = 2:1, ethyl acetate) using Florisil to yield the titled compound as pale yellow crystals (155 mg), m.p. 182-183°C(decomp.).

IR $\nu_{max}^{KBr}cm^{-1}$: 1760, 1700, 1620

UV $\lambda_{max}^{MeOH}nm(E_{1cm}^{1\%})$: 229.5(375), 262(367), 322.5(371)

NMR (90MHz, DMSO-$d_6$)$\delta$ : 1.15, 1.33(each 3H, s x 2, $-CH_3$ x 2), 1.93(3H, s, $-COCH_3$), 2.9-3.3(1H, m, $>C<^{H}_{H}$ ), 3.55(1H, d, J=6Hz, >CH-CO-), 3.9-4.3(2H, m, $>C<^{H}_{H}$ , >CH-N< ), 4.7(1H, b, -OH), 5.37(2H, ABq, J=21, 14Hz, $-OCH_2Ar$), 5.87(1H, d, J=14Hz, -S-C<u>H</u>=CH-N< ), 7.07(1H, dd, J=14, 13Hz, -S-CH=C<u>H</u>-NH-), 7.75(2H, d, J=8Hz, arom. H), 8.20(2H, d, J=8Hz, arom. H), 10.28(1H, d, J=13Hz, =CH-N<u>H</u>-)

Elemental Analysis:

Calcd. for $C_{21}H_{23}N_3O_7S$: C 54.66; H 5.02; N 9.11

Found: C 54.58; H 5.26; N 8.92

Example 43

- Sodium 5,6-cis-3-(E-2-acetamidovinylthio)-6-(1-hydroxy-1-methylethyl)-7-oxo-1-azabicyclo[3.2.0]hept-2-ene-2-carboxylate -

To a solution of 150 mg (0.33 mmol) of 4-nitrobenzylester of the titled carboxylic acid in 15 ml of tetrahydrofuran

and 15 ml of water are added 36 mg of sodium hydrogen carbonate and 150 mg of 10% Pd-C. The mixture is subjected to hydrogenation under atmospheric pressure for four hours at room temperature (10% Pd-C is supplemented by 150 mg and 50 mg at one hour and three hours after starting the hydrogenation, respectively). The catalyst is separated by filtration, and washed with a small volume of water. The filtrate and the washing are combined. The organic solvent is evaporated off under reduced pressure. The resultant aqueous solution is washed with ethyl acetate and concentrated under reduced pressure. The concentrate is subjected to column-chromatography ($H_2O$, 5%EtOH) using Diaion HP-20 (manufactured by Mitsubishi Chemical Industries Ltd.). The fractions showing UV spectrum having absorptions at 230 nm and 310 nm are combined and liophilized to yield the titled compound as colorless powder (75 mg).

IR $\nu_{max}^{KBr} cm^{-1}$: 1745, 1670, 1615

UV $\lambda_{max}^{H_2O} nm (E_{1cm}^{1\%})$: 230(396), 310(444)

NMR (100MHz, $D_2O$)$\delta$: 1.30, 1.44(each 3H, s x 2, $-CH_3$ x 2), 2.09(3H, s, $-COCH_3$), 3.02(1H, dd, J=18, 10Hz, $>C<^{H}_{\underline{H}}$), 3.72(1H, d, J=6Hz, $>$CH-CO-), 3.75(1H, dd, J=18, 9Hz, $>C<^{H}_{\underline{H}}$), Ca. 4.3(1H, m, $>$CH-N$<$), 6.07(1H, d, J=13Hz, -SC$\underline{H}$=CH-N$<$), 7.14(1H, d, J=13Hz, -SCH=C$\underline{H}$-N$<$)

Example 44

- Sodium 5,6-cis-3-(E-2-acetamidovinylsulfinyl)-6-(1-hydroxy-1-methylethyl)-7-oxo-1-azabicyclo[3.2.0]hept-2-ene-2-carboxylate -

To a solution of 63 mg of sodium 5,6-cis-3-(E-2-acetamidovinylthio)-6-(1-hydroxy-1-methylethyl)-7-oxo-1-azabicyclo-[3.2.0]hept-2-ene-2-carboxylate in 8 ml of water is added 45 mg of m-chloroperbenzoic acid (80% purity), and the mixture is stirred for 30 minutes. The reaction mixture is washed with ethyl acetate, followed by addition of 0.5 ml of pH 7.0 phosphate buffer and concentration under reduced pressure. The concentrate is subjected to column-chromatography

using Diaion HP-20 (manufactured by Mitsubishi Chemical Industries Ltd.). The fractions are combined and purified to yield two kinds of isomers (A, B) of sulfoxide of the titled compound as colorless powders, respectively.

Isomer A (this is eluted before isomer B by column-chromatography)

Yield 26.5 mg

IR $\nu_{max}^{KBr}cm^{-1}$: 1770, 1690, 1620

UV $\lambda_{max}^{H_2O}nm(E_{1cm}^{1\%})$: 252(400), 287(319)

NMR ($D_2O$, 100MHz)$\delta$: 1.34, 1.45(each 3H, s x 2, $-CH_3$ x 2), 2.15(3H, s, $-COCH_3$), 3.14(1H, dd, J=17, 11Hz, $>C<{H \atop H}$), 3.84(1H, d, J=6Hz, $>CH-CO-$), 3.84(1H, dd, J=17, 8Hz, $>C<{H \atop \underline{H}}$), Ca. 4.6(1H, m, $>CH-N<$), 6.30(1H, d, J=14Hz, $-SC\underline{H}=CH-N<$), 7.55(1H, d, J=14Hz, $-SCH=C\underline{H}-N<$)

Isomer B

Yield 34.2 mg

IR $\nu_{max}^{KBr}cm^{-1}$: 1770, 1700, 1620

UV $\lambda_{max}^{H_2O}nm(E_{1cm}^{1\%})$: 244(409), 290(352)

NMR ($D_2O$, 100MHz)$\delta$: 1.34, 1.45(each 3H, s x 2, $-CH_3$ x 2), 2.15(3H, s, $-COCH_3$), 3.05(1H, dd, J=17, 11Hz, $>C<{H \atop H}$), 3.81(1H, d, J=6Hz, $>CH-CO-$), 3.90(1H, dd, J=17, 9Hz, $>C<{H \atop \underline{H}}$), Ca. 4.5(1H, m, $>CH-N<$), 6.39(1H, d, J=14Hz, $-SC\underline{H}=CH-N<$), 7.55(1H, d, J=14Hz, $-SCH=C\underline{H}-N<$)

## Example 45

- Sodium 5,6-cis-3-(3-acetamidopropylthio)-6-(1-hydroxy-1-methylethyl)-7-oxo-1-azabicyclo[3.2.0]hept-2-ene-2-carboxylate -

To a solution of 20 mg of 5,6-cis-3-(3-aminopropylthio)-6-(1-hydroxy-1-methylethyl)-7-oxo-1-azabicyclo[3.2.0]hept-2-ene-2-carboxylic acid in a mixture of 6.5 ml of water and 6.5 ml of tetrahydrofuran is added a solution of 15 mg of sodium hydrogen carbonate in 4.2 ml of water, followed by addition of 8.6 ml of acetic anhydride dissolved in 1.66 ml of tetrahydrofuran and stirring for 20 minutes. The reaction mixture is concentrated under

reduced pressure. The concentrate is washed with ethyl acetate, which is then subjected to column-chromatography ($H_2O$) using Diaion HP-20 (manufactured by Mitsubishi Chemical Industries Ltd.). Fractions showing UV spectrum having absorption at 299 nm are combined and lyophilized to yield the titled compound as colorless powder (10 mg).

IR $\nu_{max}^{KBr}cm^{-1}$: 1750, 1635

UV $\lambda_{max}^{H_2O}nm(E_{1cm}^{1\%})$: 299(248)

NMR (100MHz, $D_2O$)δ: 1.33, 1.44(each 3H, s x 2, $-CH_3$ x 2), 1.7-2.0(2H, m, $-SCH_2C\underline{H}_2CH_2-$), 2.02(3H, s, $-COCH_3$), 2.8-4.0(6H, m, $-SC\underline{H}_2CH_2C\underline{H}_2N<$, $-CH_2-$), 3.74(1H, d, J=6Hz, $>CH-CO-$), 4.20-4.43(1H, m, $>CH-N<$)

## Example 46

- 5,6-cis-3-(2-formimidoylaminoethylthio)-6-(1-hydroxy-1-methylethyl)-7-oxo-1-azabicyclo[3.2.0]hept-2-ene-2-carboxylic acid -

To a solution of 44 mg of 5,6-cis-3-(2-aminoethylthio)-6-(1-hydroxy-1-methylethyl)-7-oxo-1-azabicyclo[3.2.0]hept-2-ene-2-carboxylic acid in 11 ml of pH 7.0 phosphate buffer is added 2N-NaOH under ice-cooling to adjust the pH to be 8.5. To this solution is added 190 mg of benzylformimidate portionwise in the course of five minutes. During the addition, pH of the reaction mixture is maintained at 8.5 with 2N-NaOH. The mixture is stirred for five minutes, followed by addition of 1N HCl to adjust the pH to be 7.0, which is washed with ethyl acetate. The aqueous layer is concentrated and is subjected to column-chromatography ($H_2O$, 3% acetone) using Diaion HP-20 (manufactured by Mitsubishi Chemical Industries Ltd.). Fractions eluting with 3% acetone are combined and lyophilized to yield the titled compound as colorless powder (32 mg).

IR $\nu_{max}^{KBr}cm^{-1}$: 1750, 1715

UV $\lambda_{max}^{H_2O}nm(E_{1cm}^{1\%})$: 296(230)

NMR (90MHz, $D_2O$)$\delta$: 1.43, 1.56(each 3H, s x 2, $-CH_3$ x 2), 3.0-3.4(3H, m, $-SC\underline{H}_2CH_2-$, $>C<^{H}_{\underline{H}}$), 3.6-4.2(3H, m, $-SCH_2C\underline{H}_2N<$, $>C<^{\underline{H}}_{H}$), 3.87(1H, d, J=6Hz, $>CH-CO-$), 4.4(1H, m, $>CH-N<$), 8.00(1H, s, $>N-CH=N-$)

Example 47

- 5,6-cis-3-(3-formimidoylaminopropylthio)-6-(1-hydroxy-1-methylethyl)-7-oxo-1-azabicyclo[3.2.0]hept-2-ene-2-carboxylic acid -

To a solution of 30 mg of 5,6-cis-3-(3-aminopropylthio)-6-(1-hydroxy-1-methylethyl)-7-oxo-1-azabicyclo[3.2.0]hept-2-ene-2-carboxylic acid in 7.5 ml of a pH 7.0 phosphate buffer is added 2N-NaOH under ice-cooling to adjust the pH to be 8.5. To this solution is added 134 mg of benzylformimidate hydrochloride little by little in the course of 2 to 3 minutes, while maintaining the pH of the reaction mixture at 8.5 with 2N-NaOH. After stirring for six minutes, pH of the reaction mixture is lowered to 7.0 with 1N-HCl, followed by washing with ethyl acetate. The aqueous layer is concentrated and subjected to column-chromatography ($H_2O$, 3% acetone) using Diaion HP-20 (manufactured by Mitsubishi Chemical Industries Ltd.). The fractions eluted with 3% acetone are combined and lyophilized to yield the titled compound as colorless powder (25 mg).

IR $\nu_{max}^{KBr}cm^{-1}$: 1750, 1715

UV $\lambda_{max}^{H_2O}nm(E_{1cm}^{1\%})$: 298(261)

NMR (90MHz, $D_2O$)$\delta$: 1.41, 1.58(each 3H, s x 2, $-CH_3$ x 2), 1.9-2.3(2H, m, $-SCH_2C\underline{H}_2CH_2-$), 2.9-3.4 (5H, m, $-SC\underline{H}_2CH_2C\underline{H}_2-N<$, $>C<^{\underline{H}}_{H}$), 3.85(1H, d, J=6Hz, $>CH-CO-$), 3.5-4.1(1H, m, $>C<^{H}_{\underline{H}}$), 4.4(1H, m, $>CH-N<$), 7.95(1H, s, $>N-CH=N-$)

Example 48

- 4-Nitrobenzyl 5,6-cis-3-(2-pyrrolidinoethylthio)-6-(1-hydroxy-1-methylethyl)-7-oxo-1-azabicyclo[3.2.0]hept-2-ene-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.14 mℓ (0.63 mmol) of diphenylphosphoryl chloride, followed by stirring for 90 minutes. After 0.14 mℓ (0.8 mmol) of diisopropylethylamine and 84 mg (0.64 mmol) of pyrrolidineethanethiol are added to the mixture at 0°C and stirring is continued for 90 minutes, 0.07 mℓ (0.4 mmol) of diisopropylethylamine and 42 mg (0.32 mmol) of pyrrolidineethanethiol are further added, followed by stirring at 0°C for 18 hours. The reaction solution is diluted with ethyl acetate, washed with water, dried ($Na_2SO_4$) and the solvent is distilled off. The residue is chromatographed on a column of Florisil (ethyl acetate - acetone = 3:2), thus affording the subject compound as colorless crystals (80 mg).

m.p. 172-174°C.

IR $\nu_{max}^{KBr}$ $cm^{-1}$: 1760

UV $\lambda_{max}^{EtOH}$ nm ($E_{1cm}^{1\%}$): 264 (243), 318 (272).

NMR (90 MHz, $CDC\ell_3$) δ: 1.29, 1.52 (each, 3H,s x 2, $-CH_3x2$), 1.70 to 3.20 (14H,m,-OH,$-SCH_2CH_2-N<$,$-(CH_2)_4-$,$>C<^H_H$), 3.59 (1H,d, J=6Hz,>CH-CO), 4.05 to 4.32 (2H,m,>CH-N<,$>C<^H_H$), 5.37 (2H,q, J=24,15Hz,$-OCH_2Ar$), 7.69 (2H,d,J=8Hz,arom.H), 8.22 (2H,d, J=8Hz,arom.H).

Elemental analysis, for $C_{23}H_{29}N_3O_6S$

Calcd.: C, 58.09; H, 6.14; N, 8.83

Found : C, 58.04; H, 6.11; N, 8.40

Example 49

- 5,6-cis-3-(2-Pyrrolidinoethylthio)-6-(2-hydroxy-1-methylethyl)-7-oxo-1-azabicyclo[3.2.0]hept-2-ene-2-carboxylic acid -

To a mixed solution of 85 mg (0.18 mmol) of 4-nitrobenzyl ester of the subject carboxylic acid in 14 mℓ of tetrahydrofuran, 2 mℓ of methanol, 7 mℓ of a phosphate buffer with a pH of 7.0 and 7 mℓ of water is added 85 mg of 10% Pd-C, and hydrogenation is conducted at room temperature under atmospheric pressure for 2 hours. After the catalyst is filtered out and washed with a small amount of water, the filtrate and washings are combined and washed with ethyl acetate. The water layer is concentrated under reduced pressure, and the concentrate is chromatographed on a column of Diaion HP-20 (produced by Mitsubishi Chemical Ind.Ltd.,)($H_2O$-15% EtOH). The fractions eluted with 15% EtOH are collected and lyophilized, thus giving the subject compound as colorless powder (36 mg).

IR $\nu_{max}^{KBr}$ $cm^{-1}$ : 1750

UV $\lambda_{max}^{H_2O}$ nm ($E_{1\,cm}^{1\%}$) : 292 (197)

NMR (90 MHz, $D_2O$)δ: 1.49, 1.62 (each, 3H,s x 2, $-CH_3$x2), 2.13 to 2.34 (4H,m,$-CH_2(C\underline{H}_2)_2CH_2-$), 2.93 to 3.62 (9H,m,$>C<^{H}_{H}$,$-SCH_2CH_2N<$,$>N<^{CH_2-}_{CH_2-}$), 3.90 (1H,d,J=6Hz,>CH-CO-), 3.87 to 4.15 (1H, m,$>C<^{H}_{H}$), 4.51 (1H,m,>CH-N<).

Example 50

- 4-Nitrobenzyl 5,6-cis-3-(2-piperidinoethylthio)-6-(1-hydroxy-1-methylethyl)-7-oxo-1-azabicyclo[3.2.0]hept-2-ene-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.14 mℓ (0.63 mmol) of diphenylphosphoryl chloride, followed by stirring for 90 minutes. To the mixture are added 0.21 mℓ (1.2 mmol)

of diisopropylethylamine and 140 mg (0.96 mmol) of 2-mercapto-ethylpiperidine, and stirring is continued at 0°C for 18 hours. The reaction solution is diluted with ethyl acetate, washed with water, dried ($Na_2SO_4$) and the solvent is distilled off. The residue is chromatographed on a column of Florisil (acetone-ethyl acetate = 1:3), thus affording the subject compound as colorless crystals (88 mg).

m.p. 147-148°C

IR $\nu_{max}^{KBr}$ $cm^{-1}$: 1770

UV $\lambda_{max}^{EtOH}$ nm ($E_{1\,cm}^{1\%}$): 265 (202), 319 (229).

NMR (60 MHz, $CDCl_3$) δ: 1.23 (3H,s,$-CH_3$), 1.42 to 3.21 (19H,m, $-CH_3$,-OH,$-SCH_2CH_2N<$,$-(CH_2)_5-$,$>C<^H_H$), 3.56 (1H,d,J=6Hz,>CH-CO-), 3.84 to 4.27 (2H,m,$>C<^H_{\underline{H}}$,>CH-N<), 5.28 (2H,q,J=24,15Hz,$-OCH_2Ar$), 7.57 (2H,d,J=8Hz,arom.H), 8.20 (2H,d,J=8Hz,arom.H).

Elemental analysis, for $C_{24}H_{31}N_3O_6S\cdot 1/2H_2O$)

Calcd.: C, 57.81; H, 6.46; N, 8.42

Found : C, 57.98; H, 6.47; N, 8.35

## Example 51

- 5,6-cis-3-(2-Piperidinoethylthio)-6-(1-hydroxy-1-methylethyl)-7-oxo-1-azabicyclo[3.2.0]hept-2-ene-carboxylic acid -

To a mixed solution of 125 mg (0.26 mmol) of 4-nitrobenzyl ester of the subject carboxylic acid in 20 mℓ of tetrahydrofuran, 10 mℓ of a phosphate buffer with a pH of 7.0 and 10 mℓ of water is added 125 mg of 10% Pd-C, and hydrogenation is conducted at room temperature under atmospheric pressure for 3 hours. After the catalyst is filtered out and washed with a small amount of water, the filtrate and washings are combined, and washed with ethyl acetate. The water layer is concentrated under reduced pressure, and the concentrate is chromatographed on a column of Amberlite XAD-2 ($H_2O$, 5% EtOH). The fractions eluted with 5% EtOH are collected and lyophilized thus giving the subject carboxylic acid as colorless powder (68 mg).

IR $\nu_{max}^{KBr}$ $cm^{-1}$: 1750

UV $\lambda_{max}^{H_2O}$ nm ($E_{1\,cm}^{1\%}$) : 295 (192)

NMR (90 MHz, $D_2O$) δ: 1.55, 1.68 (each, 3H, s x 2, $-CH_3x2$), 1.79 to 2.16 (6H, m, $-CH_2(C\underline{H}_2)_3CH_2-$), 3.01 to 3.52 (9H, m, $>C<^{H}_{\underline{H}}$, $-SCH_2CH_2N<$, $-CH_2-N<^{CH_2-}$), 3.92 (1H, d, J=6Hz, >CH-CO-), 3.82 to 4.18 (1H, m, $>C<^{\underline{H}}_{H}$), 4.58 (1H, m, -CH-N<).

Example 52

- 4-Nitrobenzyl 5,6-cis-3-[2-(4-methylpiperazino)ethylthio]-6-(1-hydroxy-1-methylethyl)-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.13 mℓ (0.63 mmol) of diphenylphosphoryl chloride, followed by stirring for 90 minutes. To this mixture are added 0.21 mℓ (1.2 mmol) of diisopropylethylamine and 154 mg (0.93 mmol) of 4-methyl-piperazinoethanethiol, and stirring is continued at 0°C for 20 hours. The reaction solution is diluted with ethyl acetate, washed with water, dried ($Na_2SO_4$) and the solvent is distilled off. The residue is chromatographed on a column of Florisil (ethyl acetate - methanol = 3:1), thus giving the subject compound as light yellow crystals (120 mg).

m.p. 169-171°C

IR $\nu_{max}^{KBr}$ $cm^{-1}$: 1760

UV $\lambda_{max}^{EtOH}$ nm ($E_{1\,cm}^{1\%}$): 265 (224), 318 (258)

NMR (90 MHz, $CDC\ell_3$) : 1.31, 1.53 (each, 3H, s x 2, $-CH_3x2$), 1.92 (1H, s, -OH), 2.03 to 3.18 (13H, m, $-SCH_2CH_2$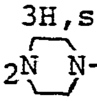-, $>C<^{\underline{H}}_{H}$), 2.29 (3H, s, $>N-CH_3$), 3.59 (1H, d, J=6Hz, >CH-CO-), 4.04 to 4.43 (2H, m, $>C<^{H}_{\underline{H}}$, >CH-N<), 5.37 (2H, q, J=24, 15Hz, $-OCH_2Ar$), 7.65 (2H, d, J=8Hz, arom.H), 8.21 (2H, d, J=8Hz, arom.H).

Elemental analysis, for $C_{24}H_{32}N_4O_6S \cdot 1/2H_2O$

Calcd.: C, 56.12; H, 6.47; N, 10.91

Found : C, 55.80; H, 6.16; N, 10.63

## Example 53

- 5,6-cis-3-[2-(4-Methylpiperazino)ethylthio]-6-(1-hydroxy-1-methylethyl)-7-oxo-1-azabicyclo[3.2.0]hept-2-ene-2-carbboxylic acid -

To a mixed solution of 100 mg (0.2 mmol) of 4-nitrobenzyl ester of the subject compound in 20 mℓ of tetrahydrofuran, 10 mℓ of a phosphate buffer with a pH of 7.0 and 10 mℓ of water is added 100 mg of 10% Pd-C, and hydrogenation is conducted at room temperature under atmospheric pressure for 2 hours. After the catalyst is filtered out and washed with a small amount of water, the filtrate and washings are combined and washed with ethyl acetate. The water layer is concentrated under reduced pressure, and the concentrate is chromatographed on a column of Diaion HP-20 ($H_2O$ - 20% EtOH). The fractions eluted with 20% EtOH are collected and lyophilized thus affording the subject compound as colorless powder (43 mg).

IR $\nu_{max}^{KBr}$ $cm^{-1}$: 1755

UV $\lambda_{max}^{H_2O}$ nm ($E_{1\,cm}^{1\%}$): 299 (222)

NMR (90 MHz, $D_2O$) $\delta$: 1.49, 1.66 (each, 3H, s x 2, $-CH_3$x2), 2.89 to 3.58 (16H, m, $-SCH_2CH_2N\langle\;\rangle NCH_3$, $>C<^{H}_{H}$), 3.84 to 4.21 (1H, m, $>C<^{H}_{H}$), 3.91 (1H, d, J=6Hz, >CH-CO-), 4.49 (1H, m, $>C<^{H}_{H}$)

## Example 54

- 4-Nitrobenzyl 5,6-cis-3-(2-ethoxyethylthio)-6-(1-hydroxy-1-methylethyl)-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-azetidin-2-one (benzene solvation product) in accordance with the procedure of Reference Example 21 are added under nitrogen atmosphere at 0°C 0.11 mℓ (0.63 mmol) of diisopropylethylamine and then 0.14 mℓ (0.63 mmol) of diphenylphosphoryl chloride, followed by stirring for 90 minutes. To this mixture are added 0.21 mℓ (1.2 mmol) of diisopropylethylamine and 102 mg (0.96 mmol) of 2-ethoxyethylmercaptan, and stirring is continued

at 0°C for 20 hours. The reaction solution is diluted with ethyl acetate, washed with water, dried ($Na_2SO_4$) and the solvent is distilled off. The residue is chromatographed on a column of Florisil (ethyl acetate - hexane = 1:1), thus giving the subject compound as colorless crystals (98 mg).

m.p. 150-151°C

IR $\nu_{max}^{KBr}$ $cm^{-1}$: 1760

UV $\lambda_{max}^{EtOH}$ nm ($E_{1\,cm}^{1\%}$): 263 (250), 315 (279)

NMR (60 MHz, $CDCl_3$) δ: 1.12 (3H,t,J=7Hz,$-OCH_2C\underline{H}_3$), 1.28, 1.52 (each, 3H,s x 2, $-CH_3$x2), 1.68 (1H,s,-OH), 2.82 to 3.61 (8H,m, $-C\underline{H}_2C\underline{H}_2OC\underline{H}_2CH_3$, $>C<^{H}_{H}$, >CHCO-), 3.80 to 4.23 (2H,m,$>C<^{H}_{H}$,>CH-N<), 5.20 (2H,q,J=24,15Hz,$-OCH_2Ar$), 7.44 (2H,d,J=8Hz,arom.H), 8.02 (2H,d,J=8Hz,arom.H).

Elemental analysis, for $C_{21}H_{26}N_2O_7S$

Calcd.: C, 55.98; H, 5.81; N, 6.22

Found : C, 56.35; H, 6.80; N, 6.42

Example 55

- Sodium 5,6-cis-3-(2-ethoxyethylthio)-6-(1-hydroxy-1-methylethyl)-7-oxo-1-azabicyclo[3.2.0]hept-2-ene-2-carboxylate -

To a mixed solution of 90 mg (0.19 mmol) of 4-nitrobenzyl ester of the subject carboxylic acid in 18 mℓ of tetrahydrofuran, 9 mℓ of a phosphate buffer with a pH of 7.0 and 9 mℓ of water is added 90 mg of 10% Pd-C, and hydrogenation is conducted at room temperature under atmospheric pressure for 2 hours. After the catalyst is filtered out and washed with a small amount of water, the filtrate and washings are combined and washed with ethyl acetate. The water layer is concentrated under reduced pressure, and the concentrate is chromatographed on a column of Diaion HP-20 ($H_2O$ - 10% EtOH). The fractions eluted with 10% EtOH are collected and lyophilized, thus giving the subject compound as colorless powder (20 mg).

IR $\nu_{max}^{KBr}$ $cm^{-1}$: 1755

UV $\lambda_{max}^{H_2O}$ nm ($E_{1\,cm}^{1\%}$): 297 (269)

NMR (100 MHz, $D_2O$) δ: 1.30 (3H,t,J=7Hz,$-OCH_2CH_3$), 1.42, 1.56 (each, 3H,s x 2, $-CH_3$x2), 3.17 to 3.46 (3H,m,$>C<^{H}_{H}$,$-SC\underline{H}_2CH_2-$), 3.73

to 4.26 (5H,m,>CHCO-,$-SCH_2C\underline{H}_2OC\underline{H}_2CH_3$), 4.48 to 4.72 (2H,m, >CHN<,$>C<^{H}_{\underline{H}}$)

Example 56

- 4-Nitrobenzyl 5,6-cis-3-[2-[(4-methylpiperadinyl)-1-carbonyloxy]ethylthio]-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.13 mℓ (0.63 mmol) of diphenylphosphoryl chloride, followed by stirring for 90 minutes. To this mixture are added 0.21 mℓ (1.2 mmol) of diisopropylethylamine and 196 mg (0.96 mmol) of 2-[(4-methylpiperazinyl)-1-carbonyloxy]ethylmercaptan, and stirring is continued for 20 hours. The reaction solution is diluted with chloroform and poured in an aqueous potassium carbonate solution under ice-cooling. The organic layer is separated, washed with water, dried ($Na_2SO_4$) and the solvent is distilled off. The residue is chromatographed on a column of Florisil (ethyl acetate-methanol = 5:1), thus giving the subject compound as light yellow crystals (136 mg).

m.p. 183-185°C.

IR $\nu_{max}^{KBr}$ $cm^{-1}$: 1750

UV $\lambda_{max}^{EtOH}$ nm ($E_{1\,cm}^{1\%}$): 264 (386), 318 (181)

NMR (60 MHz, $CDC\ell_3$)$\delta$: 1.22, 1.47 (each, 3H,s x 2, $-CH_3$x2), 2.0 to 2.48 (9H,m,-OH,$>C<^{H}_{\underline{H}}$,$>N<^{CH_2-}_{CH_2-}$,$>N-CH_3$), 2.88 to 3.6 (7H,m, >CH-CO-,$-SCH_2C\underline{H}_2O-$,$N<^{CH_2-}_{CH_2-}$), 4.0 to 4.3 (4H,m,$>C<^{H}_{\underline{H}}$,$-SC\underline{H}_2CH_2-$), 5.30 (2H,q,J=24,15Hz,$-OCH_2Ar$), 7.51 (2H,d,J=8Hz,arom.H), 8.08 (2H,d,J=8Hz,arom.H).

Elemental analysis, for $C_{25}H_{32}N_4O_8S\cdot1/2H_2O$

Calcd.: C, 53.84; H, 5.97; N, 10.04

Found : C, 53.55; H, 5.81; N, 9.98

## Example 57

- 5,6-cis-3-[2-[(4-Methylpiperadinyl)-1-carbonyloxy]ethylthio]-7-oxo-1-azabicyclo[3.2.0]hept-2-ene-2-carboxylic acid -

To a mixed solution of 120 mg (0.22 mmol) of 4-nitrobenzyl ester of the subject compound in 20 mℓ of tetrahydrofuran, 10 mℓ of a phosphate buffer with a pH of 7.0 and 10 mℓ of water is added 120 mg of 10% Pd-C, and hydrogenation is conducted at room temperature under atmospheric pressure for 4 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$, 5% EtOH). The fractions eluted with 5% EtOH are collected and lyophilized, thus giving the subject compound as colorless powder (33 mg).

IR $\nu_{max}^{KBr}$ $cm^{-1}$: 1750, 1700

UV $\lambda_{max}^{H_2O}$ nm ($E_{1\,cm}^{1\%}$): 298 (188)

NMR (90 MHz, $D_2O$) δ: 1.50, 1.63 (each, 3H, s x 2, $-CH_3$x2), 2.58 (3H, s, $>NCH_3$), 2.74 to 2.88 (4H, m, $>N<^{CH_2-}_{CH_2-}$), 3.10 to 4.0 (8H, m, >CH-CO-, $-SCH_2\underline{CH_2}O-$, $-N<^{CH_2-}_{CH_2-}$, $>C<^{\underline{H}}_{H}$), 4.06 to 4.65 (4H, m, >CH-N<, $>C<^{H}_{\underline{H}}$, $-S\underline{CH_2}CH_2-$)

## Example 58

- 4-Nitrobenzyl 5,6-cis-3-[2-(N-methylcarbamoyl)aminoethylthio]-6-(1-hydroxy-1-methylethyl)-7-oxo-1-azabicyclo[3.2.0]heptene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.13 mℓ (0.63 mmol) of diphenylphosphoryl chloride, followed by stirring for 90 minutes. To this mixture are added 0.21 mℓ (1.2 mmol) of

diisopropylethylamine and 129 mg (0.96 mmol) of N-(2-mercaptoethyl)-N'-methyl urea, and stirring is continued at 0°C for 18 hours. The reaction solution is diluted with ethyl acetate, washed with water, dried ($Na_2SO_4$) and the solvent is distilled off. The residue is treated with ethyl acetate, thus affording the subject compound as light yellow crystals (90 mg). The mother liquor is chromatographed on a column of Florisil (ethyl acetate), thus giving additional crystals (19 mg).

m.p. 195-196°C.

IR $\nu_{max}^{KBr}$ $cm^{-1}$: 1750

UV $\lambda_{max}^{EtOH}$ nm ($E_{1\,cm}^{1\%}$): 264 (229), 315 (251)

NMR (90 MHz, DMSO-$d_6$) δ: 1.17, 1.31 (each, 3H,s x 3, $-CH_3$x2), 2.51 (3H,d,$-NHC\underline{H}_3$), 2.88 to 3.67 (6H,m,$-SCH_2CH_2N<$,$>CH-CO<$,$>C<\frac{H}{\underline{H}}$), 3.92-4.32 (2H,m,$>C<\frac{H}{\underline{H}}$,$>CH-N<$), 4.68 (1H,bs,-OH), 5.32 (2H,q, J=24,15Hz,$-OCH_2Ar$), 5.68 to 6.30 (2H,m,$-N\underline{H}CON\underline{H}-$), 7.72 (2H,d, J=8Hz,arom.H), 8.23 (2H,d,J=8Hz,arom.H).

Elemental analysis, for $C_{21}H_{26}N_4O_7S\cdot1/2H_2O$

Calcd.: C, 51.74; H, 5.58; N, 11.49

Found : C, 51.45; H, 5.49; N, 11.10

Example 59

- Sodium 5,6-cis-3-[2-(N-methylcarbamoyl)aminoethylthio]-6-(1-hydroxy-1-methylethyl)-7-oxo-1-azabicyclo[3.2.0]hept-2-ene-2-carboxylate -

To a mixed solution of 85 mg (0.177 mmol) of 4-nitrobenzyl ester of the subject carboxylic acid in 18 mℓ of tetrahydrofuran, 9 mℓ of a phosphate buffer with a pH of 7.0 and 9 mℓ of water is added 85 mg of 10% Pd-C, and hydrogenation is conducted at room temperature under atmospheric pressure for 4 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$), thus giving the subject compound as colorless powder (38 mg).

IR $_{max}^{KBr}$ $cm^{-1}$: 1750

UV $_{max}^{H_2O}$ nm ($E_{1\,cm}^{1\%}$): 296 (215)

NMR (90 MHz, $D_2O$)δ: 1.53, 1.66 (each, 3H,s x 2, $-CH_3x2$), 2.91 (3H,s-$NHC\underline{H}_3$), 3.04 to 3.92 (5H,m,$-SCH_2CH_2N<$,$>C<^{\underline{H}}_{H}$), 3.91 (1H,d,J=6Hz,$>C<^{H}_{\underline{H}}$), 4.06 to 4.67 (2H,m,$>C<^{H}_{\underline{H}}$,>CH-N<).

## Example 60

- 4-Nitrobenzyl 5,6-cis-3-[2-(N-methylthiocarbamoyl)aminoethylthio]-6-(1-hydroxy-1-methylethyl)-7-oxo-1-azabicyclo-[3.2.0]hept-2-ene-2-carboxylate -

The reaction and treatment are conducted in the same manner as in Example 58, except that N-(2-mercaptoethyl)-N'-methylthiourea is used in place of N-(2-mercaptoethyl)-N'-methylurea, to give the subject compound as light yellow crystals.

m.p. 174-176°C

IR $\nu^{KBr}_{max}$ $cm^{-1}$: 1750

UV $\lambda^{EtOH}_{max}$ nm ($E^{1\%}_{1\,cm}$): 243 (382), 315 (224)

NMR (90 MHz, DMSO-$d_6$)δ: 1.19, 1.32 (each, 3H,s x 2, $-CH_3x2$), 2.77 (3H,d,$-NHC\underline{H}_3$), 2.91 to 3.69 (6H,m,$-SCH_2CH_2N<$,>CH-CO-, $>C<^{H}_{\underline{H}}$), 3.97 to 4.23 (2H,m,$>C<^{H}_{\underline{H}}$,>CH-N<), 4.66 (1H,s,-OH), 5.32 (2H,q,J=24,15Hz,$-OCH_2Ar$), 7.10 to 7.62 (2H,m,$-N\underline{H}CSN\underline{H}-$), 7.72 (2H,d,J=8Hz,arom.H), 8.22 (2H,d,J=8Hz,arom.H).

Elemental analysis, for $C_{21}H_{26}N_4O_6S_2 \cdot H_2O$

Calcd.: C, 49.20; H, 5.51; N, 10.93

Found : C, 48.85; H, 5.36; N, 10.99

## Example 61

- Sodium 5,6-cis-3-[2-(N-methylthiocarbamoyl)aminoethylthio]-6-(1-hydroxy-1-methylethyl)-7-oxo-1-azabicyclo[3.2.0]hept-2-ene-2-carboxylate -

The reaction and treatment are codnucted with 4-nitrobenzyl ester of the subject carboxylic acid in the same manner as in Example 59, to give the subject compound as colorless powder.

IR $\nu^{KBr}_{max}$ $cm^{-1}$: 1750

UV $\lambda^{H_2O}_{max}$ nm ($E^{1\%}_{1\,cm}$): 233 (325), 297 (222).

NMR (90 MHz, $D_2O$)δ: 1.49, 1.62 (each, 3H,s x 2, $-CH_3x2$), 3.04 to 3.95 (5H,m,$-SCH_2CH_2N<$,$>C<^{\underline{H}}_{H}$), 3.10 (3H,s,$NHC\underline{H}_3$), 3.91 (1H,

d,J=6Hz,>CH-CO-), 4.08 to 4.67 (2H,m,>CH-N<,>C<$^{H}_{\underline{H}}$)

Example 62

- 4-Nitrobenzyl 5,6-cis-3-[2-(4-nitrobenzyloxycarbonylamino)-4-thiazolylmethylthio]-6-(1-hydroxy-1-methylethyl)-7-oxo-1-azabicyclo[3.2.0]hept-2-ene-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin solvation product) in accordance with the procedure of Reference Example 21 are added under nitrogen atmosphere at 0°C 0.11 mℓ (0.63 mmol) of diisopropylethylamine and then 0.13 m (0.63 mmol) of diphenylphosphoryl chloride, followed by stirring for 90 minutes. To this mixture are added 305 mg (0.71 mmol) of silver salt of 2-(4-nitrobenzyloxycarbonylamino)-4-mercapto-methylthiazole and 750 mg (5 mmol) of sodium iodide, and stirring is continued at 0°C for 23 hours. The reaction solution is diluted with chloroform and poured in an aqueous sodium chloride solution under cooling, and the insoluble material is filtered out with use of Celite. The organic layer of the filtrate is separated, washed with water, dried ($Na_2SO_4$) and then the solvent is distilled off. The residue is chromatographed on a column of Florisil (acetone-hexane = 3:5), thus giving the subject compound as colorless crystals (62 mg).

m.p. 124-126°C.

IR $\nu_{max}^{KBr}$ $cm^{-1}$: 1770

UV $\lambda_{max}^{EtOH}$ nm ($E_{1\,cm}^{1\%}$): 264 (395), 318 (199)

NMR (90 MHz, $CDC\ell_3$)δ: 1.26, 1.52 (each, 3H,s x 2, $-CH_3$x2), 2.05 (1H,s,-OH), 3.02 (1H,q,J=17,9Hz,>C<$^{H}_{\underline{H}}$), 3.61 (1H,d,J=6Hz, >CH-CO-), 4.0 (2H,s,$-SCH_2$-C≤), 4.09 to 4.75 (2H,m,>C<$^{H}_{\underline{H}}$,>CH-N<), 5.30 (2H,s,$-NHCOOC\underline{H}_2Ar$), 5.34 (2H,q,J=24,15Hz,$-OCH_2Ar$), 6.72 (1H,s,thiazole H), 7.56 to 8.19 (8H,m,arom.H).

Elemental analysis, for $C_{29}H_{27}N_5O_{10}S_2 \cdot H_2O$

Calcd.: C, 50.64; H, 4.25; N, 10.18

Found : C, 50.58; H, 4.17; N, 10.00

Example 63

- Sodium 5,6-cis-3-(2-amino-4-thiazolylmethylthio)-6-(1-hydroxy-1-methylethyl)-7-oxo-1-azabicyclo[3.2.0]hept-2-ene-carboxylate -

To a mixed solution of 115 mg (0.171 mmol) of 4-nitrobenzyl 5,6-cis-3-[2-(4-nitrobenzyloxycarbonylamino)-4-thiazolylmethylthio]-6-(1-hydroxy-1-methylethyl)-7-oxo-1-azabicyclo-[3.2.0]hept-2-ene-2-carboxylate in 20 mℓ of tetrahydrofuran, 10 mℓ of a phosphate buffer with a pH of 7.0 and 10 mℓ of water is added 200 mg of 10% Pd-C, and hydrogenation is conducted at room temperature under atmospheric pressure for 4 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$), thus giving the subject compound as colorless powder (25 mg).

IR $\nu_{max}^{KBr}$ $cm^{-1}$: 1740

UV $\lambda_{max}^{H_2O}$ nm ($E_{1\,cm}^{1\%}$): 260 (204), 300 (249)

NMR (90 MHz, $D_2O$)δ: 1.46, 1.59 (each, 3H, s x 3, $-CH_3$x2), 3.27 (1H,q,J=17,10Hz,$>C<\frac{H}{H}$), 4.87 (1H,d,J=6Hz,>CH-CO-), 4.93 (1H, m,$>C<\frac{H}{\underline{H}}$), 4.12 (2H,s,$-SCH_2C<$), 4.47 (1H,m,>CH-N<), 6.78 (1H,s, thiazole H).

Example 64

- 4-Nitrobenzyl 5,6,-cis-3-(2-acetylaminothiazolylmethylthio)-6-(1-hydroxy-1-methylethyl)-7-oxo-1-azabicyclo[3.2.0]hept-2-ene-2-carboxylate -

The reaction and treatment are conducted in the same manner as in Example 62, except that silver salt of 2-acetylamino-4-mercaptomethylthiazole is used in place of silver salt of 2-(4-nitrobenzyloxycarbonylamino)-4-mercaptomethylthiazole, to give the subject compound as light yellow crystals.

m.p. 131-133°C.

IR $\nu_{max}^{KBr}$ $cm^{-1}$: 1770

UV $\lambda_{max}^{EtOH}$ nm ($E_{1\,cm}^{1\%}$): 267 (300), 318 (195).

NMR (90 MHz, $CDC\ell_3$)δ: 1.30, 1.51 (each, 3H,s x 2, $-CH_3$x2), 2.21

(3H,s,-NHCOC$\underline{H}_3$), 2.91 (1H,bs,-OH), 3.10 (1H,q,J=17,9Hz,>C<$^{\underline{H}}_{H}$), 4.0 (2H,s,-SCH$_2$C<), 4.14 to 4.75 (2H,m,>CH-N<,>C<$^{H}_{\underline{H}}$), 5.32 (2H,q,J=24,15Hz,-OCH$_2$Ar), 6.71 (1H,s,thiazole H), 7.60 (2H, d,J=8Hz,arom.H), 8.19 (2H,d,J=8Hz,arom.H), 9.89 (1H,bs,-N$\underline{H}$COCH$_2$).

Elemental analysis, for $C_{23}H_{24}N_4O_7S_2 \cdot 2H_2O$

Calcd.: C, 48.58; H, 4.96; N, 9.85

Found : C, 48.68; H, 4.74; N, 9.79

## Example 65

- Sodium 5,6-cis-3-(2-acetylaminothiazolylmethylthio)-6-(1-hydroxy-1-methylethyl)-7-oxo-1-azabicyclo[3.2.0]hept-2-ene-2-carboxylate -

The reaction and treatment are conducted with 4-nitrobenzyl ester of the subject carboxylic acid in the same manner as in Example 63, to give the subject compound as colorless powder.

IR $\nu_{max}^{KBr}$ cm$^{-1}$: 1750

UV $\lambda_{max}^{H_2O}$ nm ($E_{1\,cm}^{1\%}$): 280 (210)

NMR (90 MHz, $D_2O$)δ: 1.42, 1.58 (each, 3H,s x 2, -CH$_3$x2), 2.49 (3H,s,-NHCOC$\underline{H}_3$), 3.22 (1H,q,J=17,9Hz,>C<$^{\underline{H}}_{H}$), 3.80 (1H,d,J=6Hz, >CH-CO-), 3.88 (2H,s,-SCH$_2$C<), 3.96 to 4.60 (2H,m,>C<$^{H}_{\underline{H}}$,>CH-N<), 7.21 (1H,s,thiazole H).

## Example 66

- 4-Nitrobenzyl 5,6-cis-3-[2-(4-nitrobenzyloxycarbonyl)-2-(4-nitrobenzyloxycarbonylamino)ethylthio]-6-(1-hydroxy-1-methylethyl)-7-oxo-1-azabicyclo[3.2.0]hept-2-ene-2-carboxylate -

To a solution in 40 mℓ of anhydrous acetonitrile of 4-nitrobenzyl 5,6-cis-6-(1-hydroxy-1-methylethyl)-1-azabicyclo-[3.2.0]heptane-3,7-dione-2-carboxylate as prepared from 464 mg (1.0 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.22 mℓ (1.26 mmol) of diisopropylethylamine and then 0.26 mℓ (1.26 mmol) of diphenylphosphoryl chloride, followed by stirring

for 90 minutes. To this mixture are added 813 mg (1.5 mmol) of silver salt of L-2-(4-nitrobenzyloxycarbonyl)-2-(4-nitrobenzyloxycarbonylamino)ethanethiol and 1.5 g (10 mmol) of sodium iodide, and stirring is continued at 0°C for 21 hours. The reaction solution is diluted with ethyl acetate and poured in an aqueous sodium chloride solution under cooling, and the insoluble material is filtered out with Celite. The organic layer of the filtrate is separated, washed with water, dried ($Na_2SO_4$) and freed of the solvent. The residue is chromatographed on a column of Florisil (ethyl acetate - hexane = 1:1), thus giving the subject compound (the isomer mixture) as light yellow powder (210 mg).

IR $\nu_{max}^{KBr}$ $cm^{-1}$: 1770

UV $\lambda_{max}^{EtOH}$ nm ($E_{1\,cm}^{1\%}$): 265 (382), 313 (155)

NMR (90 MHz, $CDCl_3$)δ: 1.29, 1.53 (each, 3H,s x 2, $-CH_3$x2), 2.72 to 3.60 (3H,m,$-SCH_2-$,$>C<^{H}_{H}$), 3.49 (1H,d,J=6Hz,>CH-CO-), 4.0 to 4.8 (3H,m,>CH-NH-,$>C<^{H}_{H}$,>CH-N<), 5.08 to 5.81 (6H,m,$-OCH_2$Arx3), 7.25 to 8.28 (12H,m,arom.H).

## Example 67

- Disodium 5,6-cis-3-(2-amino-2-carboxyethylthio)-6-(1-hydroxy-1-methylethyl)-7-oxo-1-azabicyclo[3.2.0]hept-2-ene-2-carboxylate -

To a mixed solution of 380 mg (0.49 mmol) of 4-nitrobenzyl 5,6-cis-3-[2-(4-nitrobenzyloxycarbonyl)-2-(4-nitrobenzyloxycarbonylamino)ethylthio]-6-(1-hydroxy-1-methylethyl)-7-oxo-1-azabicyclo[3.2.0]hept-2-ene-2-carboxylate as obtained in Example 66 in 60 mℓ of tetrahydrofuran, 40 mℓ of a phosphate buffer with a pH of 7.0 and 20 mℓ of water is added 570 mg of 10% Pd-C, and hydrogenation is conducted for 4 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2 ($H_2O$) and then on a column of Diaion HP-20. The fractions eluted with water are collected and lyophilized, thus giving two forms of the isomers of the

subject compound as colorless powder.

Isomer (I): 22 mg

IR $\nu_{max}^{KBr}$ cm$^{-1}$: 1750

UV $\lambda_{max}^{H_2O}$ nm ($E_{1\,cm}^{1\%}$): 294 (214)

NMR (100 MHz, $D_2O$) δ: 1.37, 1.47 (each, 3H, s x 2, $-CH_3$x2), 2.95 to 3.66 (3H, m, >C<$\frac{H}{H}$, $-SCH_2-$), 3.77 (1H, d, J=6Hz, >CH-CO-), 3.72 to 4.00 (2H, m, >C<$\frac{H}{\underline{H}}$, <C$\underline{H}$-$NH_2$), 4.41 (1H, m, >CH-N<).

Isomer (II): 19 mg

IR $\nu_{max}^{KBr}$ cm$^{-1}$: 1750

UV $\lambda_{max}^{H_2O}$ nm ($E_{1\,cm}^{1\%}$): 296 (179)

NMR (100 MHz, $D_2O$) δ: 1.37, 1.46 (each, 3H, s x 2, $-CH_3$x2), 2.93 to 3.67 (3H, m, >C<$\frac{H}{H}$, $-SCH_2-$), 3.76 (1H, d, J=6Hz, >CH-CO-), 3.72 to 4.04 (2H, m >C<$\frac{H}{\underline{H}}$, >C$\underline{H}$-$NH_2$), 4.40 (1H, m, >CH-N<).

Example 68

- 4-Nitrobenzyl 5,6-cis-3-[2-methoxycarbonyl-2-(4-nitrobenzyloxycarbonylamino)ethylthio]-6-(1-hydroxy-1-methylethyl)-7-oxo-1-azabicyclo[3.2.0]hept-2-ene-2-carboxylate -

To a solution in 20 mℓ of anhydrous acetonitrile of 4-nitrobenzyl 5,6-cis-6-(1-hydroxy-1-methylethyl)-1-azabicyclo-[3.2.0]heptane-3,7-dione-carboxylate as prepared from 234 mg (0.5 mmol) of 5,6-cis-4-[3-diazo-3-(4-nitrobenzyloxycarbonyl)-2-oxopropyl]-3-(1-hydroxy-1-methylethyl)azetidin-2-one (benzene solvation product) in accordance with the procedure of Reference Example 21 are added under nitrogen atmosphere at 0°C 0.11 mℓ (0.63 mmol) of diisopropylethylamine and then 0.13 mℓ (0.63 mmol) of diphenylphosphoryl chloride, followed by stirring for 90 minutes. To this mixture are added 0.14 mℓ (0.8 mmol) of diisopropylethylamine and 201 mg (0.64 mmol) of L-2-methoxycarbonyl-2-(4-nitrobenzyloxycarbonylamino)ethanethiol, and stirring is continued at 0°C for 2 hours. The reaction solution is diluted with ethyl acetate, washed with water, dried ($Na_2SO_4$) and the solvent is distilled off. The residue is chromatographed on a column of Florisil (ethyl acetate - hexane = 3:2), thus giving the subject compound as colorless crystals (160 mg). m.p. 170-173°C.

IR $\nu_{max}^{KBr}$ $cm^{-1}$: 1750

UV $\lambda_{max}^{EtOH}$ nm ($E_{1\,cm}^{1\%}$): 266 (328), 315 (197)

NMR (90 MHz, $CDCl_3$)$\delta$: 1.27, 1.51 (each 3H,s. x 2,$-CH_3$x2), 2.90 to 3,43 (3H,m,$-SCH_2-$,>C<H H), 3.60 (1H,d,J=6Hz,>CH-CO-), 3.81 (3H,s,$-OCH_3$), 4.10 to 4.77 (3H,m,>C<u>H</u>-NH-,>C<H <u>H</u>,>CH-N<), 5.24 (2H,s,$-OCH_2Ar$), 5.30 (2H,q,J=24,15Hz,$-OCH_2Ar$), 7.30 to 8.37 (8H,m,arom.H)

Elemental analysis, for $C_{29}H_{30}N_4O_{12}S$

Calcd.: C, 52.89; H, 4.59; N, 8.50

Found : C, 52.50; H, 4.53; N, 8.28

<u>Example 69</u>

- Sodium 5,6-cis-3-(2-amino-2-methoxycarbonylethylthio)-6-(1-hydroxy-1-methylethyl)-7-oxo-1-azabicyclo[3.2.0]hept-2-ene-2-carboxylate -

To a mixed solution of 155 mg (0.235 mmol) of 4-nitrobenzyl 5,6-cis-3-[2-methoxycarbonyl-2-(4-nitrobenzyloxycarbonylamino)ethylthio]-6-(1-hydroxy-1-methylethyl)-7-oxo-1-azabic clo-[3.2.0]hept-2-ene-2-carboxylate as obtained in Example 68 in 25 mℓ of tetrahydrofuran, 13 mℓ of a phosphate buffer with a pH of 7.0 and 13 mℓ of water is added 275 mg of 10% Pd-C, and hydrogenation is conducted at room temperature under atmospheric pressure for 3.5 hours. After the catalyst is filtered out and washed with a small amount of water, the filtrate and washings are combined and washed with ethyl acetate. The water layer is concentrated under reduced pressure, and the concentrate is chromatographed on a column of Amberlite XAD-2. The fractions eluted with water are collected and lyophilized, thus giving the subject compound as colorless powder (18 mg).

IR $\nu_{max}^{KBr}$ $cm^{-1}$: 1735

UV $\lambda_{max}^{H_2O}$ nm ($E_{1\,cm}^{1\%}$): 296 (202)

NMR (90 MHz, $D_2O$)$\delta$: 1.41, 1.54 (each 3H,s x 2,$-CH_3$x2), 3.05 to 3.60 (3H,m,$-SCH_2-$,>C<H H), 3.82 (1H,d,J=6Hz,>CH-CO-), 3.81 to 4.10 (2H,m,>C<H <u>H</u>,>C<u>H</u>-$NH_2$), 3.90 (3H,s,$-OCH_3$), 4.41 (1H,m,>CH-N<)

Example 70

5,6-cis-3-(2-Formimidoylaminoethylthio)-6-(1-hydroxy-1-methylethyl)-7-oxo-1-azabicyclo[3.2.0]hept-2-ene-2-carboxylic acid 500 mg is placed in a 17 ml vial. A solution ready for injection is prepared by further adding in the vial. 1 ml of pyrogen-free distilled water.

Example 71

5,6-cis-3-(2-Aminoethylthio)-6-(1-hydroxy-2-methylethyl)-7-oxo-1-azabicyclo[3.2.0]hept-2-ene-2-carboxylic acid is placed in a sterile 12 ml vial in amount of 250 mg (potency). A solution ready for injection is prepared by further adding 1 ml of pyrogen-free distilled water.

Test Example

Minimal Inhibitory Concentration (MIC)

Method

The Minimal Inhibitory Concentrations of the tested compounds were determined according to the agar dilution method. Namely, 1.0 ml each of aqueous solution of the tested compounds diluted by serial dilutions was poured into test petri dishes, subsequently 9.0 ml each of Trypticase soy agar was poured into the dishes and mixed. On each of the mixed agar plates, one loopful of bacterial suspension (about $10^8$ CFU/ml) of test microorganism was streaked.

After the incubation at 37°C for 18 hours, the lowest concentration of the tested compounds in the medium which caused apparently complete inhibition of growth of the test microorganism was taken to be minimal inhibitory concentration. Antibacterial spectra (minimum inhibitory concentration) of the test compounds are shown in the Table 1.

Test Microorganism

(1) S. aureus FDA 209P
(2) S. aureus 308 A-1
(3) S. aureus 1840
(4) E. coli NIHJ JC-2
(5) E. coli 0-111
(6) E. coli T-7
(7) C. freundii IFO 12681
(8) K. pneumoniae DT
(9) E. cloacae IFO 12937
(10) S. marcescens IFO 12648
(11) P. vulgaris IFO 3988
(12) P. mirabilis IFO 3849
(13) P. morganii IFO 3168
(14) P. aeruginosa IFO 3455
(15) P. aeruginosa U 31
(16) A. calcoaceticus IFO 13006

**Table 1**

| Test Microorganism \ Test compound (Example No.) | 49 | 51 | 67 Isomer (I) | 46 | 2 | 26 | 47 | 33 | 20 |
|---|---|---|---|---|---|---|---|---|---|
| ( 1) | 0.2 | 0.2 | 3.13 | 0.39 | 0.39 | 0.39 | 0.39 | 0.78 | 0.39 |
| ( 2) | 0.39 | 0.39 | 3.13 | 0.78 | 0.78 | 0.39 | 0.39 | 0.78 | 0.39 |
| ( 3) | 0.78 | 0.78 | 6.25 | 0.78 | 1.56 | 0.78 | 0.78 | 0.78 | 0.78 |
| ( 4) | 0.78 | 0.78 | 1.56 | 0.39 | 1.56 | 0.78 | 0.39 | 0.39 | 0.78 |
| ( 5) | 0.39 | 0.39 | 0.39 | 0.39 | 0.39 | 0.39 | 0.39 | 0.39 | 0.2 |
| ( 6) | 0.78 | 0.78 | 1.56 | 0.78 | 1.56 | 0.78 | 0.78 | 0.78 | 3.13 |
| ( 7) | 3.13 | 3.13 | 3.13 | 1.56 | 3.13 | 3.13 | 1.56 | 3.13 | 0.78 |
| ( 8) | 6.25 | 6.25 | 6.25 | 6.25 | 12.5 | 25 | 6.25 | 6.25 | 0.78 |
| ( 9) | 12.5 | 6.25 | 6.25 | 6.25 | 12.5 | 6.25 | 6.25 | 6.25 | 1.56 |
| (10) | 6.25 | 6.25 | 6.25 | 3.13 | 12.5 | 6.25 | 3.13 | 6.25 | 1.56 |
| (11) | 100 | 25 | 25 | 25 | 50 | 50 | 25 | 50 | 3.13 |
| (12) | 50 | 25 | 25 | 25 | 50 | 50 | 25 | 50 | 3.13 |
| (13) | 50 | 25 | 50 | 25 | 50 | 50 | 25 | 50 | 3.13 |
| (14) | 12.5 | 50 | >100 | 3.13 | 6.25 | 25 | 3.13 | 12.5 | 50 |
| (15) | 50 | >100 | >100 | 6.25 | 25 | 50 | 12.5 | 50 | >100 |
| (16) | 12.5 | 25 | 12.5 | 6.25 | 3.13 | 12.5 | 6.25 | 3.13 | 12.5 |

What is claimed is:

1. A compound of the general formula:

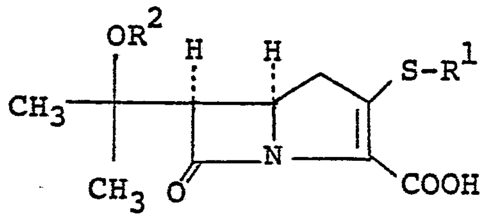

wherein $R^1$ is alkyl, cycloalkyl, cycloalkenyl, alkynyl, aryl, aralkyl, or a 5 to 8 membered heterocyclic group containing 1 to 4 hetero-atoms, each of the above members may be further substituted; and $R^2$ is hydrogen or hydroxy-protecting group, or a pharmaceutically acceptable salt thereof, or an ester thereof.

2. A compound as claimed in Claim 1, wherein alkyl or alkynyl represented by $R^1$ may be substituted by one to three substituents selected from cycloalkyl, cycloalkenyl, aryl, a 5 to 8 membered heterocylcic group containing 1 to 4 hetero-atoms, alkoxycarbonyl, acyl, oxo, halogen, cyano, hydroxy, alkoxy, aryloxy, acyloxy, carbamoyloxy, hydroxysulfonyloxy, alkylsulfonyloxy, arylsulfonyloxy, nitro, amino, carboxy, aminocarbonyl, alkylthiocarbonyl, mercapto, alkylthio, aminoalkylthio, acylaminoalkylthio, aralkylthio, arylthio, a group of Het-S in which Het means a 5 to 8 membered heterocyclic group containing 1 to 4 hetero-atoms and quaternary ammonium, and cycloalkyl, cycloalkenyl, aryl or aralkyl or a 5 to 8 membered heterocyclic group containing 1 to 4 hetero-atoms represented by $R^1$ may be substituted by 1 to 3 substituents selected from alkyl, alkoxy, alkenyl, aryl, aralkyl, mercapto, alkylthio, arylthio, aralkylthio, alkylsulfonyl, arylsulfonyl, aralkylsulfonyl, trihalogenoalkyl, hydroxy, oxo, thioxo, halogen, nitro, amino, cyano, carbamoyl, carboxy, acyl, acyloxy, acylamino, hydroxyalkyl, carboxyalkyl, halogenoalkyl and mono- or di-alkylaminoalkyl.

3. A compound as claimed in Claim 1, wherein $R^1$ is alkyl of 1 to 6 carbon atoms which may be substituted by 1 to 3 substituents selected from amino, carboxyl, alkylamino and a 5 to 6 membered cyclic amino, or alternatively $R^1$ is pyridazinyl.

4. A compound as claimed in Claim 1, which is 5,6-cis-3-(2-aminoethylthio)-6-(1-hydroxy-1-methylethyl)-7-oxo-1-azabicyclo[3.2.0]hept-2-ene-2-carboxylic acid or a pharmaceutically acceptable salt thereof.

5. A compound as claimed in Claim 1,which is 5,6-cis-3-(3-pyridazinylthio)-6-(1-hydroxy-1-methylethyl)-7-oxo-1-azabicyclo[3.2.0]hept-2-ene-2-carboxylic acid or a pharmaceutically acceptable salt thereof.

6. A compound as claimed in Claim 1, which is 5,6-cis-3-[2-(N,N-dimethylaminoethyl)thio]-6-(1-hydroxy-1-methylethyl)-7-oxo-1-azabicyclo[3.2.0]hept-2-ene-2-carboxylic acid or a pharmaceutically acceptable salt thereof.

7. A compound as claimed in Claim 1, which is 5,6-cis-3-(3-aminopropylthio)-6-(1-hydroxy-1-methylethyl)-7-oxo-1-azabicyclo[3.2.0]hept-2-ene-2-carboxylic acid or a pharmaceutically acceptable salt thereof.

8. A compound as claimed in Claim 1, which is 5,6-cis-3-(2-formimidoylaminoethylthio)-6-(1-hydroxy-1-methylethyl)-7-oxo-1-azabicyclo[3.2.0]hept-2-ene-2-carboxylic acid or a pharmaceutically acceptable salt thereof.

9. A compound as claimed in Claim 1, which is 5,6-cis-3-(3-formimidoylaminopropylthio)-6-(1-hydroxy-1-methylethyl)-7-oxo-1-azabicyclo[3.2.0]hept-2-ene-2-carboxylic acid or a pharmaceutically acceptable salt.

10. A compound as claimed in Claim 1, which is 5,6-cis-3-(2-pyrrolidinoethylthio)-6-(2-hydroxy-1-methylethyl)-7-oxo-1-azabicyclo[3.2.0]hept-2-ene-2-carboxylic acid or a pharmaceutically acceptable salt thereof.

11. A compound as claimed in Claim 1, which is 5,6-cis-3-(2-piperidinoethylthio)-6-(1-hydroxy-1-methylethyl)-7-oxo-1-azabicyclo[3.2.0]hept-2-ene-2-carboxylic acid or a pharmaceutically acceptable salt thereof.

12. A compound as claimed in Claim 1, which is 5,6-cis-3-(2-amino-2-carboxyethylthio)-6-(1-hydroxy-1-methylethyl)-7-oxo-1-azabicyclo[3.2.0]hept-2-ene-2-carboxylic acid or a pharmaceutically acceptable salt thereof.

13. A process for producing a compound of the general formula:

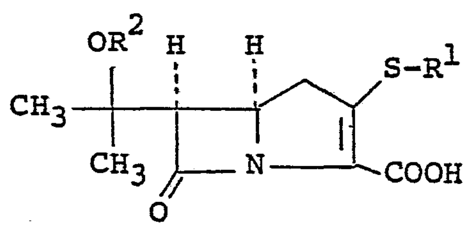

wherein $R^1$ is alkyl, cycloalkyl, cycloalkenyl, alkynyl, aryl, aralkyl, or a 5 to 8 membered heterocyclic group containing 1 to 4 hetero-atoms, each of the above members may be further substituted; and $R^2$ is hydrogen or hydroxy-protecting group, or a pharmaceutically acceptable salt thereof, or an ester thereof, which comprises reacting a compound of the general formula:

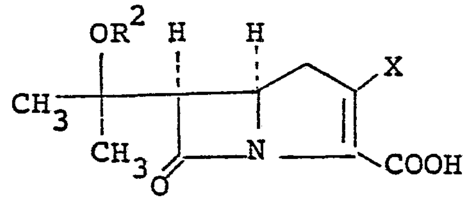

wherein X is a group to be eliminated; and $R^2$ is of the

same meaning as defined above, or a salt thereof or an ester thereof with a compound of the general formula:

$R^1SH$

wherein $R^1$ is of the same meaning as defined above, or a salt thereof.

14. A pharmaceutical composition which contains a medicinally effective amount of a compound of the general formula:

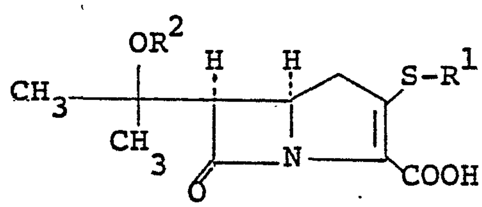

wherein $R^1$ is alkyl, cycloalkyl, cycloalkenyl, alkynyl, aryl, aralkyl, or a 5 to 8 membered heterocyclic group containing 1 to 4 hetero-atoms, each of the above members may be further substituted; and $R^2$ is hydrogen or hydroxy-protecting group, or a pharmaceutically acceptable salt thereof, or an ester thereof.

European Patent Office

# EUROPEAN SEARCH REPORT

0074599

Application number

EP 82 10 8210

## DOCUMENTS CONSIDERED TO BE RELEVANT

| Category | Citation of document with indication, where appropriate, of relevant passages | Relevant to claim | CLASSIFICATION OF THE APPLICATION (Int Cl 3) |
|---|---|---|---|
| X | EP-A-0 002 564 (BEECHAM) *Pages 79-81, examples 31-33; claims 1-9 and 23-26,35-38* | 1,14 | C 07 D 487/04<br>A 61 K 31/40 //<br>C 07 D 498/04<br>C 07 D 205/08<br>(C 07 D 487/04<br>C 07 D 209/00<br>C 07 D 205/00 ) |
| Y | EP-A-0 008 497 (BEECHAM) *Pages 39-42, examples 1,2; page 63, formula; page 65, below; claims* | 1,14 | |
| Y | GB-A-2 047 700 (KOWA) *Claims* | 1,14 | |
| P,X | EP-A-0 050 961 (KOWA) *Claims 1,2,4,8* | 1,2,14 | |
| P,Y | GB-A-2 071 099 (TAKEDA) *Claims* | 1,14 | TECHNICAL FIELDS SEARCHED (Int. Cl. 3)<br>C 07 D 487/00<br>A 61 K 31/00 |

The present search report has been drawn up for all claims

| Place of search | Date of completion of the search | Examiner |
|---|---|---|
| THE HAGUE | 07-12-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document